# EUROPÄISCHE PATENTSCHRIFT

(11) **EP 1 448 521 B1**
(45) Veröffentlichungstag und Bekanntmachung des Hinweises auf die Patenterteilung: **28.09.2005**
(21) Anmeldenummer: 02803358.7
(22) Anmeldetag: 07.11.2002
(51) Int. Cl.: C07D 207/40, C07D 405/12, A61K 31/4015, A61P 31/04

(54) **DERIVATE VON ANDRIMID UND MOIRAMID B MIT ANTIBAKTERIELLEN EIGENSCHAFTEN**
DERIVATIVES OF ANDRIMIDE AND MOIRAMIDE B HAVING ANTIBACTERIAL PROPERTIES
DERIVES D'ANDRIMIDE ET DE MOIRAMIDE B AYANT DES PROPRIETES ANTIBACTERIENNES

(30) Priorität: 20.11.2001 DE 10156894
(43) Veröffentlichungstag der Anmeldung: 25.08.2004
(73) Patentinhaber: Bayer HealthCare AG, 51368 Leverkusen (DE)
(72) Erfinder: BRUNNER, Nina, 45147 Essen (DE); FREIBERG, Christoph, 42115 Wuppertal (DE); LAMPE, Thomas, 40223 Düsseldorf (DE); NEWTON, Ben, Chesham Bois, Amersham, Bucks HP6 6BX (GB); OTTENEDER, Michael, 4144 Arlesheim (AT); PERNERSTORFER, Josef, 40721 Hilden (DE); POHLMANN, Jens, 42285 Wuppertal (DE); SCHIFFER, Guido, 42111 Wuppertal (DE); SHIMADA, Mitsuyuki, Nara-shi, Nara-ken 630-8323 (JP)
(86) Internationale Anmeldenummer: PCT/EP2002/012428
(87) Internationale Veröffentlichungsnummer: WO 2003/043982

(56) Entgegenhaltungen:
- EP-A- 0 250 115
- PATENT ABSTRACTS OF JAPAN vol. 014, no. 090 (C-0691), 20. Februar 1990 (1990-02-20) & JP 01 301657 A (SUNTORY LTD), 5. Dezember 1989 (1989-12-05) in der Anmeldung erwähnt
- MCWHORTER, WILLIAM ET AL: "Stereocontrolled synthesis of andrimid and a structural requirement for the activity" JOURNAL OF THE CHEMICAL SOCIETY, CHEMICAL COMMUNICATIONS (1989), (5), 299-301 , XP009007806
- NEEDHAM, JUDY ET AL: "Andrimid and moiramides A-C, metabolites produced in culture by a marine isolate of the bacterium Pseudomonas fluorescens: structure elucidation and biosynthesis" JOURNAL OF ORGANIC CHEMISTRY (1994), 59(8), 2058-63 , XP002234916

## Beschreibung

Die vorliegende Erfindung betrifft Verbindungen, Verfahren zur ihrer Herstellung, sie umfassende pharmazeutische Zusammensetzungen sowie ihre Verwendung zur Herstellung von Medikamenten für die Behandlung und/oder Prophylaxe von Erkrankungen bei Menschen oder Tieren, insbesondere bakteriellen Infektionskrankheiten.

Die Naturstoffe Moiramid B (R^{a} = Wasserstoff, R^{b} = Methyl) und Andrimid (R^{a}= Wasserstoff, R^{b} = Propenyl) sind als antibakteriell wirksam beschrieben, während Moiramid C (R^{a} = Hydroxy, R^{b} = Propenyl) unwirksam ist. (A. Fredenhagen, S. Y. Tamura, P. T. M. Kenny, H. Komura, Y. Naya, K. Nakanishi, *J. Am. Chem. Soc*., 1987, **109**, 4409-4411; J. Needham, M. T. Kelly, M. Ishige, R. J. Andersen, *J Org. Chem*., 1994, **59,** 2058-2063; M. P. Singh, M. J. Mroczenski-Wildey, D. A. Steinberg, R. J. Andersen, W. M. Maiese, M. Greenstein, *J. Antibiot*., 1997, **50(3),** 270-273). Die Isolierung und antibakterielle Wirksamkeit von Andrimid ist auch in EP-A-250 115 beschrieben. JP 01301657 beschreibt die Verwendung von Andrimid und einiger amidischer Derivate als agrochemische Antibiotika.

Die Synthese von Andrimid wird in A. V. Rama Rao, A. K. Singh, Ch. V. N. S. Varaprasad, *Tetrahedron Letters*, **1991,** *32*, 4393-4396 beschrieben, diejenige von Moiramid B und Andrimid in S. G. Davies, D. J. Dixon, *J. Chem. Soc. Perkin Trans. 1*, **1998**, 2635-2643.

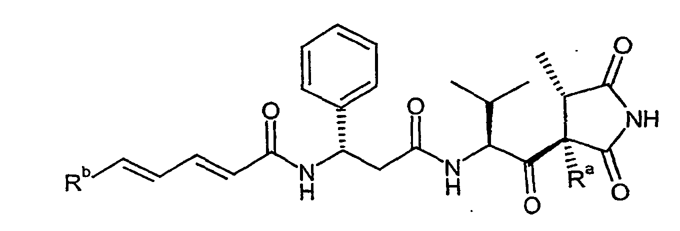

Die Naturstoffe entsprechen hinsichtlich ihrer Eigenschaften, wie z.B. ihrer Wirksamkeit nicht den Anforderungen, die an antibakterielle Arzneimittel gestellt werden.

Auf dem Markt sind zwar strukturell andersartige antibakteriell wirkende Mittel vorhanden, es kann aber regelmäßig zu einer Resistenzentwicklung kommen. Neue Mittel für eine bessere und wirksame Therapie sind daher wünschenswert.

Eine Aufgabe der vorliegenden Erfindung ist es daher, neue und alternative Verbindungen mit gleicher oder verbesserter antibakterieller Wirkung zur Behandlung von bakteriellen Erkrankungen bei Menschen und Tieren zur Verfügung zu stellen.

Überraschenderweise wurde gefunden, dass Derivate dieser Verbindungsklasse, worin die beta-Phenylalanin-Amidgruppe durch eine Harnstoffgruppe, eine Sulfonamidgruppe oder eine Carbamatgruppe ersetzt wird, antibakteriell wirksam sind.

Gegenstand der vorliegenden Erfindung sind daher Verbindungen der allgemeinen Formel (I) 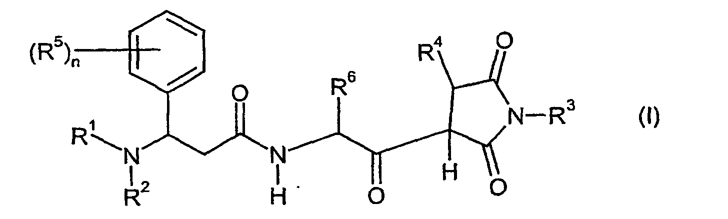 worin
- R¹: eine Gruppe 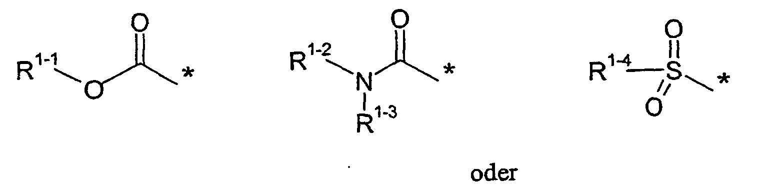 bedeutet, wobei
- R¹⁻¹: gleich Alkyl, Cycloalkyl oder Aryl,
wobei
- R¹⁻¹: gegebenenfalls substituiert sein kann mit 1 bis 3 Substituenten R¹⁻¹⁻¹, wobei R¹⁻¹⁻¹ unabhängig voneinander ausgewählt wird aus der Gruppe bestehend aus Halogen, Alkyl, Aryl, Alkoxy, Phenoxy, Amino, Monoalkylamino, Dialkylamino, Aminocarbonyl, Monoalkylaminocarbonyl, Dialkylaminocarbonyl, Carboxyl, Alkoxycarbonyl, Alkylcarbonyl, Heteroaryl und Heterocyclyl,
- R¹⁻² und R¹⁻³: gleich oder verschieden sind und Wasserstoff, Alkyl, Alkenyl, Alkinyl, Cycloalkyl, Aryl, Heteroaryl oder Heterocyclyl bedeuten,
wobei
- R¹⁻²: gegebenenfalls substituiert sein kann mit 1 bis 3 Substituenten R¹⁻²⁻¹, wobei R¹⁻²⁻¹ unabhängig voneinander ausgewählt wird aus der Gruppe bestehend aus Halogen, Trifluormethyl, Trifluormethoxy, Nitro, Amino, Alkyl, Monoalkylamino, Dialkylamino, Cycloalkyl, Aryl, Heteroaryl, Heterocyclyl, Hydroxy, Alkoxy, Phenoxy, Carboxyl, Alkoxycarbonyl, Alkylcarbonyl, Aminocarbonyl, Monoalkylaminocarbonyl, Dialkylaminocarbonyl, Aminosulfonyl, Alkylaminosulfonyl, Dialkylaminosulfonyl, Arylaminosulfonyl, Heterocyclylaminosulfonyl, Heteroarylaminosulfonyl, Aminocarbonylamino, Hydroxycarbonylamino und Alkoxycarbonylamino, wobei Aryl und Heteroaryl substituiert sein können mit 1 bis 2 Substituenten, die unabhängig voneinander ausgewählt werden aus der Gruppe bestehend aus Halogen, Hydroxy, Alkyl, Alkoxy, Trifluormethyl, Trifluormethoxy, Nitro, Amino, Cyano, Monoalkylamino und Dialkylamino,
oder
- R¹⁻² und R¹⁻³: zusammen mit dem Stickstoffatom, an das sie gebunden sind, einen Heterocyclus bilden, der gegebenenfalls benzanneliert sein kann,
- R¹⁻⁴: gleich Alkyl, Alkenyl, Alkinyl, Cycloalkyl oder Aryl,
wobei
- R¹⁻⁴: gegebenenfalls substituiert sein kann mit 1 bis 3 Substituenten R¹⁻⁴⁻¹, wobei R¹⁻⁴⁻¹ unabhängig voneinander ausgewählt wird aus der Gruppe bestehend aus Halogen, Alkyl, Aryl, Amino, Monoalkylamino, Dialkylamino, Aminocarbonyl, Monoalkylaminocarbonyl, Dialkylaminocarbonyl, Carboxyl, Alkoxycarbonyl, Alkylcarbonyl, Heteroaryl und Heterocyclyl,
- R²: gleich Wasserstoff oder Methyl,
- R³: gleich Wasserstoff oder C₁-C₃-Alkyl,
- R⁴: gleich Wasserstoff oder C₁-C₃-Alkyl,
- R⁵: ausgewählt wird aus der Gruppe bestehend aus Halogen, Trifluormethyl, Trifluormethoxy, Nitro, Amino, Monoalkylamino, Dialkylamino, Hydroxy, Alkyl, Alkoxy, Carboxyl, Alkoxycarbonyl, Aminocarbonyl, Monoalkylaminocarbonyl, Dialkylaminocarbonyl, Aryl und Heteroaryl,
oder
zwei Substituenten R⁵ zusammen mit den Kohlenstoffatomen, an die sie gebunden sind, ein Cycloalkyl oder Heterocyclyl bilden,
wobei dieses Cycloalkyl oder Heterocyclyl substituiert sein kann mit 0, 1 oder 2 Substituenten R⁵⁻¹, wobei die Substituenten R⁵⁻¹ unabhängig voneinander ausgewählt werden aus der Gruppe bestehend aus Halogen, Alkyl, Nitro, Amino, Trifluormethyl, Hydroxy und Alkoxy,
- n: gleich einer Zahl 0, 1, 2 oder 3,
wobei bei n gleich 2 oder 3 die Reste R⁵ gleich oder verschieden sein können,
- R⁶: gleich Alkyl, Cycloalkyl oder Heterocyclyl,
wobei R⁶ substituiert sein kann mit 0, 1 oder 2 Substituenten R⁶⁻¹, wobei die Substituenten R⁶⁻¹ unabhängig voneinander ausgewählt werden aus der Gruppe bestehend aus Halogen, Nitro, Amino, Trifluormethyl, Hydroxy, Alkyl und Alkoxy,
sowie deren pharmazeutisch verträgliche Salze, Solvate und Hydrate.

Die erfindungsgemäßen Verbindungen der allgemeinen Formel (I) können in verschiedenen stereoisomeren Formen auftreten, die sich entweder wie Bild und Spiegelbild (Enantiomere), oder die sich nicht wie Bild und Spiegelbild (Diastereomere) verhalten. Die Erfindung betrifft sowohl die Enantiomeren als auch die Diastereomeren sowie deren jeweilige Mischungen. Die Racemformen lassen sich ebenso wie die Diastereomeren in bekannter Weise in die stereoisomer einheitlichen Bestandteile trennen.

Weiterhin können bestimmte Verbindungen in tautomeren Formen vorliegen. Dies ist dem Fachmann bekannt, und derartige Verbindungen sind ebenfalls vom Umfang der Erfindung umfasst.

Die erfindungsgemäßen Stoffe der allgemeinen Formel (I) können auch als Salze vorliegen. Im Rahmen der Erfindung sind physiologisch unbedenkliche Salze bevorzugt.

Pharmazeutisch verträgliche Salze können Salze der erfindungsgemäßen Verbindungen mit anorganischen oder organischen Säuren sein. Bevorzugt werden Salze mit anorganischen Säuren wie beispielsweise Chlorwasserstoffsäure, Bromwasserstoffsäure, Phosphorsäure oder Schwefelsäure, oder Salze mit organischen Carbon- oder Sulfonsäuren wie beispielsweise Essigsäure, Propionsäure, Maleinsäure, Fumarsäure, Äpfelsäure, Zitronensäure, Weinsäure, Milchsäure, Benzoesäure, oder Methansulfonsäure, Ethansulfonsäure, Benzolsulfonsäure, Toluolsulfonsäure oder Naphthalindisulfonsäure.

Pharmazeutisch verträgliche Salze können ebenso Salze der erfindungsgemäßen Verbindungen mit Basen sein, wie beispielsweise Metall- oder Ammoniumsalze. Bevorzugte Beispiele sind Alkalimetallsalze (z.B. Natrium- oder Kaliumsalze), Erdalkalisalze (z.B. Magnesium- oder Calciumsalze), sowie Ammoniumsalze, die abgeleitet sind von Ammoniak oder organischen Aminen, wie beispielsweise Ethylamin, Di- bzw. Triethylamin, Ethyldiisopropylamin, Monoethanolamin, Di- bzw. Triethanolamin, Dicyclohexylamin, Dimethylaminoethanol, Dibenzylamin, N-Methylmorpholin, Dihydroabietylamin, 1-Ephenamin, Methylpiperidin, Arginin, Lysin, Ethylendiamin oder 2-Phenylethylamin.

Die Verbindungen der vorliegenden Erfindung zeichnen sich durch ein breites Wirkspektrum gegenüber Gram-positiven und Gram-negativen Bakterien aus, wobei auch multiresistente Keime erfasst werden können, insbesondere Staphylokokken, Pneumokokken und Enterokokken einschließlich Vancomycin-resistenter Stämme.

Alkyl sowie die Alkylteile in Alkoxy, Mono- und Dialkylamino, Alkylsulfonyl steht für geradliniges oder verzweigtes Alkyl und umfasst, wenn nicht anders angegeben, C₁-C₆-Alkyl, insbesondere Methyl, Ethyl, Propyl, Isopropyl, Butyl und Isobutyl.

**Alkenyl** umfasst lineares und verzweigtes C₂-C₆- und C₂-C₄-Alkenyl, wie z.B. Vinyl, Allyl, Prop-1-en-1-yl, Isopropenyl, But-1-enyle, But-2-enyle, Buta-1.2-dienyle, Buta-1.3-dienyle.

**Alkinyl** umfasst lineares und verzweigtes C₂-C₆- und C₂-C₄-Alkinyl, wie z.B. Ethinyl, n-Prop-2-in-1-yl, n-But-2-in-1-yl.

**Cycloalkyl** umfasst monocyclisches C₃-C₈-Alkyl, wie z.B. Cyclopropyl, Cyclobutyl, Cyclopentyl, Cyclohexyl, Cycloheptyl, Cyclooctyl.

**Alkoxy** steht im Rahmen der Erfindung vorzugsweise für einen geradkettigen oder verzweigten Alkoxyrest insbesondere mit 1 bis 6, 1 bis 4 bzw. 1 bis 3 Kohlenstoffatomen. Bevorzugt ist ein geradkettiger oder verzweigter Alkoxyrest mit 1 bis 3 Kohlenstoffatomen. Beispielhaft und vorzugsweise seien genannt: Methoxy, Ethoxy, n-Propoxy, Isopropoxy, t-Butoxy, n-Pentoxy und n-Hexoxy.

**Alkoxycarbonyl** steht im Rahmen der Erfindung vorzugsweise für einen geradkettigen oder verzweigten Alkoxyrest mit 1 bis 6 bzw. 1 bis 4 Kohlenstoffatomen, der über eine Carbonylgruppe verknüpft ist. Bevorzugt ist ein geradkettiger oder verzweigter Alkoxycarbonylrest mit 1 bis 4 Kohlenstoffatomen. Beispielhaft und vorzugsweise seien genannt: Methoxycarbonyl, Ethoxycarbonyl, n-Propoxycarbonyl, Isopropoxycarbonyl und t-Butoxycarbonyl.

**Monoalkylamino** steht im Rahmen der Erfindung für eine Amino-Gruppe mit einem geradkettigen oder verzweigten Alkylsubstimenten, der vorzugsweise 1 bis 6, 1 bis 4 bzw. 1 bis 2 Kohlenstoffatome aufweist. Bevorzugt ist ein geradkettiger oder verzweigter Monoalkylamino-Rest mit 1 bis 4 Kohlenstoffatomen. Beispielhaft und vorzugsweise seien genannt: Methylamino, Ethylamino, n-Propylamino, Isopropylamino, t-Butylamino, n-Pentylamino und n-Hexylamino.

**Dialkylamino** steht im Rahmen der Erfindung für eine Amino-Gruppe mit zwei gleichen oder verschiedenen geradkettigen oder verzweigten Alkylsubstituenten, die vorzugsweise jeweils 1 bis 6, 1 bis 4 bzw. 1 bis 2 Kohlenstoffatome aufweisen. Bevorzugt sind geradkettige oder verzweigte Dialkylamino-Reste mit jeweils 1 bis 4 Kohlenstoffatomen. Beispielhaft und vorzugsweise seien genannt: *N*,*N*-Dimethylamino, *N*,*N*-Diethylamino, *N*-Ethyl-*N*-methylamino, *N*-Methyl-*N*-n-propylamino, *N*-Isopropyl-*N*-n-propylamino, *N*-t-Butyl-*N*-methylamino, *N*-Ethyl-*N*-n-pentylamino und *N*-n-Hexyl-*N*-methylamino.

**Mono- oder Dialkylaminocarbonyl** steht im Rahmen der Erfindung für eine Amino-Gruppe, die über eine Carbonylgruppe verknüpft ist und die einen geradkettigen oder verzweigten bzw. zwei gleiche oder verschiedene geradkettige oder verzweigte Alkylsubstituenten mit vorzugsweise jeweils 1 bis 4 bzw. 1 bis 2 Kohlenstoffatomen aufweist. Beispielhaft und vorzugsweise seien genannt: Methylaminocarbonyl, Ethylaminocarbonyl, Isopropylaminocarbonyl, t-Butylaminocarbonyl, *N*,*N*-Dimethylaminocarbonyl, *N*,*N*-Diethylaminocarbonyl, *N*-Ethyl-*N*-methylaminocarbonyl und *N*-t-Butyl-*N*-methylaminocarbonyl.

**Alkylcarbonylamino** (Acylamino) steht im Rahmen der Erfindung für eine Amino-Gruppe mit einem geradkettigen oder verzweigten Alkanoylsubstituenten, der vorzugsweise 1 bis 6, 1 bis 4 bzw. 1 bis 2 Kohlenstoffatome aufweist und über die Carbonylgruppe verknüpft ist. Bevorzugt ist ein Monoacylamino-Rest mit 1 bis 2 Kohlenstoffatomen. Beispielhaft und vorzugsweise seien genannt: Formamido, Acetamido, Propionamido, n-Butyramido und Pivaloylamido.

**Alkoxycarbonylamino** steht im Rahmen der Erfindung für eine Amino-Gruppe mit einem geradkettigen oder verzweigten Alkoxycarbonylsubstituenten, der vorzugsweise im Alkoxyrest 1 bis 6 bzw. 1 bis 4 Kohlenstoffatome aufweist und über die Carbonylgruppe verknüpft ist. Bevorzugt ist ein Alkoxycarbonylamino-Rest mit 1 bis 4 Kohlenstoffatomen. Beispielhaft und vorzugsweise seien genannt: Methoxycarbonylamino, Ethoxycarbonylamino, n-Propoxycarbonylamino und t-Butoxycarbonylamino.

**Aminosulfonyl** steht für eine -S(O)₂NH₂-Gruppe. Dementsprechend sind Alkyllaminosulfonyl, Dialkylaminosulfonyl, Arylaminosulfonyl, Heterocyclylaminosulfonyl und Heteroarylaminosulfonyl an der Aminogruppe mit den entsprechenden Resten substituiert, d.h. Alkyl, Aryl etc.

**Aryl** steht im allgemeinen für einen aromatischen Rest mit 6 bis 10 Kohlenstoffatomen. Bevorzugte Arylreste sind Phenyl und Naphthyl.

**Heteroaryl** (Heteroaromat) steht für einen 5- bis 10-gliedrigen aromatischen Heterocyclus mit bis zu 3 Heteroatomen aus der Reihe S, O und/oder N, beispielsweise für Pyridyl, Pyrimidyl, Thienyl, Furyl, Pyrrolyl, Thiazolyl, N-Triazolyl, Oxazolyl oder Imidazolyl. Bevorzugt sind Pyridyl, Furyl, Thienyl und Thiazolyl.

**Heterocyclyl** (Heterocyclus) steht für einen gegebenenfalls über ein Stickstoffatom gebundenen 3- bis 8- gliedrigen gesättigten oder ungesättigten, nicht aromatischen Heterocyclus der bis zu 3 Heteroatome aus der Reihe S, O und N enthalten kann. Er kann aus zwei Substituentengruppen zusammen mit dem Stickstoffatom, an das sie gebunden sind, gebildet werden und schließt z.B. Morpholinyl, Piperidinyl, Piperazinyl, Methylpiperazinyl, Thiomorpholinyl oder Pyrrolidinyl ein sowie 3-, 7- und 8-gliedrige Heterocyclen, wie z.B. Aziridine (z.B. 1-Azacyclopropan-1-yl), Azetidine (z.B. 1-Azacyclobutan-1-yl) und Azepine (z.B. 1-Azepan-1-yl) ein. Die ungesättigten Vertreter können 1 bis 2 Doppelbindungen im Ring enthalten.

**Halogen** steht für Fluor, Chlor, Brom oder Jod, wobei Fluor und Chlor bevorzugt sind, wenn nichts anderes angegeben ist.

**Benzanneliert** steht im Rahmen der Erfindung für ein Phenyl, welches über zwei Kohlenstoffatome an einen Heterocyclus oder ein Cycloalkyl gebunden ist.

Die oben aufgeführten allgemeinen oder in Vorzugsbereichen angegebenen Restedefinitionen gelten sowohl für die Endprodukte der Formel (I) als auch entsprechend für die jeweils zur Herstellung benötigten Ausgangsstoffe bzw. Zwischenprodukte.

Die in den jeweiligen Kombinationen bzw. bevorzugten Kombinationen von Resten im einzelnen angegebenen Restedefinitionen werden unabhängig von den jeweilig angegebenen Kombinationen der Reste beliebig auch durch Restedefinitionen anderer Kombinationen ersetzt.

In einer weiteren Ausführungsform betrifft die Erfindung Verbindungen der allgemeinen Formel (I),
worin
- R¹⁻¹: gleich Alkyl, Cycloalkyl oder Aryl,
wobei
- R¹⁻¹: gegebenenfalls substituiert sein kann mit 1 bis 3 Substituenten R¹⁻¹⁻¹, wobei R¹⁻¹⁻¹ unabhängig voneinander ausgewählt wird aus der Gruppe bestehend aus Halogen, Alkyl, Aryl und Alkoxy,
- R¹⁻² und R¹⁻³: gleich oder verschieden sind und Wasserstoff, Alkyl, Alkenyl, Cycloalkyl, Aryl oder Heteroaryl bedeuten,
wobei R¹⁻² gegebenenfalls substituiert sein kann mit 1 bis 2 Substituenten R¹⁻²⁻¹, wobei R¹⁻²⁻¹ unabhängig voneinander ausgewählt wird aus der Gruppe bestehend aus Halogen, Trifluormethyl, Trifluormethoxy, Nitro, Amino, Alkyl, Monoalkylamino, Dialkylamino, Cycloalkyl, Aryl, Heteroaryl, Heterocyclyl, Hydroxy, Alkoxy, Phenoxy, Alkylcarbonyl, Aminocarbonyl, Monoalkylaminocarbonyl, Dialkylaminocarbonyl, Aminosulfonyl, wobei Aryl und Heteroaryl substituiert sein können mit 1 bis 2 Substituenten, die unabhängig voneinander ausgewählt werden aus der Gruppe bestehend aus Halogen, Hydroxy, Alkoxy, Trifluormethyl, Trifluormethoxy, Nitro und Amino,
oder R¹⁻² und R¹⁻³ zusammen mit dem Stickstoffatom, an das sie gebunden sind, einen Heterocyclus bilden, der gegebenenfalls benzanneliert sein kann,
- R¹⁻⁴: gleich Alkyl, Alkenyl oder Aryl,
wobei
- R¹⁻⁴: gegebenenfalls substituiert sein kann mit 1 bis 3 Substituenten R¹⁻⁴⁻¹, wobei R¹⁻⁴⁻¹ unabhängig voneinander ausgewählt wird aus der Gruppe bestehend aus Halogen, Alkyl und Aryl,
- R²: gleich Wasserstoff,
- R³: gleich Wasserstoff oder Methyl,
- R⁴: gleich Methyl,
- R⁵: ausgewählt wird aus der Gruppe bestehend aus Fluor, Chlor, Trifluormethyl, Trifluormethoxy, Nitro, Amino, Monoalkylamino, Dialkylamino, Hydroxy, Alkyl, Alkoxy, Alkoxycarbonyl, Aminocarbonyl, Phenyl und 5- bis 6-gliedriges Heteroaryl,
oder
zwei Substituenten R⁵ zusammen mit den Kohlenstoffatomen, an die sie gebunden sind, ein Cycloalkyl oder Heterocyclyl bilden,
- n: gleich einer Zahl 0, 1 oder 2,
wobei bei n gleich 2 die Reste R⁵ gleich oder verschieden sein können,
- R⁶: gleich Alkyl oder Cycloalkyl,
wobei R⁶ substituiert sein kann mit 0, 1 oder 2 Substituenten R⁶⁻¹, wobei die Substituenten R⁶⁻¹ unabhängig voneinander ausgewählt werden aus der Gruppe bestehend aus Halogen, Trifluormethyl und Alkoxy,
sowie deren pharmazeutisch verträgliche Salze, Solvate und Hydrate.

In einer weiteren Ausführungsform betrifft die Erfindung Verbindungen der allgemeinen Formel (I),
worin
- R¹⁻¹: gleich C₁-C₄-Alkyl, Cyclopentyl, Cyclohexyl, Phenyl oder Naphtyl,
wobei
- R¹⁻¹: gegebenenfalls substituiert sein kann mit 1 bis 2 Substituenten R¹⁻¹⁻¹, wobei R¹⁻¹⁻¹ unabhängig voneinander ausgewählt wird aus der Gruppe bestehend aus Fluor, Chlor, Methyl, Ethyl, Phenyl, Methoxy und Ethoxy,
- R¹⁻²: Alkyl, Alkenyl, Cycloalkyl, Aryl oder Heteroaryl bedeutet,
wobei R¹⁻² gegebenenfalls substituiert sein kann mit 1 Substituenten R¹⁻²⁻¹, wobei R¹⁻²⁻¹ ausgewählt wird aus der Gruppe bestehend aus Fluor, Chlor, Trifluormethyl, Amino, Alkyl, Monoalkylamino, Dialkylamino, Alkylcarbonyl, Aryl, Heteroaryl, Hydroxy, Methoxy und Phenoxy, wobei Aryl und Heteroaryl substituiert sein können mit 1 Substituenten ausgewählt aus der Gruppe bestehend aus Halogen, Methoxy, Trifluormethyl und Amino,
- R¹⁻³: gleich Wasserstoff oder Methyl bedeutet,
oder R¹⁻² und R¹⁻³ zusammen mit dem Stickstoffatom, an das sie gebunden sind, einen Heterocyclus bilden, der gegebenenfalls benzanneliert sein kann,
- R¹⁻⁴: gleich Alkyl, Alkenyl oder Phenyl,
wobei
- R¹⁻⁴: gegebenenfalls substituiert sein kann mit 1 Substituenten R¹⁻⁴⁻¹, wobei R¹⁻⁴⁻¹ ausgewählt wird aus der Gruppe bestehend aus Fluor, Chlor und Phenyl,
- R²: gleich Wasserstoff,
- R³: gleich Wasserstoff,
- R⁴: gleich Methyl,
- R⁵: ausgewählt wird aus der Gruppe bestehend aus Fluor, Chlor, Trifluormethyl, Alkoxy, Methoxycarbonyl, C₁-C₄-Alkyl, Phenyl und Pyridyl,
- n: gleich einer Zahl 0, 1 oder 2,
wobei bei n gleich 2 die Reste R⁵ gleich oder verschieden sein können,
- R⁶: gleich Isopropyl, Isobutyl, Isopentyl oder Cyclopentyl,
sowie deren pharmazeutisch verträgliche Salze, Solvate und Hydrate.

Bevorzugt im Rahmen der vorliegenden Erfindung sind Verbindungen der allgemeinen Formel (I), welche die allgemeine Formel (Ic) aufweisen: 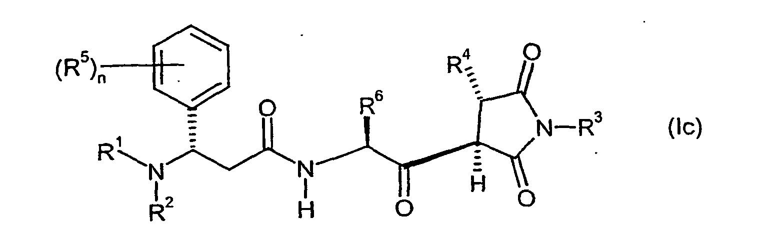 worin

R¹ bis R⁶ und n wie oben definiert sind.

Bevorzugt im Rahmen der vorliegenden Erfindungen sind auch Verbindungen der allgemeinen Formel (I), worin R¹⁻² gleich Alkyl, Alkenyl, Cycloalkyl oder Aryl bedeutet,
wobei
- R¹⁻²: gegebenenfalls substituiert sein kann mit 1 bis 2, insbesondere einem Substituenten R¹⁻²⁻¹, wobei R¹⁻²⁻¹ ausgewählt wird aus der Gruppe bestehend aus Halogen, Nitro, Amino, Alkyl, Aryl, Heteroaryl, Hydroxy, Alkoxy, Carboxyl, Alkylcarbonyl, Alkoxycarbonyl und Aminocarbonyl, insbesondere ausgewählt aus der Gruppe bestehend aus Alkyl, Aryl, Heteroaryl und Alkoxy.

In einer weiteren Ausführungsform betrifft die Erfindung Verbindungen der allgemeinen Formel (Ia), 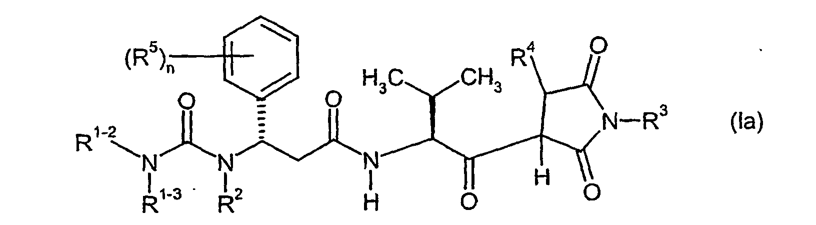 worin R¹⁻² und R¹⁻³ gleich oder verschieden sind und Wasserstoff, Alkyl, Alkenyl, Alkinyl, Cycloalkyl, Aryl, Heteroaryl oder Heterocyclyl bedeuten,
wobei R¹⁻² gegebenenfalls substituiert sein kann mit 1 bis 3 Substituenten R¹⁻²⁻¹, wobei R¹⁻²⁻¹ unabhängig voneinander ausgewählt wird aus der Gruppe bestehend aus Halogen, Trifluormethyl, Trifluormethoxy, Nitro, Amino, Monoalkylamino, Dialkylamino, Cycloalkyl, Aryl, Heteroaryl, Heterocyclyl, Hydroxy, Alkoxy, Carboxyl, Alkoxycarbonyl, Aminocarbonyl, Monoalkylaminocarbonyl, Dialkylaminocarbonyl, Aminosulfonyl, Alkylaminosulfonyl, Dialkylaminosulfonyl, Arylaminosulfonyl, Heterocyclylaminosulfonyl, Heteroarylaminosulfonyl, Aminocarbonylamino, Hydroxycarbonylamino und Alkoxycarbonylamino, wobei Aryl und Heteroaryl substituiert sein können mit 1 bis 2 Substituenten, die unabhängig voneinander ausgewählt werden aus der Gruppe bestehend aus Halogen, Hydroxy, Alkoxy, Trifluormethyl, Trifluormethoxy, Nitro, Amino, Cyano, Monoalkylamino und Dialkylamino,
oder R¹⁻² und R¹⁻³ zusammen mit dem Stickstoffatom, an das sie gebunden sind, einen Heterocyclus bilden,
- R²: gleich Wasserstoff oder Methyl,
- R³: gleich Wasserstoff oder C₁-C₃-Alkyl,
- R⁴: gleich Wasserstoff oder C₁-C₃-Alkyl,
- R⁵: ausgewählt wird aus der Gruppe bestehend aus Halogen, Trifluormethyl, Trifluormethoxy, Nitro, Amino, Monoalkylamino, Dialkylamino, Hydroxy, Alkoxy, Carboxyl, Alkoxycarbonyl, Aminocarbonyl, Monoalkylaminocarbonyl und Dialkylaminocarbonyl,
- n: gleich einer Zahl 0, 1, 2 oder 3,
wobei bei n gleich 2 oder 3 die Reste R⁵ gleich oder verschieden sein können,
sowie deren pharmazeutisch verträgliche Salze, Solvate und Hydrate.

In einer weiteren Ausführungsform betrifft die Erfindung Verbindungen der allgemeinen Formel (Ia),
worin
- R¹⁻² und R¹⁻³: gleich oder verschieden sind und Wasserstoff, Alkyl, Alkenyl, Aryl oder Heteroaryl bedeuten,
wobei R¹⁻² gegebenenfalls substituiert sein kann mit 1 bis 2 Substituenten R¹⁻²⁻¹, wobei R¹⁻²⁻¹ unabhängig voneinander ausgewählt wird aus der Gruppe bestehend aus Halogen, Trifluormethyl, Trifluormethoxy, Nitro, Amino, Monoalkylamino, Dialkylamino, Cycloalkyl, Aryl, Heteroaryl, Heterocyclyl, Hydroxy, Alkoxy, Aminocarbonyl, Monoalkylaminocarbonyl, Dialkylaminocarbonyl und Aminosulfonyl, wobei Aryl und Heteroaryl substituiert sein können mit 1 bis 2 Substituenten, die unabhängig voneinander ausgewählt werden aus der Gruppe bestehend aus Halogen, Hydroxy, Alkoxy, Trifluormethyl, Trifluormethoxy, Nitro und Amino,
oder R¹⁻² und R¹⁻³ zusammen mit dem Stickstoffatom, an das sie gebunden sind, einen Heterocyclus bilden,
- R²: gleich Wasserstoff,
- R³: gleich Wasserstoff oder Methyl,
- R⁴: gleich C₁-C₃-Alkyl,
- R⁵: ausgewählt wird aus der Gruppe bestehend aus Fluor, Chlor, Trifluormethyl, Trifluormethoxy, Nitro, Amino, Monoalkylamino, Dialkylamino, Hydroxy, Alkoxy, Aminocarbonyl,
- n: gleich einer Zahl 0, 1 oder 2,
wobei bei n gleich 2 die Reste R⁵ gleich oder verschieden sein können,
sowie deren pharmazeutisch verträgliche Salze, Solvate und Hydrate.

In einer weiteren Ausführungsform betrifft die Erfindung Verbindungen der allgemeinen Formel (Ia),
worin
- R¹⁻²: Alkyl, Alkenyl, Aryl oder Heteroaryl bedeutet,
wobei R¹⁻² gegebenenfalls substituiert sein kann mit 1 Substituenten R¹⁻²⁻¹, wobei R¹⁻²⁻¹ ausgewählt wird aus der Gruppe bestehend aus Fluor, Chlor, Trifluormethyl, Amino, Monoalkylamino, Dialkylamino, Aryl, Heteroaryl, Hydroxy und Methoxy, wobei Aryl und Heteroaryl substituiert sein können mit 1 Substituenten ausgewählt aus der Gruppe bestehend aus Halogen, Hydroxy, Alkoxy, Trifluormethyl und Amino,
- R¹⁻³: Wasserstoff oder Methyl bedeutet,
oder R¹⁻² und R¹⁻³ zusammen mit dem Stickstoffatom, an das sie gebunden sind, einen Heterocyclus bilden,
- R²: gleich Wasserstoff,
- R³: gleich Wasserstoff,
- R⁴: gleich Methyl,
- R⁵: ausgewählt wird aus der Gruppe bestehend aus Fluor, Chlor, Trifluormethyl, Amino, Monoalkylamino, Dialkylamino, Hydroxy und Alkoxy,
- n: gleich einer Zahl 0, 1 oder 2,
wobei bei n gleich 2 die Reste R⁵ gleich oder verschieden sein können,
sowie deren pharmazeutisch verträgliche Salze, Solvate und Hydrate.

Bevorzugt im Rahmen der vorliegenden Erfindung sind Verbindungen der allgemeinen Formel (I), welche die allgemeine Formel (Ib) aufweisen: 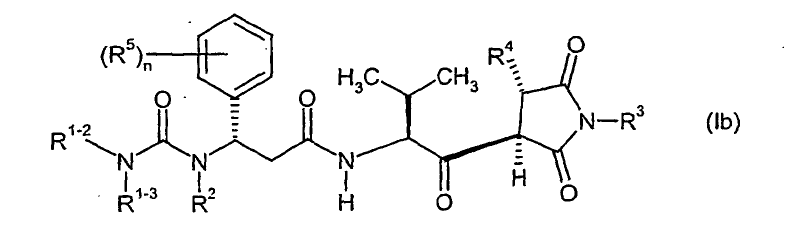 worin
R¹⁻² bis R⁵ und n wie oben definiert sind.

Bevorzugt im Rahmen der vorliegenden Erfindungen sind auch Verbindungen der allgemeinen Formel (I), worin
- R¹: eine Gruppe 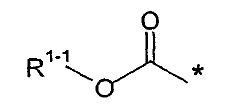
bedeutet, wobei
R¹⁻¹ gleich C₁-C₄-Alkyl ist.

Bevorzugt im Rahmen der vorliegenden Erfindungen sind auch Verbindungen der allgemeinen Formel (I), worin
- R¹: eine Gruppe 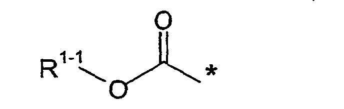
bedeutet, wobei
R¹⁻¹ gleich Phenyl oder Naphtyl ist, wobei R¹⁻¹ gegebenenfalls substituiert sein kann mit 1 bis 2 Substituenten R¹⁻¹⁻¹, wobei R¹⁻¹⁻¹ unabhängig voneinander ausgewählt wird aus der Gruppe bestehend aus Fluor, Chlor, Methyl, Ethyl, Phenyl, Methoxy und Ethoxy.

Bevorzugt im Rahmen der vorliegenden Erfindungen sind auch Verbindungen der allgemeinen Formel (I), (Ia), (Ib) oder (Ic), worin
- R¹: eine Gruppe 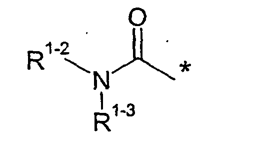
bedeutet, wobei
R¹⁻² gleich Alkyl, Alkenyl oder Aryl bedeutet,
wobei R¹⁻² gegebenenfalls substituiert sein kann mit 1 bis 2, insbesondere einem Substituenten R¹⁻²⁻¹, wobei R¹⁻²⁻¹ ausgewählt wird aus der Gruppe bestehend aus Halogen, Nitro, Amino, Aryl, Heteroaryl, Hydroxy, Alkoxy, Carboxyl, Alkoxycarbonyl und Aminocarbonyl, insbesondere ausgewählt aus der Gruppe bestehend aus Aryl, Heteroaryl und Alkoxy.

Bevorzugt im Rahmen der vorliegenden Erfindungen sind auch Verbindungen der allgemeinen Formel (I), (Ia), (Ib) oder (Ic),
worin
- R¹: eine Gruppe 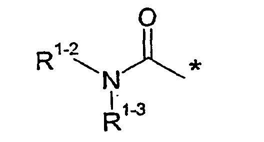
bedeutet, wobei
R¹⁻³ gleich Wasserstoff oder Methyl ist.

Bevorzugt im Rahmen der vorliegenden Erfindungen sind auch Verbindungen der allgemeinen Formel (I), worin
- R¹: eine Gruppe 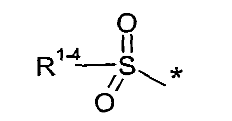
bedeutet, wobei R¹⁻⁴ gleich Alkyl ist.

Bevorzugt im Rahmen der vorliegenden Erfindungen sind auch Verbindungen der allgemeinen Formel (I), worin
- R¹: eine Gruppe 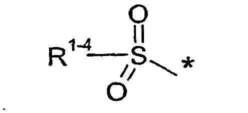
bedeutet, wobei
R¹⁻⁴ gleich Alkenyl oder Phenyl ist, wobei R¹⁻⁴ gegebenenfalls substituiert sein kann mit 1 Substituenten R¹⁻⁴⁻¹, wobei R¹⁻⁴⁻¹ ausgewählt wird aus der Gruppe bestehend aus Fluor, Chlor und Phenyl.

Bevorzugt im Rahmen der vorliegenden Erfindungen sind auch Verbindungen der allgemeinen Formel (I), (Ia), (Ib) oder (Ic), worin R² gleich Wasserstoff ist.

Bevorzugt im Rahmen der vorliegenden Erfindungen sind auch Verbindungen der allgemeinen Formel (I), (Ia), (Ib) oder (Ic), worin R³ gleich Wasserstoff ist.

Bevorzugt im Rahmen der vorliegenden Erfindungen sind auch Verbindungen der allgemeinen Formel (I), (Ia), (Ib) oder (Ic), worin R⁴ gleich Methyl ist.

Bevorzugt im Rahmen der vorliegenden Erfindungen sind auch Verbindungen der allgemeinen Formel (I), (Ia), (Ib) oder (Ic), worin R⁵ gleich Wasserstoff ist.

Bevorzugt im Rahmen der vorliegenden Erfindungen sind auch Verbindungen der allgemeinen Formel (I) oder (Ic), worin R⁶ gleich Alkyl oder Cycloalkyl ist.

Bevorzugt im Rahmen der vorliegenden Erfindungen sind auch Verbindungen der allgemeinen Formel (I) oder (Ic), worin R⁶ gleich Isopropyl ist.

Bevorzugt im Rahmen der vorliegenden Erfindungen sind auch folgende Verbindungen:
(*S*)-3-(3-Benzyl-ureido)-N-{(*S*)-2-methyl-1-[1-((3*R*,4*S*)-4-methyl-2,5-dioxo-pyrrolidin-3-yl)-methanoyl]-propyl}-3-phenyl-propionamid,
(*S*)-3-[3-(4-Methoxy-phenyl)-ureido]-N-{(*S*)-2-methyl-1-[1-((3*R*,4*S*)-4-methyl-2,5-dioxo-pyrrolidin-3-yl)-methanoyl]-propyl}-3-phenyl-propionamid,
(*S*)-N-{(*S*)-2-Methyl-1-[1-((3*R*,4*S*)-4-methyl-2,5-dioxo-pyrrolidin-3-yl)-methanoyl]-propyl}-3-(3-methyl-3-phenyl-ureido)-3-phenyl-propionamid,
N-((1*S*)-2-Methyl-1-{[(3*R*,4*S*)-4-methyl-2,5-dioxo-3-pyrrolidinyl]carbonyl}propyl)-3-{[(1-naphthylamino)carbonyl]amino}-3-phenylpropionamid,
3-[({[4-Chlor-3-(trifluormethyl)phenyl]amino}carbonyl)amino]-N-((1*S*)-2-methyl-1-{[(3*R*,4*S*)-4-methyl-2,5-dioxo-3-pyrrolidinyl]carbonyl}propyl)-3-phenyl-propionamid,
tert-Butyl-(1S)-3-({1-cyclopentyl-2-[(3R,4S)-4-methyl-2,5-dioxo-3-pyrrolidinyl]-2-oxoethyl}amino)-3-oxo-1-phenylpropylcarbamat,
(R,2S)-N-(3-{[(1,1'-Biphenyl-4-yloxy)carbonyl]amino}-3-phenylpropanoyl)-(3R,4S)-3-[(R)-N-(3-{[(1,1'-biphenyl-4-yloxy)carbonyl]amino}-3-phenylpropanoyl)-L-valyl]-4-methyl-2,5-dioxo-2-pyrrolidinyl-{(3S,4R)-4-[(R)-N-(3-{[(1,1'-biphenyl-4-yloxy)-carbonyl]amino}-3-phenylpropanoyl)-L-valyl]-3-methyl-2,5-dioxo-3-pyrrolidinyl}-L-valin, {2-[(S)-2-Methyl-1-((3R, 4S)-4-methyl-2,5-dioxo-pyrrolidin-3-carbonyl)-propylcarbamoyl]-1-phenyl-ethyl}-carbaminsäure-biphenyl-4-yl-ester.
(3*S*)-N-((1*S*)-2-Methyl-1-{[(3*R*,4*S*)-4-methyl-2,5-dioxo-3-pyrrolidinyl]carbonyl}propyl)-3-phenyl-3-({[(*E*)-2-phenylethenyl]sulfonyl}amino)propionamid.

Die vorliegende Erfindung betrifft weiterhin ein Verfahren zur Herstellung der Verbindungen der allgemeinen Formel (I), worin Verbindungen der allgemeinen Formel (II) 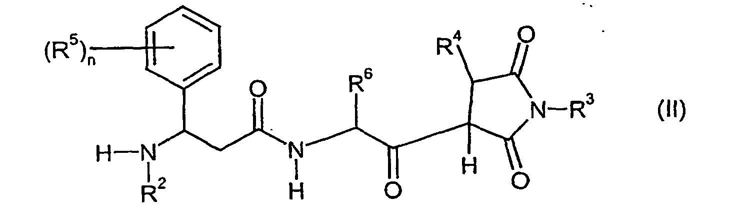 in welcher
R² bis R⁶ und n die oben angegebene Bedeutung aufweisen,
wobei diese gegebenenfalls in Form ihrer Salze vorliegen können,
für den Fall, dass
- R¹: eine Gruppe 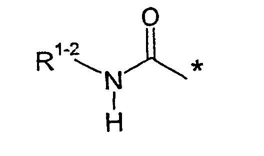
in welcher R¹⁻² die oben angegebene Bedeutung aufweist, bedeutet,
[A] mit Verbindungen der allgemeinen Formel (IIIa) 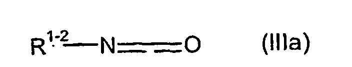 in welcher
   R¹⁻² die oben angegebene Bedeutung aufweist, umgesetzt werden, oder
   für den Fall, dass
   R¹ eine Gruppe 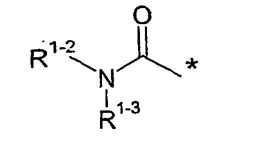 bedeutet, wobei
   R¹⁻³ ungleich Wasserstoff ist, oder R¹⁻² und R¹⁻³ einen Heterocyclus bilden,
[B] mit Verbindungen der allgemeinen Formel (IIIb) 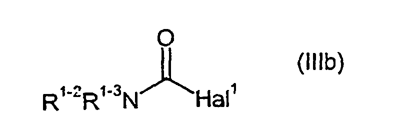 in welcher
   R¹⁻² und R¹⁻³ die oben angegebene Bedeutung aufweisen und Hal¹ für ein Halogenid oder eine andere Fluchtgruppe steht, umgesetzt werden, oder
   für den Fall, dass
   R¹ eine Gruppe 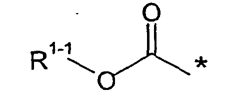 in welcher
   R¹⁻¹ die oben angegebene Bedeutung aufweist, bedeutet,
[C] mit Verbindungen der allgemeinen Formel (IIIc) 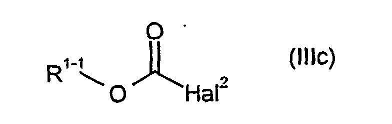 in welcher
   R¹⁻¹ die oben angegebene Bedeutung aufweist und Hal² für ein Halogenid oder eine andere Fluchtgruppe steht, umgesetzt werden, oder
   für den Fall, dass
   R¹ eine Gruppe 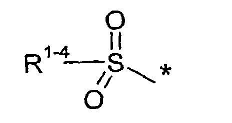 bedeutet,
   in welcher R¹⁻⁴ die oben angegebene Bedeutung aufweist,
[D] mit Verbindungen der allgemeinen Formel (IIId) 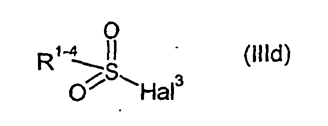
in welcher
R¹⁻⁴ die oben angegebene Bedeutung aufweist und Hal³ für ein Halogenid oder eine andere Fluchtgruppe steht, umgesetzt werden.

Die Umsetzung nach den Verfahren [A] bis [D] erfolgt im allgemeinen in Gegenwart eines Lösemittels, gegebenenfalls in Gegenwart einer Base.

Basen sind beispielsweise Alkalicarbonate wie Cäsiumcarbonat, Natrium- oder Kaliumcarbonat, oder Kalium-tert.-butylat, oder tertiäre Aminbasen wie Triethylamin oder Diisopropylethylamin, oder polymergebundene Aminbasen wie PS-DIEA, oder andere Basen wie DBU, bevorzugt sind Triethylamin, Diisopropylethylamin oder PS-DIEA.

Als Lösemittel eignen sich hierbei inerte organische Lösemittel, die sich unter den Reaktionsbedingungen nicht verändern. Hierzu gehören Halogenkohlenwasserstoffe wie Dichlormethan, Trichlormethan oder Dichlorethan, Kohlenwasserstoffe wie Benzol, Xylol, Toluol, Hexan, Cyclohexan oder Erdölfraktionen, Nitromethan, Dimethylformamid oder Acetonitril oder Ether wie Diethylether, Tetrahydrofuran oder Dioxan. Ebenso ist es möglich, Gemische der Lösemittel einzusetzen. Besonders bevorzugt sind Dichlormethan, Dichlorethan, Tetrahydrofuran oder Dimethylformamid.

Die Verbindungen der allgemeinen Formel (II) sind literaturbekannt oder neu und können hergestellt werden, indem Verbindungen der allgemeinen Formel (Id) 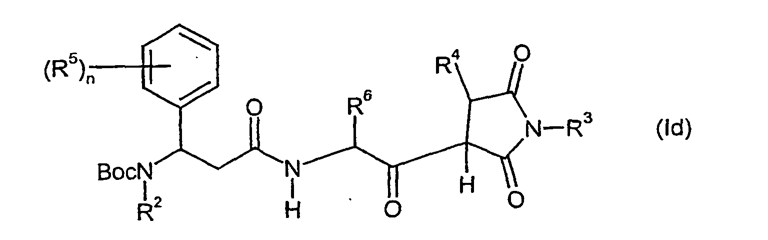 in welcher
R² bis R⁶ und n die oben angegebene Bedeutung aufweisen,
mit Säure, insbesondere mit Salzsäure oder Trifluoressigsäure versetzt werden.

Als Lösemittel eignen sich hierbei inerte organische Lösemittel, die sich unter den Reaktionsbedingungen nicht verändern. Hierzu gehören Halogenkohlenwasserstoffe wie Dichlormethan oder Trichlormethan, Kohlenwasserstoffe wie Benzol, Xylol, Toluol, Hexan, Cyclohexan, oder Erdölfraktionen, Nitromethan, Dimethylformamid oder Acetonitril oder Ether wie Diethylether, Tetrahydrofuran oder Dioxan. Ebenso ist es möglich, Gemische der Lösemittel einzusetzen. Besonders bevorzugt ist die Verwendung von Salzsäure in Dioxan oder Trifluoressigsäure in Dichlormethan.

Die Verbindungen der allgemeinen Formel (Id) stellen eine spezielle Ausführungsform der Verbindungen der allgemeinen Formel (I) dar.

Die Verbindungen der allgemeinen Formel (Id) sind literaturbekannt oder neu und können hergestellt werden, indem Verbindungen der allgemeinen Formel (V) 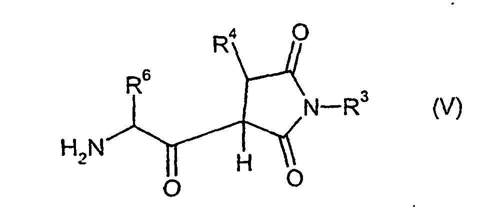 in welcher
R³, R⁴ und R⁶ die oben angegebene Bedeutung aufweisen,
mit Verbindungen der allgemeinen Formel (VI) 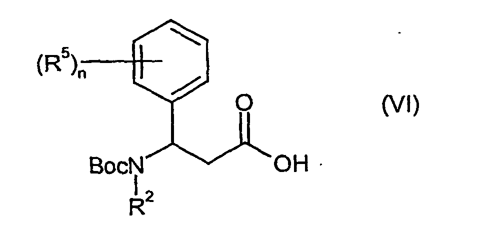 in welcher
R², R⁵ und n die oben angegebene Bedeutung aufweisen,
wobei diese gegebenenfalls in aktivierter Form vorliegen können, umgesetzt werden.

Zur Überführung der Verbindungen in die aktivierte Form sind beispielsweise Carbodiimide wie z.B. N,N'-Diethyl-, N,N,'-Dipropyl-, N,N'-Diisopropyl-, N,N'-Dicyclohexylcarbodiimid, N-(3-Dimethylaminoisopropyl)-N'-ethylcarbodiimid-Hydrochlorid (EDC) (gegebenenfalls in Gegenwart von Pentafluorphenol (PFP)), N-Cyclohexylcarbodiimid-N'-propyloxymethyl-Polystyrol (PS-Carbodiimid) oder Carbonylverbindungen wie Carbonyldiimidazol, oder 1,2-Oxazoliumverbindungen wie 2-Ethyl-5-phenyl-1,2-oxazolium-3-sulfat oder 2-tert.-Butyl-5-methyl-isoxazolium-perchlorat, oder Acylaminoverbindungen wie 2-Ethoxy-1-ethoxycarbonyl-1,2-dihydrochinolin, oder Propanphosphonsäureanhydrid, oder Isobutylchloroformat, oder Bis-(2-oxo-3-oxazolidinyl)-phosphorylchlorid oder Benzotriazolyloxy-tri(dimethylamino)phosphoniumhexafluorophosphat, oder O-(Benzotriazol-1-yl)-N,N,N',N'-tetra-methyluronium-hexafluorophosphat (HBTU), 2-(2-Oxo-1-(2H)-pyridyl)-1,1,3,3-tetramethyluroniumtetrafluoro-borat (TPTU) oder O-(7-Azabenzotriazol-1-yl)-N,N,N,N-tetramethyl-uroniumhexafluorophosphat (HATU), oder 1-Hydroxybenztriazol (HOBt), oder Benzotriazol-1-yloxytris(dimethylamino)-phosphoniumhexafluoro-phosphat (BOP), oder Mischungen aus diesen mit Basen geeignet.

Basen sind beispielsweise Alkalicarbonate, wie z.B. Natrium- oder Kaliumcarbonat, oder -hydrogencarbonat, oder organische Basen wie Trialkylamine z.B. Triethylamin, N-Methylmorpholin, N-Methylpiperidin, 4- Dimethylaminopyridin oder Diisopropylethylamin.

Bevorzugt ist die Verwendung von HATU und Diisopropylethylamin.

Als Lösemittel eignen sich hierbei inerte organische Lösemittel, die sich unter den Reaktionsbedingungen nicht verändern. Hierzu gehören Halogenkohlenwasserstoffe wie Dichlormethan oder Trichlormethan, Kohlenwasserstoff wie Benzol, Xylol, Toluol, Hexan, Cyclohexan, oder Erdölfraktionen, Nitromethan, Dimethylformamid oder Acetonitril oder Ether wie Diethylether, Tetrahydrofuran oder Dioxan. Ebenso ist es möglich, Gemische der Lösemittel einzusetzen. Besonders bevorzugt ist eine Mischung von Dichlormethan und Dimethylformamid.

Die Verbindungen der allgemeinen Formel (V) sind literaturbekannt oder neu und können hergestellt werden, indem Verbindungen der allgemeinen Formel (VII) 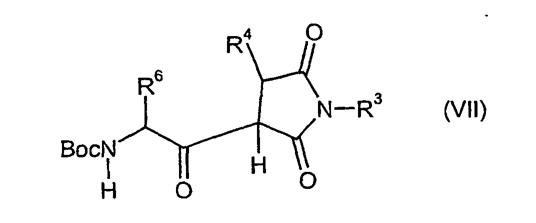 in welcher
R³, R⁴ und R⁶ die oben angegebene Bedeutung aufweisen,
mit Säure, insbesondere mit Salzsäure oder Trifluoressigsäure versetzt werden.

Als Lösemittel eignen sich hierbei inerte organische Lösemittel, die sich unter den Reaktionsbedingungen nicht verändern. Hierzu gehören Halogenkohlenwasserstoffe wie Dichlormethan oder Trichlormethan, Kohlenwasserstoffe wie Benzol, Xylol, Toluol, Hexan, Cyclohexan, oder Erdölfraktionen, Nitromethan, Dimethylformamid oder Acetonitril oder Ether wie Diethylether, Tetrahydrofuran oder Dioxan. Ebenso ist es möglich, Gemische der Lösemittel einzusetzen. Besonders bevorzugt ist die Verwendung von Salzsäure in Dioxan oder Trifluoressigsäure in Dichlormethan.

Die Verbindungen der allgemeinen Formel (VI) sind bekannt oder können nach literaturbekannten Vorschriften hergestellt werden. (Bzgl. der Darstellung von aromatischen beta-Aminosäuren s.: S. Rault, P. Dallemagne, M. Robba, *Bull. Soc. Chim. Fr*., **1987,** 1079-1083).

Die Verbindungen der allgemeinen Formel (VII) sind bekannt oder können nach literaturbekannten Verfahren hergestellt werden. (Vgl. z.B. S. G. Davies, D. J. Dixon, *J. Chem. Soc., Perkin Trans. 1*, **1998**, *17*, 2635-2643.)

Die Verbindungen der allgemeinen Formel (IIIa) sind bekannt oder können nach literaturbekannten Verfahren hergestellt werden.

Die Verbindungen der allgemeinen Formel (IIIb), (IIIc) und (IIId) sind bekannt oder können nach literaturbekannten Verfahren hergestellt werden.

In den allgemeinen Formeln (VI) und (VII) kann die Boc-Gruppe auch durch andere Aminosäureschutzgruppen, wie z.B. Fluorenylmethoxycarbonyl (Fmoc) oder Benzyloxycarbonyl ersetzt werden, die nach Standardmethoden abgespalten werden können (Greene, T.W., Wuts, G.M., Protective Groups in Organic Synthesis, 3^{rd} Ed, Wiley 1999).

Die oben beschriebenen Reaktionen erfolgen im allgemeinen in einem Temperaturbereich von -78°C bis zu Rückflusstemperatur, bevorzugt von -78°C bis +20°C.

Die Umsetzungen können bei normalem, erhöhtem oder bei erniedrigtem Druck durchgeführt werden (z.B. von 0,5 bis 5 bar). Im allgemeinen arbeitet man bei Normaldruck.

Die nachfolgenden Fliessschemata sollen die Verfahren veranschaulichen:

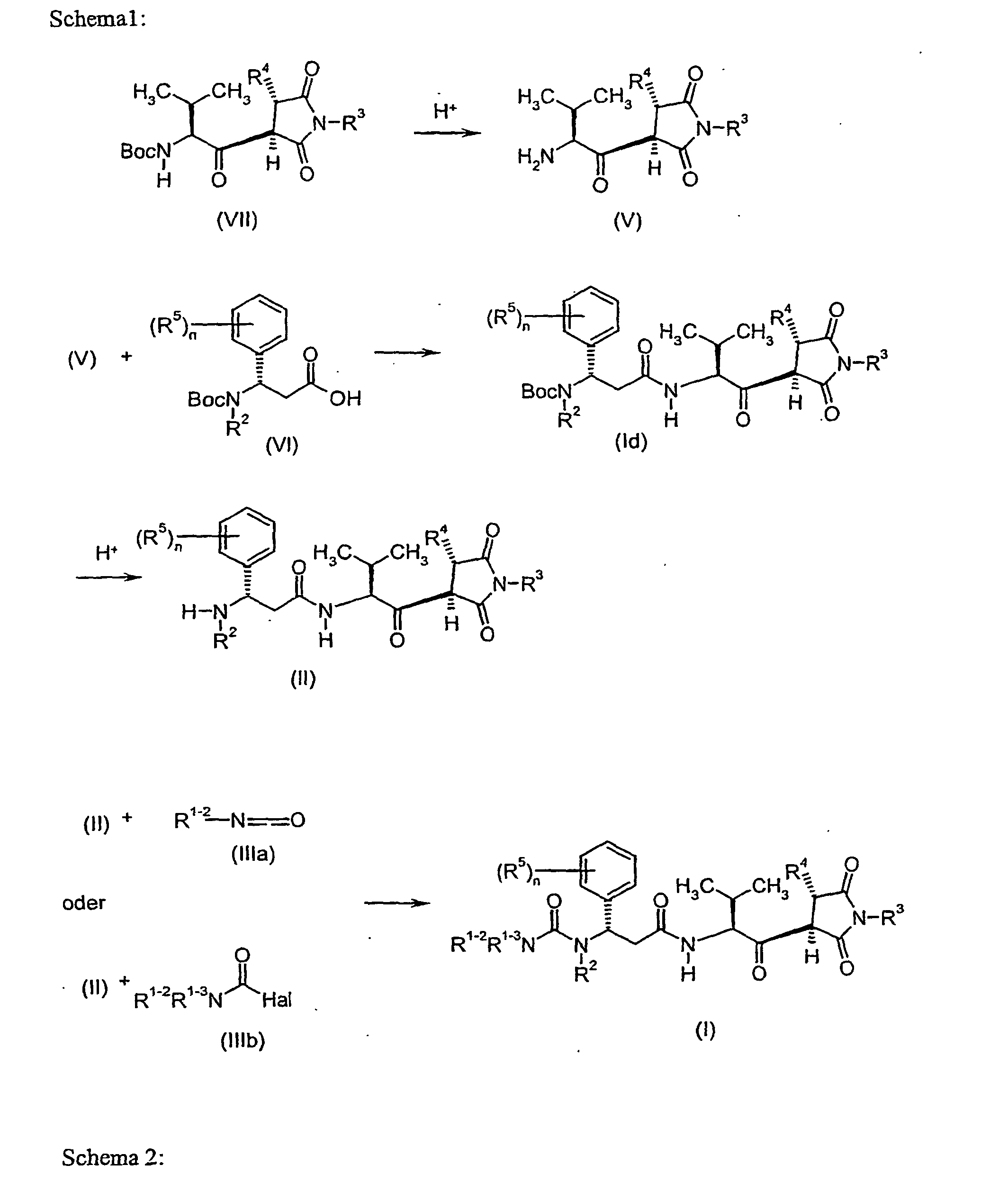

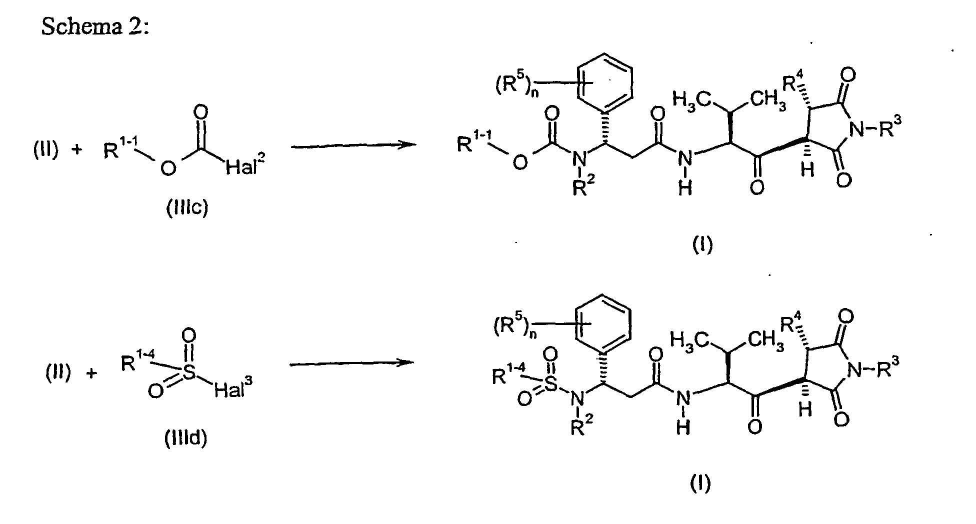

Die vorliegende Erfindung betrifft weiterhin Verbindungen der allgemeinen Formel (I) zur Bekämpfung von Erkrankungen, insbesondere bakterieller Erkrankungen, sowie Arzneimittel, enthaltend Verbindungen der allgemeinen Formel (I) und Hilfsstoffe und auch die Verwendung von Verbindungen der allgemeinen Formel (I) zur Herstellung eines Arzneimittels zur Behandlung von bakteriellen Erkrankungen.

Besonders wirksam sind die erfindungsgemäßen Zubereitungen gegen Bakterien und bakterienähnliche Mikroorganismen. Sie sind daher besonders gut zur Prophylaxe und Chemotherapie von lokalen und systemischen Infektionen in der Human- und Tiermedizin geeignet, die durch diese Erreger hervorgerufen werden.

Beispielsweise können lokale und/oder systemische Erkrankungen behandelt und/oder verhindert werden, die durch die folgenden Erreger oder durch Mischungen der folgenden Erreger verursacht werden:

Gram-positive Kokken, z.B. Staphylokokken (Staph. aureus, Staph. epidermidis), Enterokokken (E. faecalis, E. faecius) und Streptokokken (Strept. agalactiae, Strept. pneumoniae); gram-negative Kokken (Neisseria gonorrhoeae) sowie gram-negative Stäbchen wie Enterobakeriaceen, z.B. Escherichia coli, Hämophilus influenzae, Citrobacter (Citrob. freundii, Citrob. divernis), Salmonella und Shigella; ferner Klebsiellen (Klebs. pneumoniae, Klebs. oxytocy), Enterobacter (Ent. aerogenes, Ent. agglomerans), Hafnia, Serratia (Serr. marcescens), Providencia, Yersinia, sowie die Gattung Acinetobacter. Darüber hinaus umfasst das antibakterielle Spektrum strikt anaerobe Bakterien wie z.B. Bacteroides fragilis, Vertreter der Gattung Peptococcus, Peptostreptococcus sowie die Gattung Clostridium; ferner Mykoplasmen (M. pneumoniae, M. hominis, M. urealyticum) sowie Mykobakterien, z.B. Mycobacterium tuberculosis.

Die obige Aufzählung von Erregern ist lediglich beispielhaft und keineswegs beschränkend aufzufassen. Als Krankheiten, die durch die genannten Erreger oder Mischinfektionen verursacht und durch die erfindungsgemäßen Zubereitungen verhindert, gebessert oder geheilt werden können, seien beispielsweise genannt:

Infektionskrankheiten beim Menschen wie z.B. septische Infektionen, Knochen- und Gelenkinfektionen, Hautinfektionen, postoperative Wundinfektionen, Abszesse, Phlegmone, Wundinfektionen, infizierte Verbrennungen, Brandwunden, Infektionen im Mundbereich, Infektionen nach Zahnoperationen, septische Arthritis, Mastitis, Tonsillitis, Genital-Infektionen und Augeninfektionen.

Außer beim Menschen können bakterielle Infektionen auch bei anderen Spezies behandelt werden. Beispielhaft seien genannt:
Schwein: Coli-diarrhoe, Enterotoxamie, Sepsis, Dysenterie, Salmonellose, Metritis-Mastitis-Agalaktiae-Syndrom, Mastitis;
Wiederkäuer (Rind, Schaf, Ziege): Diarrhoe, Sepsis, Bronchopneumonie, Salmonellose, Pasteurellose, Mykoplasmose, Genitalinfektionen;
Pferd: Bronchopneumonien, Fohlenlähme, puerperale und postpuerperale Infektionen, Salmonellose;
Hund und Katze: Bronchopneumonie, Diarrhoe, Dermatitis, Otitis, Harnwegsinfekte, Prostatitis;
Geflügel (Huhn, Pute, Wachtel, Taube, Ziervögel und andere): Mycoplasmose, E. coli-Infektionen, chronische Luftwegserkrankungen, Salmonellose, Pasteurellose, Psittakose.

Ebenso können bakterielle Erkrankungen bei der Aufzucht und Haltung von Nutz- und Zierfischen behandelt werden, wobei sich das antibakterielle Spektrum über die vorher genannten Erreger hinaus auf weitere Erreger wie z.B. Pasteurella, Brucella, Campylobacter, Listeria, Erysipelothris, Corynebakterien, Borellia, Treponema, Nocardia, Rikettsie, Yersinia, erweitert.

Der Wirkstoff kann systemisch und/oder lokal wirken. Zu diesem Zweck kann er auf geeignete Weise appliziert werden, wie z.B. oral, parenteral, pulmonal, nasal, sublingual, lingual, buccal, rectal, transdermal, conjunctival, otisch oder als Implantat.

Für diese Applikationswege kann der Wirkstoff in geeigneten Applikationsformen verabreicht werden.

Für die orale Applikation eignen sich bekannte, den Wirkstoff schnell und/oder modifiziert abgebende Applikationsformen, wie z.B. Tabletten (nichtüberzogene sowie überzogene Tabletten, z.B. mit magensaftresistenten Überzüge versehene Tabletten oder Filmtabletten), Kapseln, Dragees, Granulate, Pellets, Pulver, Emulsionen, Suspensionen und Lösungen.

Die parenterale Applikation kann unter Umgehung eines Resorptionsschrittes geschehen (intravenös, intraarteriell, intrakardial, intraspinal oder intralumbal) oder unter Einschaltung einer Resorption (intramuskulär, subcutan, intracutan, percutan, oder intraperitoneal). Für die parenterale Applikation eignen sich als Applikationsformen u.a. Injektions- und Infusionszubereitungen in Form von Lösungen, Suspensionen, Emulsionen, Lyophilisaten und sterilen Pulvern.

Bevorzugt ist die parenterale, insbesondere die intravenöse Applikation.

Für die sonstigen Applikationswege eignen sich z.B. Inhalationsarzneiformen (u.a. Pulverinhalatoren, Nebulizer), Nasentropfen / -lösungen, Sprays; lingual, sublingual oder buccal zu applizierende Tabletten oder Kapseln, Suppositorien, Ohren- und Augenpräparationen, Vaginalkapseln, wässrige Suspensionen (Lotionen, Schüttelmixturen), lipophile Suspensionen, Salben, Cremes, Milch, Pasten, Streupuder oder Implantate.

Die Wirkstoffe können in an sich bekannter Weise in die angeführten Applikationsformen überführt werden. Dies geschieht unter Verwendung inerter nichttoxischer, pharmazeutisch geeigneter Hilfsstoffe. Hierzu zählen u.a. Trägerstoffe (z.B. mikrokristalline Cellulose), Lösungsmittel (z.B. flüssige Polyethylenglycole), Emulgatoren (z.B. Natriumdodecylsulfat), Dispergiermittel (z.B. Polyvinylpyrrolidon), synthetische und natürliche Biopolymere (z.B. Albumin), Stabilisatoren (z.B. Antioxidantien wie Ascorbinsäure), Farbstoffe (z.B. anorganische Pigmente wie Eisenoxide) oder Geschmacks- und / oder Geruchskorrigentien.

Im Allgemeinen hat es sich als vorteilhaft erwiesen, bei parenteraler Applikation Mengen von etwa 5 bis 250 mg/kg Körpergewicht je 24 Stunden zur Erzielung wirksamer Ergebnisse zu verabreichen. Bei oraler Applikation beträgt die Menge etwa 5 bis 100 mg/kg Körpergewicht je 24 Stunden.

Trotzdem kann es gegebenenfalls erforderlich sein, von den genannten Mengen abzuweichen, und zwar in Abhängigkeit von Körpergewicht, Applikationsweg, individuellem Verhalten gegenüber dem Wirkstoff, Art der Zubereitung und Zeitpunkt bzw. Intervall, zu welchem die Applikation erfolgt.

### A. Bewertung der physiologischen Wirksamkeit

### Bestimmung der Minimalen Hemmkonzentration (MHK):

Die MHK wird im Flüssigdilutionstest bestimmt. Übernachtkulturen der Testkeime werden auf eine Zellzahl von 10⁵ Keimen pro ml in Isosensitest-Medium (Hersteller: Difco) verdünnt und mit Verdünnungen der Testsubstanzen (Verdünnungsstufen 1:2) inkubiert. Ausnahmen sind die Tests mit S. pneumoniae G9A, die in BHI-Bouillon (Difco) plus 20 % Rinderserum, und mit H. influenzae, die in BHI-Bouillon (Difco) plus 20 % Rinderserum, 10 µg/ml Haemin und 1 % Isovitale (Becton Dickinson) durchgeführt werden.

Die Kulturen werden bei 37°C für 18-24 Stunden inkubiert; S. pneumoniae und H. influenzae in Gegenwart von 8 -10 % CO₂.

### Ergebnisse:

Die jeweils niedrigste Substanzkonzentration, bei der kein sichtbares Bakterienwachstum mehr auftrat, wird als MHK definiert. Die MHK-Werte in µmol/l einiger erfindungsgemäßer Verbindungen gegenüber einer Reihe von Testkeimen sind in der nachstehenden Tabelle beispielhaft aufgeführt.

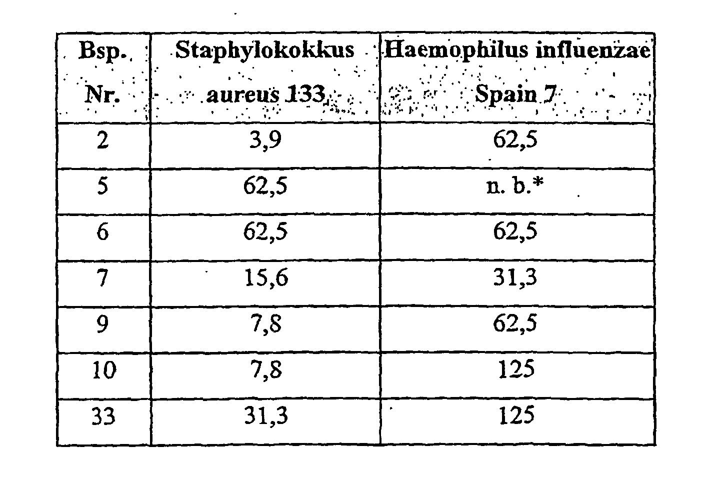

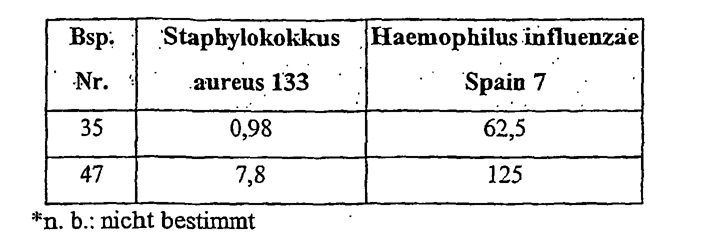

### Systemische Infektion mit S.aureus 133

S.aureus 133 Zellen werden über Nacht in BH-Bouillon (Oxoid) augezüchtet. Die Übernachtkultur wird 1:100 in frische BH-Bouillon verdünnt und für 3 Stunden hochgedreht. Die in der logarithmischen Wachstumsphase befindlichen Bakterien werden abzentrifugiert und 2 x mit gepufferter, physiologischer Kochsalzlösung gewaschen. Danach wird am Photometer (Dr. Lange LP 2W) eine Zellsuspension in Kochsalzlösung mit einer Extinktion von 50 Einheiten eingestellt. Nach einem Verdünnungsschritt (1:15) wird diese Suspension 1:1 mit einer 10 %igen Mucinsuspension gemischt. Von dieser Infektionslösung wird 0,25 ml/20 g Maus i.p. appliziert. Dies entspricht einer Zellzahl von etwa 1 x 10E⁶ Keimen/Maus. Die i.p.- oder i.v.-Therapie erfolgt 30 Minuten nach der Infektion. Für den Infektionsversuch werden weibliche CFW1-Mäuse verwendet. Das Überleben der Tiere wird über 6 Tage protokolliert.

### B. Beispiele

### Abkürzungen:

- Boc: tert.-Butoxycarbonyl
- CDCl₃: Deuterochloroform
- DIEA: *N*,*N*-Diisopropylethylamin
- DMSO: Dimethylsulfoxid
- d. Th.: der Theorie
- EDC: *N*'-(3-Dimethylaminopropyl)-*N*-ethylcarbodiimid x HCl
- eq.: Äquivalent
- ESI: Elektrospray-Ionisation (bei MS)
- Fmoc: Fluorenylmethoxycarbonyl
- HATU: *O*-(7-Azabenzotriazol-1-yl)-*N*,*N*,*N*',*N*'-tetramethyluronium-Hexafluorphosphat
- h: Stunde
- HOBt: 1-Hydroxy-1H-benzotriazol x H₂O
- HPLC: Hochdruck-, Hochleistungsflüssigchromatographie
- LC-MS: Flüssigchromatographie-gekoppelte Massenspektroskopie
- MS: Massenspektroskopie
- NMR: Kernresonanzspektroskopie
- PS-DIEA: *N*,*N*-Diisopropylethylamin-Polystyrol (-Harz)
- R_{f}: Retentionsindex (bei DC)
- RP-HPLC: Reverse Phase HPLC
- RT: Raumtemperatur
- Rₜ: Retentionszeit (bei HPLC)

### HPLC und LC-MS Methoden:

### Methode 1:

Säule: Kromasil C18, L-R Temperatur: 30°C, Fluss = 0.75 mlmin⁻¹, Eluent: A = 0.01 M HClO₄, B = Acetonitril, Gradient: → 0.5 min 98 %A → 4.5 min 10 %A → 6.5 min 10 %A

### Methode 2:

Säule: Kromasil C18 60*2, L-R Temperatur: 30°C, Fluss = 0.75 mlmin⁻¹, Eluent: A = 0.01 M H₃PO₄, B = Acetonitril, Gradient: → 0.5 min 90 %A → 4.5 min 10 %A → 6.5 min 10 %A

### Methode 3:

Säule: Kromasil C18 60*2, L-R Temperatur: 30°C, Fluss = 0.75 mlmin-¹, Eluent: A = 0.005 M HClO₄, B = Acetonitril, Gradient: → 0.5 min 98 %A → 4.5 min 10 %A → 6.5 min 10 %A

### Methode 4:

Säule: Symmetry C18 2.1x150 mm, Säulenofen: 50°C, Fluss = 0.6 mlmin⁻¹, Eluent: A = 0.6 g 30 %ige HCl/l Wasser, B = Acetonitril, Gradient: 0.0 min 90 %A → 4.0 min 10 %A → 9 min 10 %A

### Methode 5:

Instrument Micromass Quattro LCZ; Säule Symmetry C18, 50 mm x 2.1 mm, 3.5 µm, Temperatur: 40°C, Fluss = 0.5 mlmin⁻¹, Eluent A = Acetonitril + 0.1% Ameisensäure, Eluent B = Wasser + 0.1 % Ameisensäure, Gradient: 0.0 min 10 % A → 4 min 90 % A → 6 min 90 % A

### Methode 6:

Instrument Micromass Platform LCZ; Säule Symmetry C18, 50 mm x 2.1 mm, 3.5 µm, Temperatur: 40°C, Fluss = 0.5 mlmin⁻¹, Eluent A = Acetonitril + 0.1 % Ameisensäure, Eluent B = Wasser + 0.1 % Ameisensäure, Gradient: 0.0 min 10 % A → 4 min 90 % A → 6 min 90 % A

### Methode 7:

Instrument Micromass Quattro LCZ; Säule Symmetry C18, 50 mm x 2.1 mm, 3.5 µm, Temperatur: 40°C, Fluss = 0.5 mlmin⁻¹, Eluent A = Acetonitril + 0.1 % Ameisensäure, Eluent B = Wasser + 0.1 % Ameisensäure, Gradient: 0.0 min 5 % A → 1 min 5 % A → 5 min 90 % A → 6 min 90 % A

### Methode 8:

Instrument Finnigan MAT 900S; Säule: Symmetry C 18, 150 mm x 2.1 mm, 5.0 µm; Eluent B: Wasser + 0.3g 35%ige HCl, Eluent A: Acetonitril; Gradient: 0.0min 2%A → 2.5min 95%A → 5min 95%A; Ofen: 70 °C, Fluss: 1.2ml/min.

### Methode 9:

Säule: Symmetry C18 3.9 mm x 150 mm, Säulenofen: 40°C, Fluss = 1.5 mlmin⁻¹, Eluent: A = Wasser + 0.05% H₃PO₄, , B = Acetonitril, Gradient: 0.0 min 10% B → 0.6 min 10% B → 3.8 min 100% B → 5.0 min 100% B.

### Methode 10:

Instrument: Waters Alliance 2790 LC; Säule: Symmetry C18, 50 mm x 2.1 mm, 3.5 µm; Eluent A: Wasser + 0.1% Ameisensäure, Eluent B: Acetonitril + 0.1% Ameisensäure; Gradient: 0.0 min 5% B → 5.0 min 10% B → 6.0 min 10% B; Temperatur: 50°C, Fluss: 1.0 ml/min, UV-Detektion: 210 nm.

### Methode 11:

Gerätetyp MS: Micromass ZQ; Gerätetyp HPLC: Waters Alliance 2790; Säule: Symmetry C 18, 50 mm x 2.1 mm, 3.5 µm; Eluent B: Acetonitril + 0.05% Ameisensäure, Eluent A: Wasser + 0.05% Ameisensäure; Gradient: 0.0 min 10%B → 3.5 min 90%B → 5.5 min 90% B; Ofen: 50 °C, Fluss: 0.8 ml/min, UV-Detektion: 210 nm.

### Methode 12:

Instrument: Waters Alliance 2790 LC; Säule: Symmetry C18, 50 mm x 2.1 mm, 3.5 µm; Eluent A: Wasser + 0.05% Ameisensäure, Eluent B: Acetonitril + 0.05% Ameisensäure; Gradient: 0.0 min 5% B → 4.5 min 10% B → 5.5 min 10% B; Temperatur: 50°C, Fluss: 1.0 ml/min, UV-Detektion: 210 nm.

### Methode 13:

Instrument: Micromass Quattro LCZ, HP1100; Säule: Symmetry C18, 50 mm x 2.1 mm, 3.5 µm; Eluent A: Wasser + 0.05% Ameisensäure, Eluent B: Acetonitril + 0.05% Ameisensäure; Gradient: 0.0 min 90% A → 4.0 min 10% A → 6.0min 10% A; Ofen: 40°C, Fluss: 0.5 ml/min, UV-Detektion: 208-400 nm.

### Methode 14:

Instrument: Micromass Platform LCZ, HP1100; Säule: Symmetry C18, 50 mm x 2.1 mm, 3.5 µm; Eluent A: Wasser + 0.05% Ameisensäure, Eluent B: Acetonitril + 0.05% Ameisensäure; Gradient: 0.0 min 90%A → 4.0 min 10% A → 6.0 min 10% A; Ofen: 40°C, Fluss: 0.5 ml/min, UV-Detektion: 208-400 nm.

### Methode 15:

Instrument: Waters Alliance 2790 LC; Säule: Symmetry C18, 50 mm x 2.1 mm, 3.5 µm; Eluent A: Wasser + 0.05% Ameisensäure, Eluent B: Acetonitril + 0.05% Ameisensäure; Gradient: 0.0 min 10% B → 4.0 min 90% B → 6.0 min 90% B; Temperatur: 50°C, Fluss: 0.0 min 0.5 ml/min → 4.0 min 0.8 ml/min, UV-Detektion: 210 nm.

### Methode 16:

Gerätetyp MS: Micromass ZQ; Gerätetyp HPLC: Waters Alliance 2790; Säule: Symmetry C 18, 50 mm x 2.1 mm, 3.5 µm; Eluent B: Acetonitril + 0.05% Ameisensäure, Eluent A: Wasser + 0.05% Ameisensäure; Gradient: 0.0min 5%B 4.5min 90%B 5.5min 90%B; Ofen: 50 °C, Fluss: 1.0ml/, UV-Detektion: 210 nm

### Methode 17:

Gerätetyp MS: Micromass ZQ; Gerätetyp HPLC: Waters Alliance 2790; Säule: Uptisphere C 18, 50 mm x 2.0 mm, 3.0 µm; Eluent B: Acetonitril + 0.05% Ameisensäure, Eluent A: Wasser + 0.05% Ameisensäure; Gradient: 0.0min 5%B → 2.0min 40%B → 4.5min 90%B→ 5.5min 90%B; Ofen: 45 °C, Fluss: 0.0min 0.75ml/min → 4.5min 0.75ml/min→ 5.5min 1.25ml/min, UV-Detektion: 210 nm

### Ausgangsverbindungen

### Beispiel 1A

### (3R,4S)-3-[(2S)-2-Amino-3-methylbutanoyl]-4-methyl-2,5-pyrrolidindion Hydrochlorid

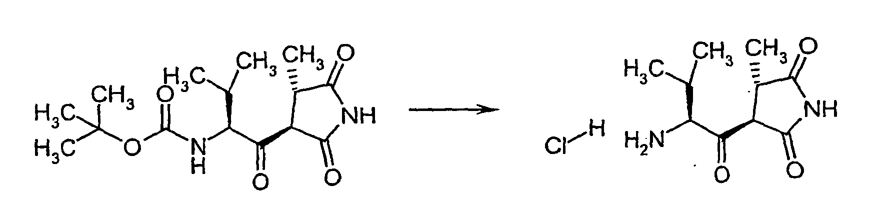

Zu einer auf 0°C abgekühlten Lösung von 4.40 g (14.09 mmol) (3*R*,4*S*)-3-[(2*S*)-2-(*tert*-Butoxycarbonyl)amino-3-methylbutanoyl]-4-methyl-2,5-pyrrolidindion (Darstellung: S. G. Davies, D. J. Dixon, *J. Chem. Soc., Perkin Trans. 1,* **1998,** 17, 2635 - 2643) in 20 ml Dioxan werden 35 ml 4N Salzsäure-Lösung in 1,4-Dioxan getropft. Nach Ende der Zugabe wird die Mischung auf Raumtemperatur erwärmt und 2 h gerührt, bevor die Mischung im Vakuum eingeengt wird. Der Rückstand wird mit Diethylether behandelt. Die ausgefallenen Kristalle werden abfiltriert und am Hochvakuum getrocknet. Ausbeute: 2.99 g farblose Kristalle (86 % der Theorie).
MS (ESI+): m/z (%) = 213 (M+H⁺) (100).
HPLC (Methode 4): Rₜ = 0.41 min.

### Allgemeine Vorschrift A

### Acylierung von (3R,4S)-3-[(2S)-2-Amino-3-methylbutanoyl]-4-methyl-2,5-pyrrolidindion Hydrochlorid mit Carbonsäurederivaten

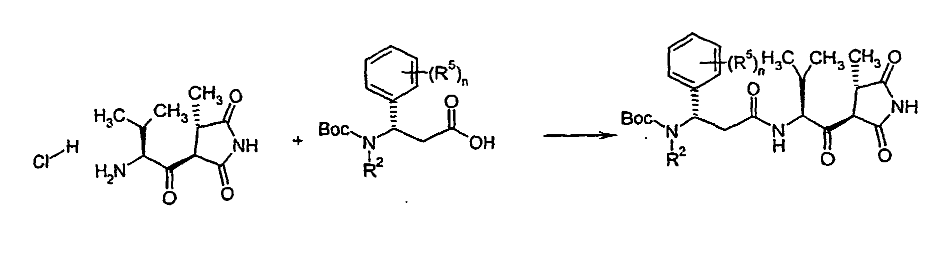

Zu einer Lösung von (3*R*,4*S*)-3-[(2*S*)-2-Amino-3-methylbutanoyl]-4-methyl-2,5-pyrrolidindion Hydrochlorid (1.0 eq.) in einer Mischung (ca. 5:1) von absolutem Dichlormethan und N,N-Dimethylformamid (ca. 0.2 bis 0.35 mol/l) werden ca. 1.2 bis 1.4 eq. N-blockiertes β-Aminosäurederivat gegeben. Die Mischung wird auf 0°C gekühlt und mit 1.2 bis 1.4 eq. HATU versetzt. Über 30 min wird tropfenweise mit 2.3 bis 2.6 eq. Diisopropylethylamin versetzt. Nach Ende der Zugabe wird die Reaktionsmischung 3 bis 5 h bei Raumtemperatur gerührt, bevor im Vakuum eingeengt wird. Das Produkt kann durch Chromatographie an Silicagel [Eluenten: Gemische aus Cyclohexan/Ethylacetat (ca. 1:2) oder Gemische aus Dichlormethan und Ethanol (ca. 98:2)] oder durch RP-HPLC (Eluenten: variable Gradienten aus Wasser und Acetonitril), alternativ durch eine Kombination beider Verfahren, erhalten werden.

### Beispiel 2A

### ((S)-2-{(S)-2-Methyl-1-[1-((3R,4S)-4-methyl-2,5-dioxo-pyrrolidin-3-yl)-methanoyl]-propylcarbamoyl}-1-phenyl-ethyl)-carbamidsäure-tert-butylester

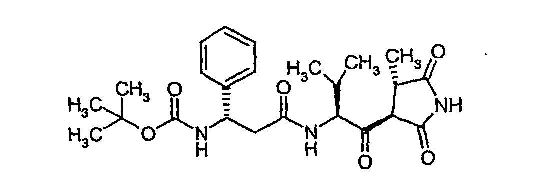

¹H-NMR (400 MHz, d₆-DMSO): δ = 11.45 (s, 1 H), 7.98 (d, 1 H), 7.31-7.24 (m, 5 H), 7.20 (br. s, 1 H), 4.88-4.82 (br. s, 1 H), 4.69 (br. s, 1 H), 3.98 (d, 1 H), 2.95-2.89 (m, 1 H), 2.77-2.69 (m, 1 H), 2.51-2.44 (m, 1 H), 2.35-2.29 (m, 1 H), 1.10 (d, 3 H), 0.85 (d, 3 H), 0.78 (d, 3 H).
MS (ESI+): m/z (%) = 460 (M+H⁺) (100).
HPLC (Methode 6): Rₜ = 3.90 min.

### Allgemeine Vorschrift B

### Deblockierung von Boc-geschützten Derivaten:

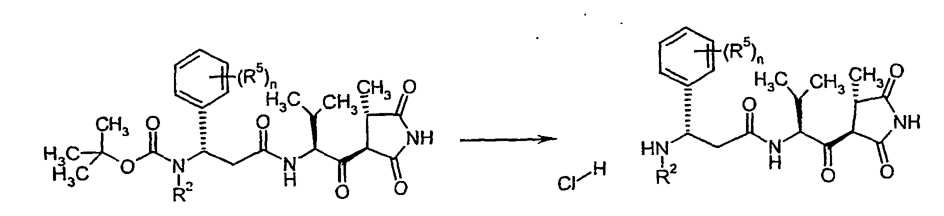

Eine auf 0°C gekühlte Mischung von *tert*.-Butoxycarbonyl (BOC) geschütztem Aminderivat in 1,4-Dioxan (ca. 0.5 bis 1.0 mol/l) wird tropfenweise über 30 min mit 3 - 5 eq 4N Salzsäure-Lösung in 1,4-Dioxan versetzt. Nach Ende der Zugabe wird die Mischung auf Raumtemperatur erwärmt und ca. 2 bis 3 h gerührt, bevor im Vakuum eingeengt wird. Der Rückstand wird mit einem Gemisch aus Dichlormethan und Diethylether (ca. 1:2) behandelt. Die ausgefallenen Kristalle werden abgesaugt und am Hochvakuum getrocknet. Man erhält das Produkt als Hydrochlorid.

### Beispiel 3A

### (S)-3-Amino-{(S)-2-methyl-1-[1-((3R,4S)-4-methyl-2,5-dioxo-pyrrolidin-3-yl)-methanoyl]-propyl}-3-phenyl-propionamid Hydrochlorid

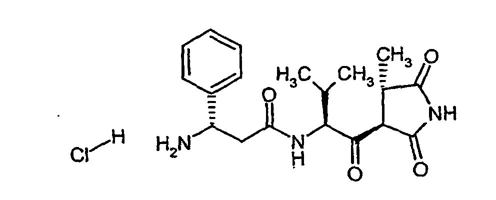

¹H-NMR (200 MHz, d₆-DMSO): δ = 11.49 (br. s, 1 H), 8.5 (br. s, ca. 3 H), 7.54-7.32 (m, 5 H), 4.69-4.55 (m, 2 H), 3.89 (d, 1 H), 3.06-2.80 (m, 3 H), 2.39-2.25 (m, 1 H), 1.01 (d, 3 H), 0.81 (d, 3 H), 0.75 (d, 3 H).
MS (ESI+): m/z (%) = 360 (M-Cl)⁺ (100).
HPLC (Methode 4): Rₜ = 1.44 min.

Analog zu Beispiel 1A können aus den entsprechenden *tert*.-Butoxycarbonylamino-Derivaten durch Behandlung mit Salzsäure in Dioxan folgende Amine in Form ihrer Hydrochloride dargestellt und direkt weiter umgesetzt werden:

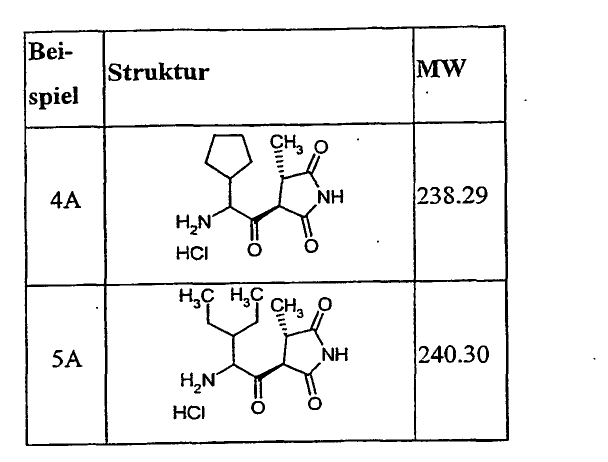

### Allgemeine Vorschrift E

### Darstellung von N-tert-butoxycarbonyl-geschützten beta-Aminosäuren

Die beta-Aminosäure (1 eq.) [Synthese nach literaturbekannten Vorschriften (z. B. S. Rault, P. Dallemagne, M. Robba, *Bull. Soc. Chim. Fr.,* **1987,** 1079-1083; L. Läzär, T. Martinek, G. Bernáth, F. Fülöp, *Synth. Comm.,* **1998,** 28, 219-224)] wird in Wasser vorgelegt (Konzentration ca. 0.3 - 1 mol/l) und mit Triethylamin (1.5 - 3 eq.) versetzt. Dann wird eine Lösung von 2-(*tert*-Butoxycarbonyloximino)-phenylacetonitril (1.1 eq.) in Dioxan (0.3 - 1 mol/l) zugegeben. Das Reaktionsgemisch wird 3 h bei Raumtemperatur gerührt, mit Wasser verdünnt und mit Diethylether gewaschen. Die wässrige Phase wird mit 5 %iger Zitronensäure angesäuert (ca. pH 2) und dreimal mit Essigsäureethylester extrahiert. Die vereinigten organischen Phasen werden mit gesättigter Kochsalzlösung gewaschen, über Natriumsulfat getrocknet, filtriert und eingeengt. Das Rohprodukt kann gegebenenfalls aus Essigsäureethylester/n-Hexan umkristallisiert werden.

Nach der Allgemeinen Vorschrift E können folgende Verbindungen erhalten werden:

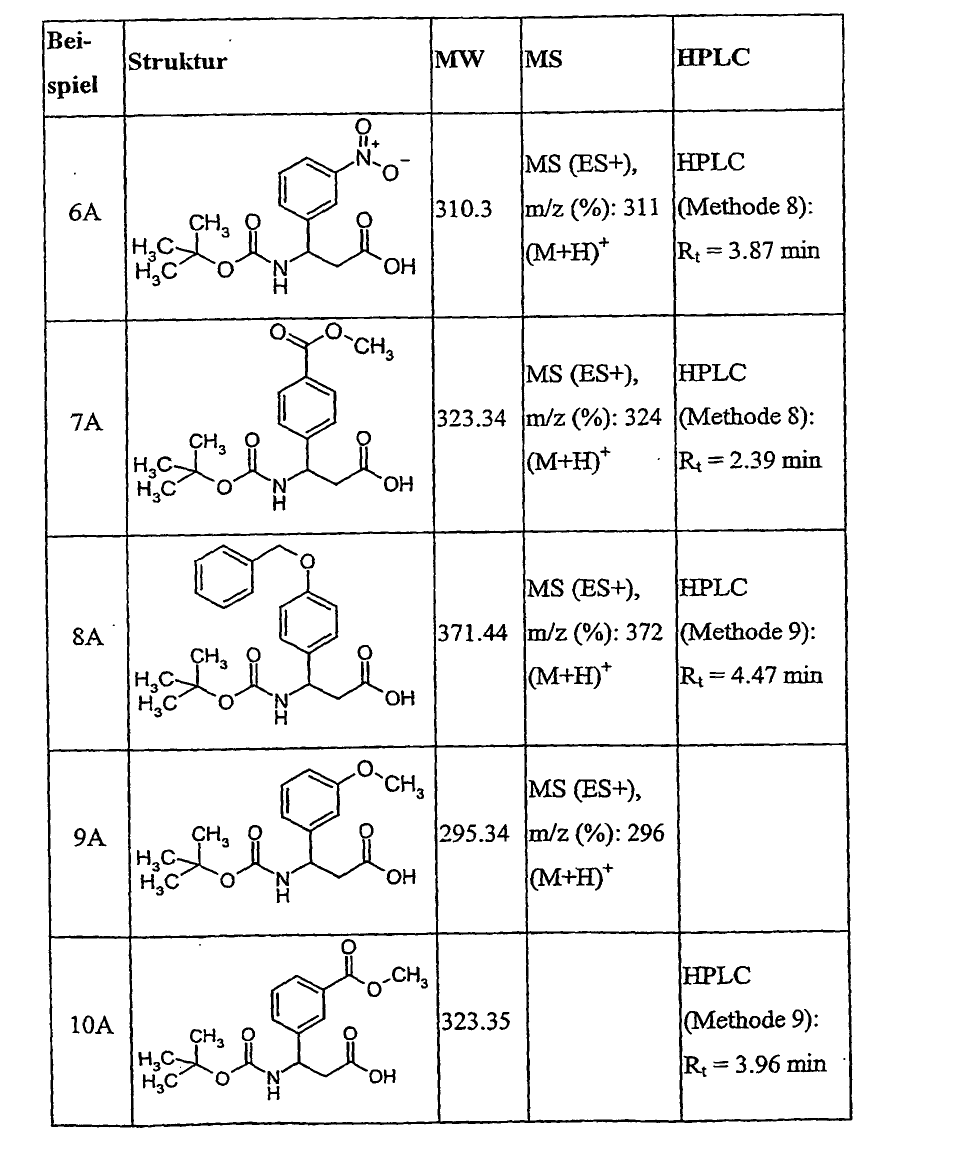

### Allgemeine Vorschrift F

### Umsetzung von (3S)-1-(Benzyloxy)-3-methyl-2,5-pyrrolidindion mit aktivierten N-(tert-Butoxycarbonyl)-Aminosäuren

Die N-(*tert*-Butoxycarbonyl)-Aminosäure wird in Tetrahydrofuran vorgelegt (ca. 0.3 - 1 mol/l) und mit 1.1 eq N,N-Carbonyldiimidazol versetzt. Das Gemisch wird 2 h bei Raumtemperatur gerührt. Dann wird 1 eq (3*S*)-1-(Benzyloxy)-3-methyl-2,5-pyrrolidindion (Darstellung: S. G. Davies, D. J. Dixon, *J. Chem. Soc., Perkin Trans. 1*, **1998,** *17*, 2635 - 2643) zugegeben und die gesamte Mischung wird innerhalb von 30 min zu einer auf -65°C gekühlten 1 molaren Lösung von Lithium-hexamethyldisilazid (2 eq) in Tetrahydrofuran getropft. Nach beendeter Zugabe wird weitere 15 min bei -65°C gerührt, bevor gesättigte wässrige AmmoniumchloridLösung zugegeben wird. Die organische Phase wird über Magnesiumsulfat getrocknet, filtriert und eingeengt. Das Rohprodukt wird durch RP-HPLC (Eluent: Wasser-Acetonitril, Gradient) gereinigt

Nach der Allgemeinen Vorschrift F können folgende Verbindungen erhalten werden:

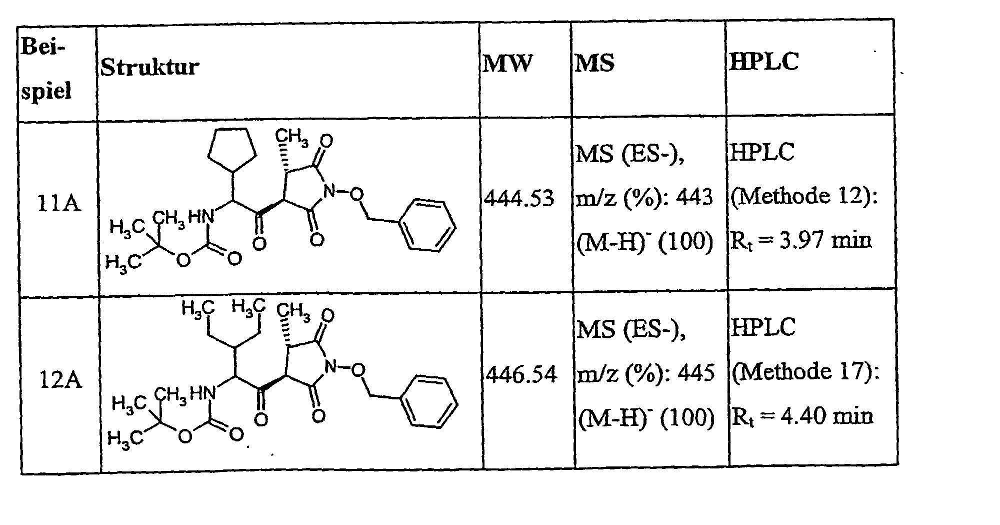

### Allgemeine Vorschrift G

### Reduktive Entschützung von 1-Benzyloxy-2,5-pyrrolidindionen

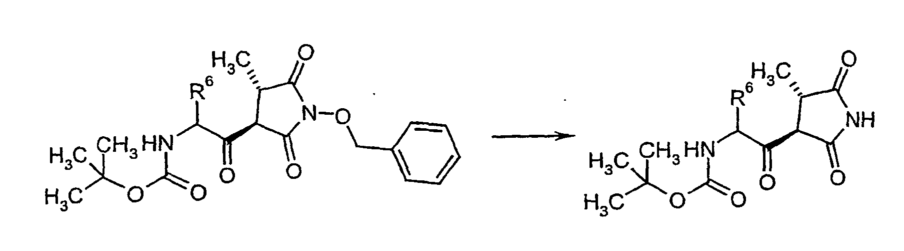

Die Entschützung erfolgt analog zu S. G. Davies, D. J. Dixon, *J. Chem. Soc., Perkin Trans. 1*, **1998**, *17*, 2635 - 2643.
Das 1-Benzyloxy-2,5-pyrrolidindion (1 eq) wird in Methanol gelöst (ca. 0.02 mol/l), mit einer katalytischen Menge Palladium-Kohle (10%ig) versetzt und 1 h unter Wasserstoffatmosphäre (Normaldruck) gerührt. Dann wird das Reaktionsgemisch filtriert und eingeengt. Der Rückstand wird in Acetonitril gelöst (ca. 0.05 mol/l) und zu einer Lösung von 2-Bromacetophenon (1 eq) in Acetonitril (ca. 0.03 mol/l) bei Raumtemperatur getropft. Danach werden über einen Zeitraum von 2 h 1.5 eq Triethylamin in Acetonitril (ca. 0.35 mol/l) zu der Reaktionsmischung getropft. Das Reaktionsgemisch wird über Nacht bei Raumtemperatur gerührt, eingeengt und das Rohprodukt wird mittels RP-HPLC (Eluent: Acetonitril/Wasser + 0.3 ml 37%ige Salzsäure/l, Gradient) gereinigt.

Nach der Allgemeinen Vorschrift G können folgende Verbindungen erhalten werden:

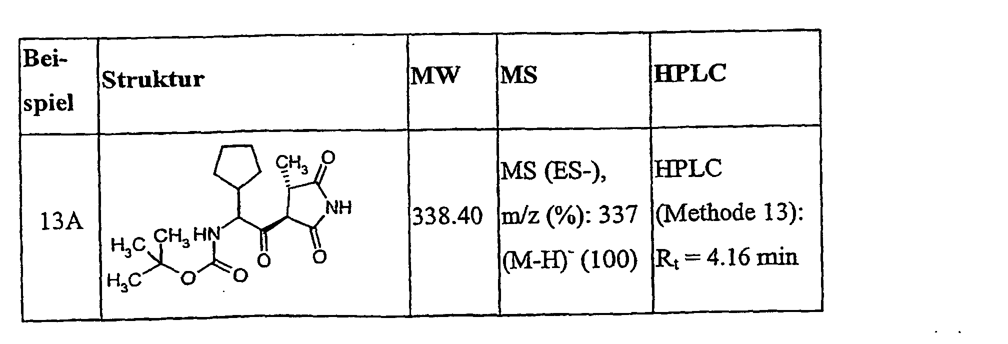

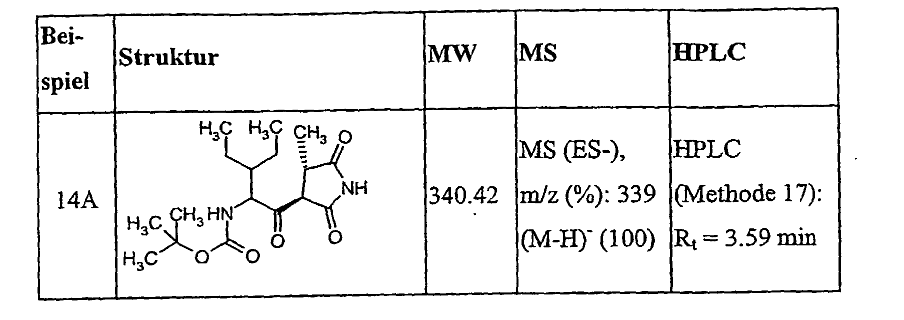

### Herstellungsbeispiele

### Allgemeine Vorschrift C

Darstellung von Harnstoffderivaten ausgehend von substituierten (3*R*,4*S*)-3-[(2*S*)-2-Amino-3-methylbutanoyl]-4-methyl-2,5-pyrrolidindion Derivaten (in Form ihrer Hydrochloride) und Isocyanaten

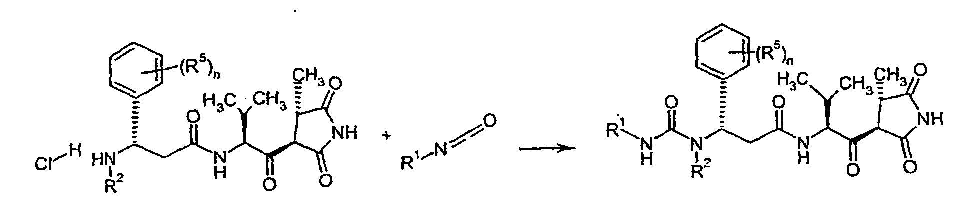

Zu einer Mischung aus Hydrochlorid (1.0 eq.) in absolutem Dichlorethan (ca. 0.1 mol/l, gegebenenfalls unter Zusatz geringer Mengen an N,N-Dimethylformamid) werden bei Raumtemperatur ca. 1.5 eq. polymergebundene Aminbase (PS-DIEA von Argonaut Technologies, Beladung ca. 3.8 mmol/g Harz) und Isocyanat (ca. 1-1.2 eq.) hinzugefügt. Die Mischung wird über Nacht bei Raumtemperatur geschüttelt und filtriert. Das Filtrat wird auf eine Silicagel-Kartusche gegeben. Das Produkt kann mit Gemischen aus Dichlormethan und Methanol (typischerweise 95:5) eluiert werden. Gegebenenfalls kann eine weitere Reinigung durch RP-HPLC erfolgen.

Alternativ können auch das Amin-Hydrochlorid und Triethylamin (1 eq.) in Tetrahydrofuran (ca. 0.1 mol/l) vorgelegt und mit dem in wenig Tetrahydrofuran gelösten Isocyanat bei Raumtemperatur versetzt werden. In diesem Fall wird das Reaktionsgemisch nach Rühren über Nacht mit Dichlormethan verdünnt und mit Wasser gewaschen. Die organische Phase wird über Magnesiumsulfat getrocknet, filtriert und eingeengt. Das Rohprodukt kann durch RP-HPLC (Eluent: Wasser/Acetonitril, Gradient) gereinigt werden.

### Beispiel 1

### (S)-3-(3-Butyl-ureido)-N-{(S)-2-methyl-1-[1-((3R,4S)-4-methyl-2,5-dioxo-pyrrolidin-3-yl)-methanoyl]-propyl}-3-phenyl-propionamid

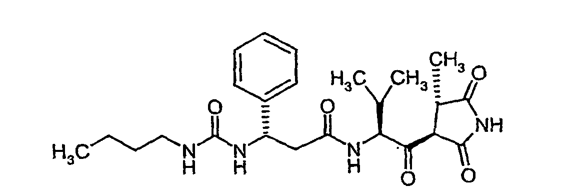

¹H-NMR (400 MHz, d₆-DMSO): δ = 11.35 (s, 1 H), 8.10 (d, 1 H), 7.30-7.16 (m, 6 H), 6.43 (d, 1 H), 6.05 (t, 1 H), 5.09-5.01 (m, 1 H), 4.53 (dd, 1 H), 3.92 (d, 1 H), 2.98-2.90 (m, ca. 1 H), 2.78 (dd, 1 H), 2.56 (dd, 1 H), 2.28-2.23 (m, 1 H), 1.35-1.20 (m, 6 H), 1.10 (d, 3 H), 0.85 (t, 3 H), 0.75 (d, 3 H), 0.70 (d, 3 H).
MS (ESI+): m/z (%) = 459 (M+H⁺) (100).
HPLC (Methode 4): Rₜ = 2.22 min.

### Beispiel 2

### (S)-3-(3-Benzyl-ureido)-N-{(S)-2-methyl-1-[1-((3R,4S)-4-methyl-2,5-dioxo-pyrrolidin-3-yl)-methanoyl]-propyl}-3-phenyl-propionamid

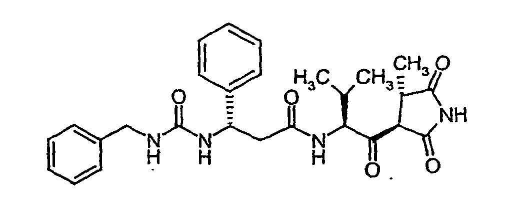

MS (ESI+): m/z (%) = 493 (M+H⁺) (100).
HPLC (Methode 4): Rₜ = 2.28 min.

### Beispiel 3

### (S)-3-(3-Allyl-ureido)-N-{(S)-2-methyl-1-[1-((3R,4S)-4-methyl-2,5-dioxo-pyrrolidin-3-yl)-methanoyl]-propyl}-3-phenyl-propionamid

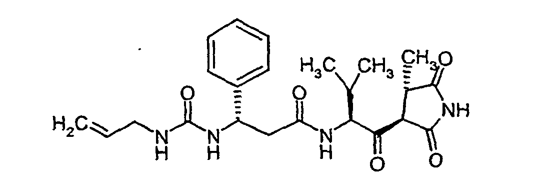

MS (ESI+): m/z (%) = 443 (M+H⁺) (100).
HPLC (Methode 5): Rₜ = 3.23 min.

### Beispiel 4

### (S)-3-(3-Biphenyl-4-yl-ureido)-N-{(S)-2-methyl-1-[1-((3R,4S)-4-methyl-2,5-dioxo-pyrrolidin-3-yl)-methanoyl]-propyl}-3-phenyl-propionamid

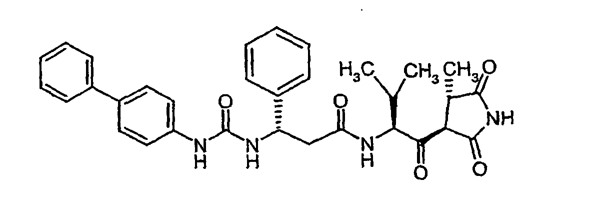

¹H-NMR (400 MHz, d₆-DMSO): δ = 11.32 (br s, 1 H), 8.87 (s, 1 H), 8.15 (d, 1 H), 7.65-7.15 (m, 14 H), 6.99 (d, 1 H), 5.21-5.10 (m, 1 H), 4.57 (dd, 1 H), 3.94 (d, 1 H), 2.95-2.85 (m, 2 H), 2.74 - 2.63 (m, 1 H), 2.31-2.18 (m, 1 H), 1.10 (d, 3 H), 0.72 (d, 3 H), 0.65 (d, 3 H).
MS (ESI+): m/z (%) = 555 (M+H⁺) (100).
HPLC (Methode 6): Rₜ = 4.31 min.

### Beispiel 5

### (S)-N-{(S)-2-Methyl-1-[1-((3R,4S)-4-methyl-2,5-dioxo-pyrrolidin-3-yl)-methanoyl]-propyl}-3-phenyl-3-(3-phenyl-ureido)-propionamid

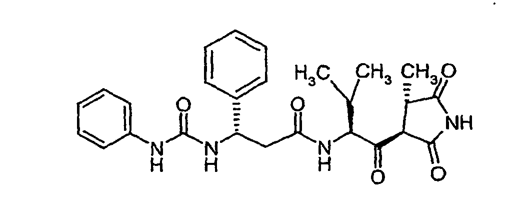

¹H-NMR (400 MHz, d₆-DMSO): δ = 11.32 (br s, 1 H), 8.74 (s, 1 H), 8.15 (d, 1 H), 7.48-7.15 (m, 9 H), 6.99-6.82 (m, 2 H), 5.20-5.10 (m, 1 H), 4.56 (dd, 1 H), 3.95 (d, 1 H), 2.95-2.85 (m, 2 H), 2.72-2.62 (m, 1 H), 2.29-2.18 (m, 1 H), 1.10 (d, 3 H), 0.71 (d, 3 H), 0.68 (d, 3 H).
MS (ESI+): m/z (%) = 501 (M+Na⁺) (100).
HPLC (Methode 5): Rₜ = 3.71 min.

### Beispiel 6

### (S)-3-[3-(4-Methoxy-phenyl)-ureido]-N-{(S)-2-methyl-1-[1-((3R,4S)-4-methyl-2,5-dioxo-pyrrolidin-3-yl)-methanoyl]-propyl}-3-phenyl-propionamid

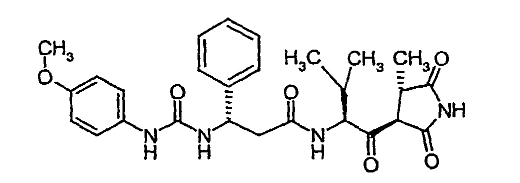

¹H-NMR (400 MHz, d₆-DMSO): δ = 11.35 (s, 1 H), 8.55 (s, 1 H), 8.16 (d, 1 H), 7.33-7.18 (m, 7 H), 6.88-6.75 (m, 3 H), 5.18-5.10 (m, 1 H), 4.57 (dd, 1 H), 3.94 (d, 1 H), 3.68 (s, 3 H), 2.95-2.82 (m, 2 H), 2.70-2.60 (m, 1 H), 2.35-2.20 (m, 1 H), 1.09 (d, 3 H), 0.70 (d, 3 H), 0.67 (d, 3 H).
MS (ESI+): m/z (%) = 509 (M+H⁺) (55), 531 (M+Na⁺) (100).
HPLC (Methode 5): Rₜ = 3.59 min.

### Allgemeine Vorschrift D

### Darstellung von N-alkylsubstituierten Harnstoffderivaten ausgehend von substituierten (3R,4S)-3-[(2S)-2-Amino-3-methylbutanoyl]-4-methyl-2,5-pyrrolidindion Derivaten in Form ihrer Hydrochloride und Chloraminsäurederivaten

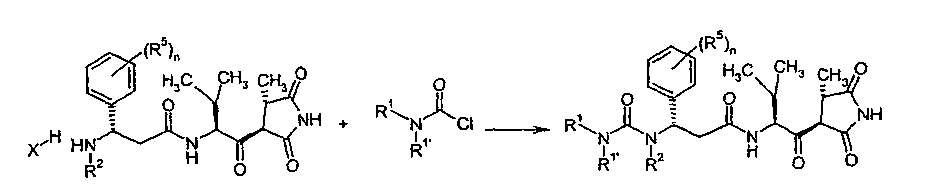

Zu einer Mischung aus Hydrochlorid (1.0 eq.) in absolutem Dichlorethan (ca. 0.1 mol/l) werden bei Raumtemperatur ca. 2.4 eq. polymergebundene Aminbase (PS-DIEA von Argonaut Technologies, Beladung ca. 3.8 mmol/g Harz) und Chloraminsäurederivat (ca. 1.2 eq.) hinzugefügt. Die Mischung wird über Nacht bei Raumtemperatur geschüttelt, und dann filtriert. Das Filtrat wird auf eine Silicagel-Kartusche gegeben. Das Produkt kann mit Gemischen aus Dichlormethan und Methanol (typischerweise 95:5) eluiert werden. Gegebenenfalls kann eine weitere Reinigung durch RP-HPLC erfolgen.

Alternativ können auch das Amin-Hydrochlorid und Triethylamin (2 eq.) in Tetrahydrofuran (ca. 0.1 mol/l) vorgelegt und mit dem, gegebenenfalls in wenig Tetrahydrofuran gelösten, Chloraminsäurederivat bei Raumtemperatur versetzt werden. In diesem Fall wird das Reaktionsgemisch nach Rühren über Nacht bei Temperaturen zwischen Raumtemperatur und 40°C eingeengt, mit Dichlormethan versetzt und mit Wasser gewaschen. Die organische Phase wird über Magnesiumsulfat getrocknet, filtriert und eingeengt. Das Rohprodukt kann durch RP-HPLC (Eluent: Wasser/Acetonitril, Gradient) gereinigt werden.

### Beispiel 7

### (S)-N-{(S)-2-Methyl-1-[1-((3R,4S)-4-methyl-2,5-dioxo-pyrrolidin-3-yl)-methanoyl]-propyl}-3-(3-methyl-3-phenyl-ureido)-3-phenyl-propionamid

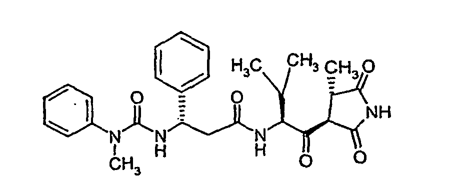

¹H-NMR (400 MHz, d₆-DMSO): δ = 11.35 (s, 1 H), 8.10 (d, 1 H), 7.40-7.15 (m, 10 H), 6.59 (d, 1 H), 5.13-5.08 (m, 1 H), 4.59 (dd, 1 H), 3.98 (d, 1 H), 2.90 (dd, 1 H), 2.82 (dd, 1 H), 2.78-2.65 (m, 1 H), 2.28-2.21 (m, 1 H), 1.11 (d, 3 H), 0.85 (t, 3 H), 0.72 (d, 3 H), 0.65 (d, 3 H).
MS (ESI+): m/z (%) = 493 (M+H⁺) (100).
HPLC (Methode 4): Rₜ = 2.23 min.

### Beispiel 8

### Morpholin-4-carbonsäure-((S)-2-{(S)-2-methyl-1-[1-((3R,4S)-4-methyl-2,5-dioxo-pyrrolidin-3-yl)-methanoyl]-propylcarbamoyl}-1-phenyl-ethyl)-amid

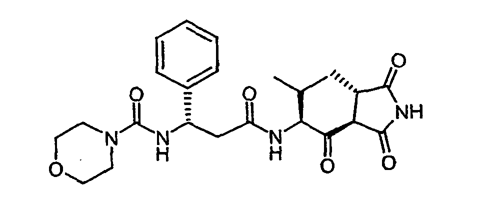

¹H-NMR (400 MHz, d₆-DMSO): δ = 11.30 (br. s, 1 H), 8.02 (d, 1 H), 7.31-7.19 (m, 5 H), 6.90-6.90 (m, 1 H), 5.12-5.08 (m, 1 H), 4.71 (dd, 1 H), 4.01 (d, 1 H), 3.57-3.50 (m, 4 H), 3.30-3.25 (m, 4 H), 3.09-3.05 (m, 1 H), 2.95 (dd, 1 H), 2.71 (dd, 1 H), 2.35-2.30 (m, 1 H), 1.11 (d, 3 H), 0.82 (d, 3 H), 0.73 (d, 3 H).
MS (ESI+): m/z (%) = 493 (M+H⁺) (100).
HPLC (Methode 4): Rₜ = 2.02 min.

### Die Synthese der nachfolgenden Verbindungen erfolgt nach der Allgemeinen Vorschrift C:

### Beispiel 9

### N-((1S)-2-Methyl-1-{[(3R,4S)-4-methyl-2,5-dioxo-3-pyrrolidinyl]carbonyl}propyl)-3-{[(1-naphthylamino)carbonyl]amino}-3-phenylpropionamid

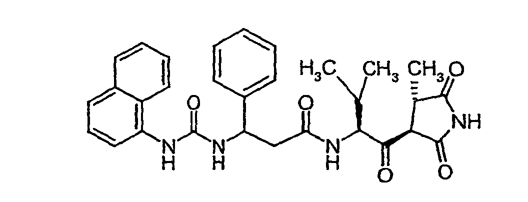

2 Diastereomere
¹H-NMR (300 MHz, d₆-DMSO): δ = 11.31 (s, 1 H), 8.74 + 8.62 (2x s, 1 H), 8.20-8.05 (m, 2 H), 7.99-7.85 (m, 2 H), 7.57-7.48 (m, 3 H), 7.42-7.18 (m, 7 H), 5.28-5.17 (m, 1 H), 4.60 (dd, 1 H), 3.98 + 3.95 (2x d, 1 H), 2.96-2.86 (m, 2 H), 2.78-2.68 (m, 1 H), 2.32-2.20 (m, 1 H), 1.11 (2x d, 3 H), 0.78-0.65 (m, 6 H).
MS (ESI+): m/z (%) = 529 (M+H⁺) (100).
HPLC (Methode 5): Rₜ = 3.87 min.

### Beispiel 10

### 3-[({[4-Chlor-3-(trifluormethyl)phenyl]amino}carbonyl)amino]-N-((1S)-2-methyl-1-{[(3R,4S)-4-methyl-2,5-dioxo-3-pyrrolidinyl]carbonyl}propyl)-3-phenylpropionamid

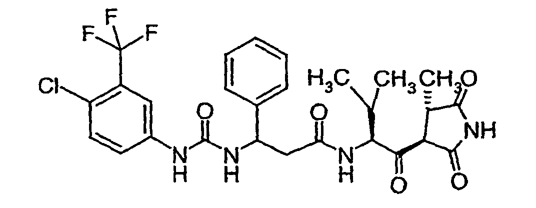

2 Diastereomere
¹H-NMR (300 MHz, d₆-DMSO): δ = 11.32 (s, 1 H), 9.31 + 9.10 (2x s, 1 H), 8.20-8.12 (m, 1 H), 8.03 (s, 1 H), 7.52-7.49 (m, 1 H), 7.34-7.00 (m, 7 H), 5.19-5.08 (m, 1 H), 4.61-4.55 (m, 1 H), 3.96 + 3.94 (2x d, 1 H), 2.98-2.62 (m, 3 H), 2.30-2.20 (m, 1 H), 1.10 (2x d, 3 H), 0.76-0.62 (m, 6 H).
MS (ESI+): m/z (%) = 581 (M+H⁺) (100).

Die Synthese der Verbindungen der Tabelle 1 erfolgt nach der Allgemeinen Vorschrift C:

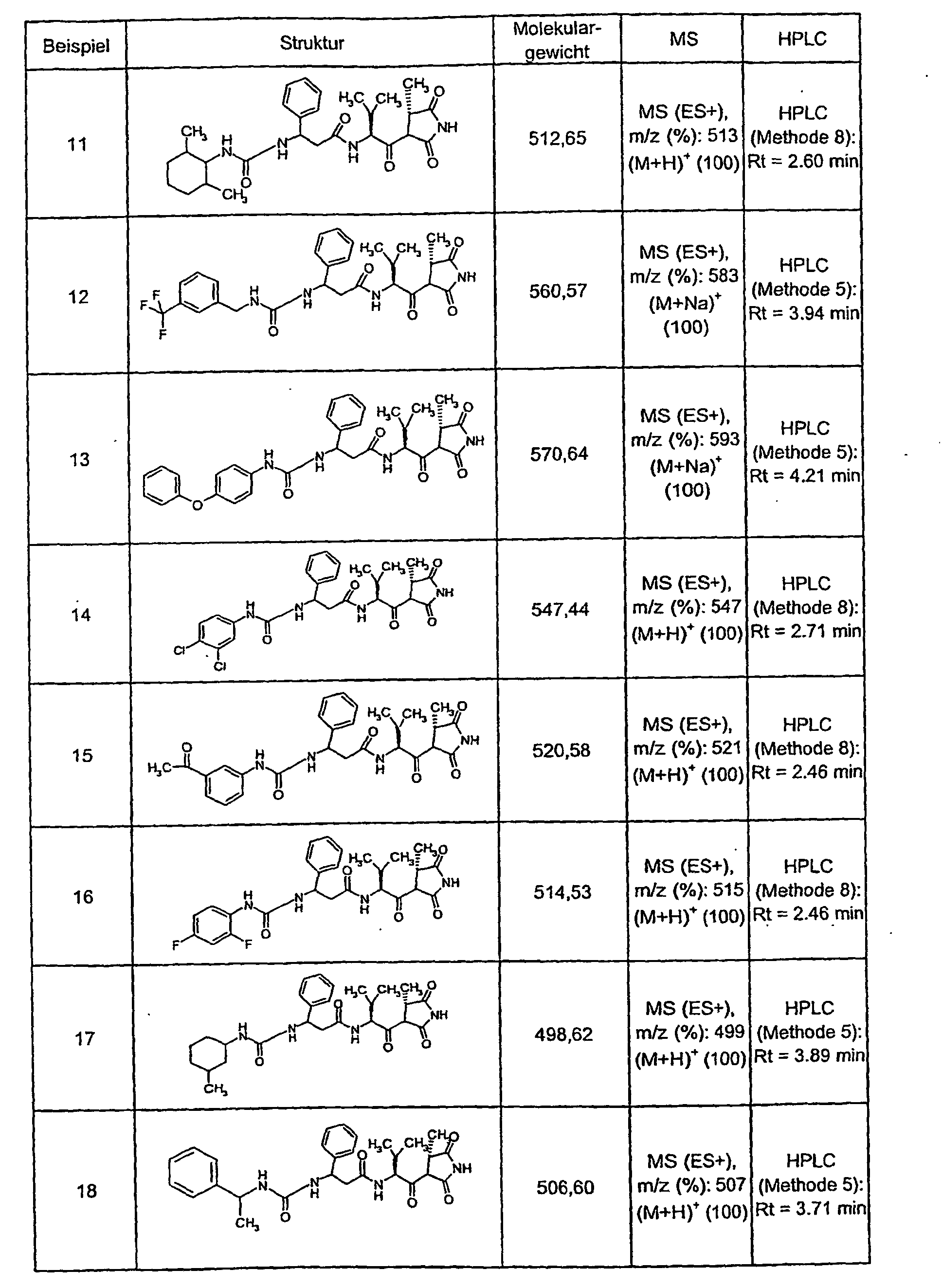

Die Synthese der Verbindungen der Tabelle 2 erfolgt nach der Allgemeinen Vorschrift D:

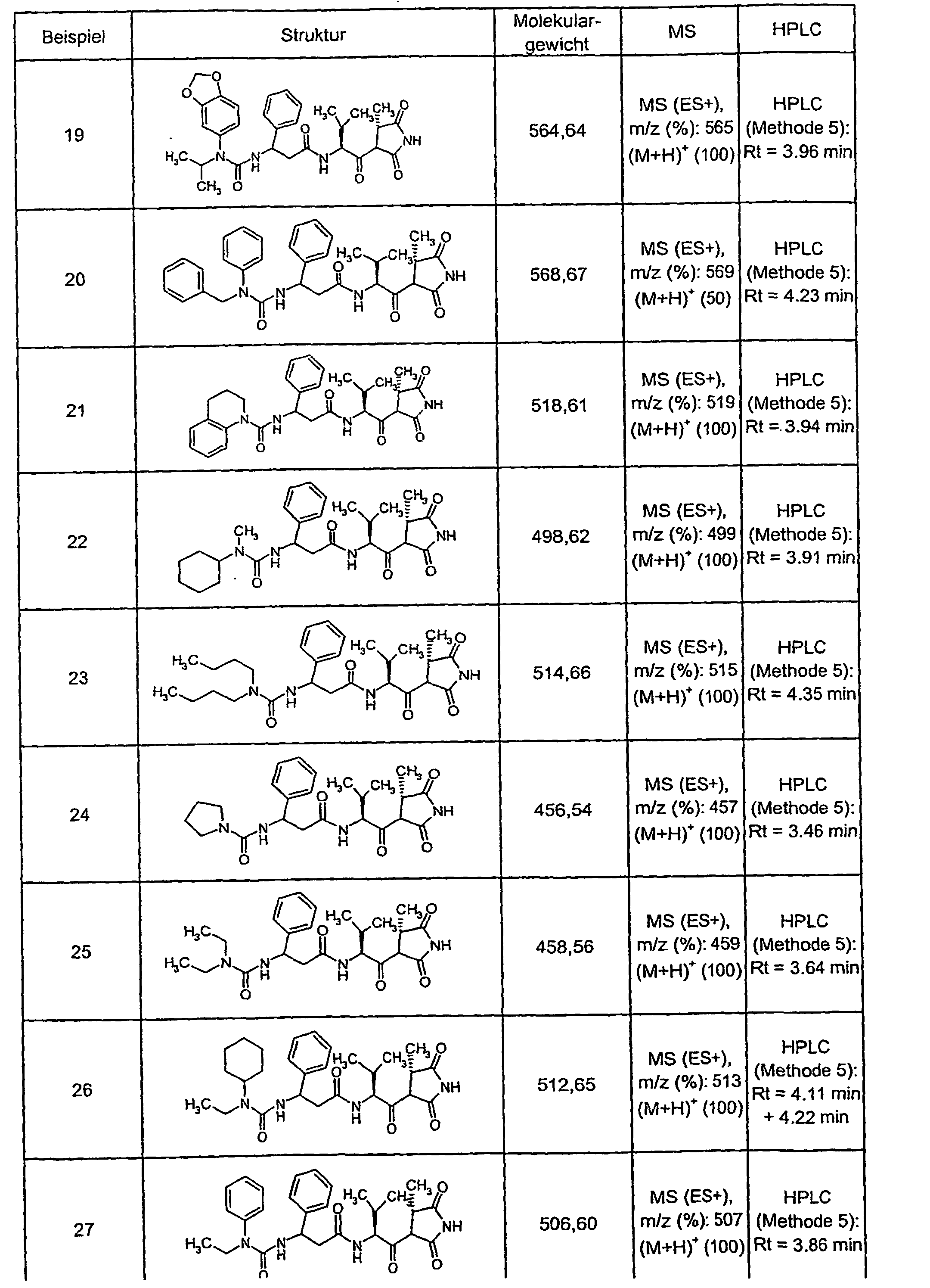

### Allgemeine Vorschrift H

### Acylierung von 3-[2-Amino-alkanoyl]-2,5-pyrrolidindion Hydrochlorid Derivaten mit Carbonsäurederivaten

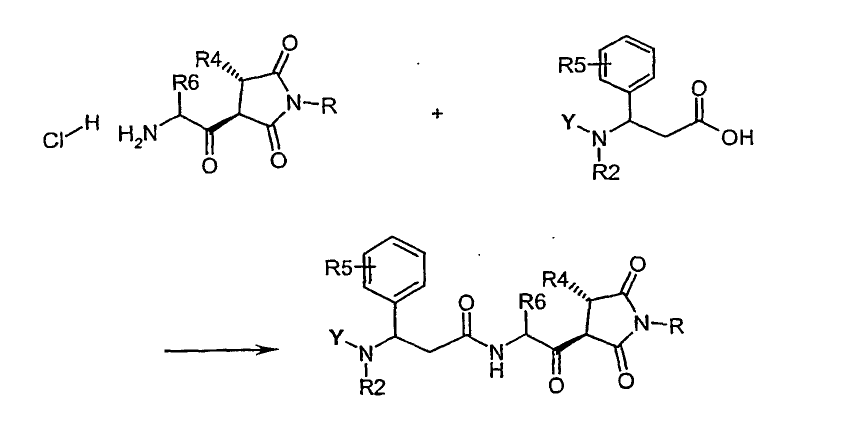

Zu einer Lösung des Carbonsäurederivates (1.2 - 1.5 eq.) in absolutem Dichlormethan oder einer Mischung (5:1 bis 1:1) von absolutem Dichlormethan und N,N-Dimethylformamid (ca. 0.1 bis 0.3 mol/1) werden bei 0°C zunächst eine äquimolare Menge HATU und dann das 3-[2-Amino-alkanoyl]-2,5-pyrrolidindion Hydrochlorid Derivat (1 eq., gegebenenfalls als Lösung in N,N-Dimethylformamid oder Dichlormethan/N,N-Dimethylformamid Gemischen) zugegeben. Anschließend wird bei 0°C eine Lösung von 2.5 - 3.5 eq. Düsopropylethylamin in einem 1:1 Gemisch von absolutem Dichlormethan und N,N-Dimethylformamid (0.2 - 1 mol/l) über einen Zeitraum von 1 h zugetropft. Nach Ende der Zugabe wird die Reaktionsmischung weitere 30 min bei 0°C und anschliessend über Nacht bei Raumtemperatur gerührt, bevor im Vakuum eingeengt wird. Das Produkt kann durch Chromatographie an Silicagel (Eluenten: Gemische aus Cyclohexan/ Essigsäureethylester oder Gemische aus Dichlormethan und Ethanol) oder durch RP-HPLC (Eluenten: variable Gradienten aus Wasser und Acetonitril), alternativ durch eine Kombination beider Verfahren, erhalten werden.

Alternativ kann die Umsetzung auch nach folgendem Verfahren erfolgen:

Zu einer Lösung des 3-[2-Amino-alkanoyl]-2,5-pyrrolidindion Hydrochlorid Derivates (1 eq.) in absolutem Dichlormethan oder einer Mischung (5:1 bis 1:1) von absolutem Dichlormethan und N,N-Dimethylformamid (ca. 0.1 bis 0.3 mol/l) werden das Carbonsäurederivat (1.1 - 1.5 eq.), Triethylamin (3 eq.), HOBt (3 eq.) und abschliessend 1.2 eq. EDC gegeben. Die Reaktionsmischung wird bei Raumtemperatur gerührt (2 h bis über Nacht), bevor im Vakuum eingeengt wird. Der Rückstand wird in Essigsäureethylester oder Dichlormethan aufgenommen und die organische Phase wird mit Wasser, gesättigter Natriumhydrogencarbonatlösung und gesättigter Kochsalzlösung gewaschen, über Natriumsulfat getrocknet, filtriert und eingeengt. Das Produkt kann durch Chromatographie an Silicagel (Eluenten: Gemische aus Cyclohexan/ Essigsäureethylester oder Gemische aus Dichlormethan und Ethanol) oder durch RP-HPLC (Eluenten: variable Gradienten aus Wasser und Acetonitril), alternativ durch eine Kombination beider Verfahren, gereinigt werden.

Nach der Allgemeinen Vorschrift H können folgende Verbindungen erhalten werden:

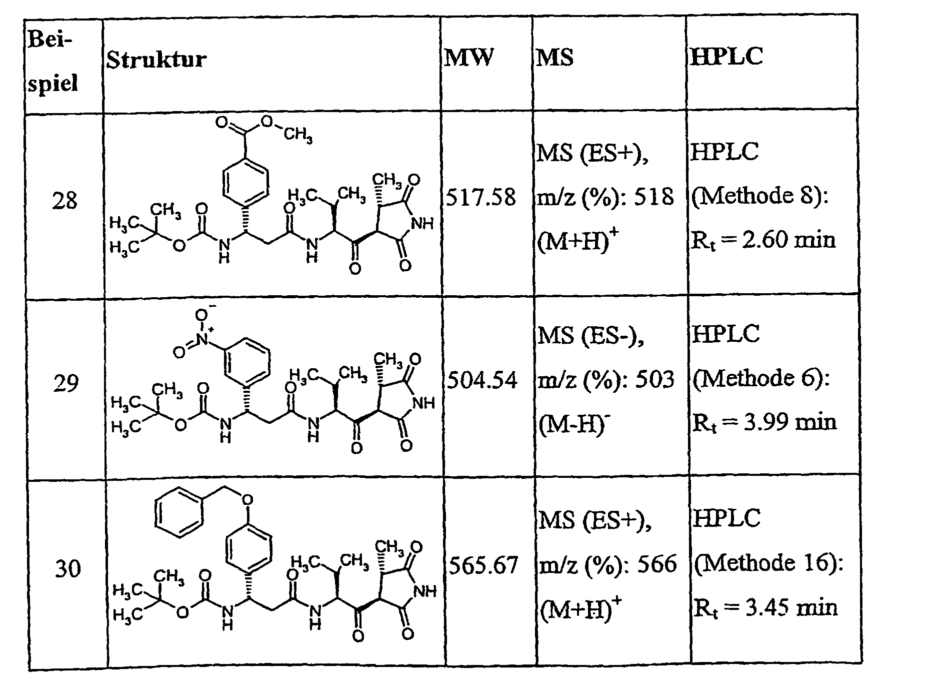

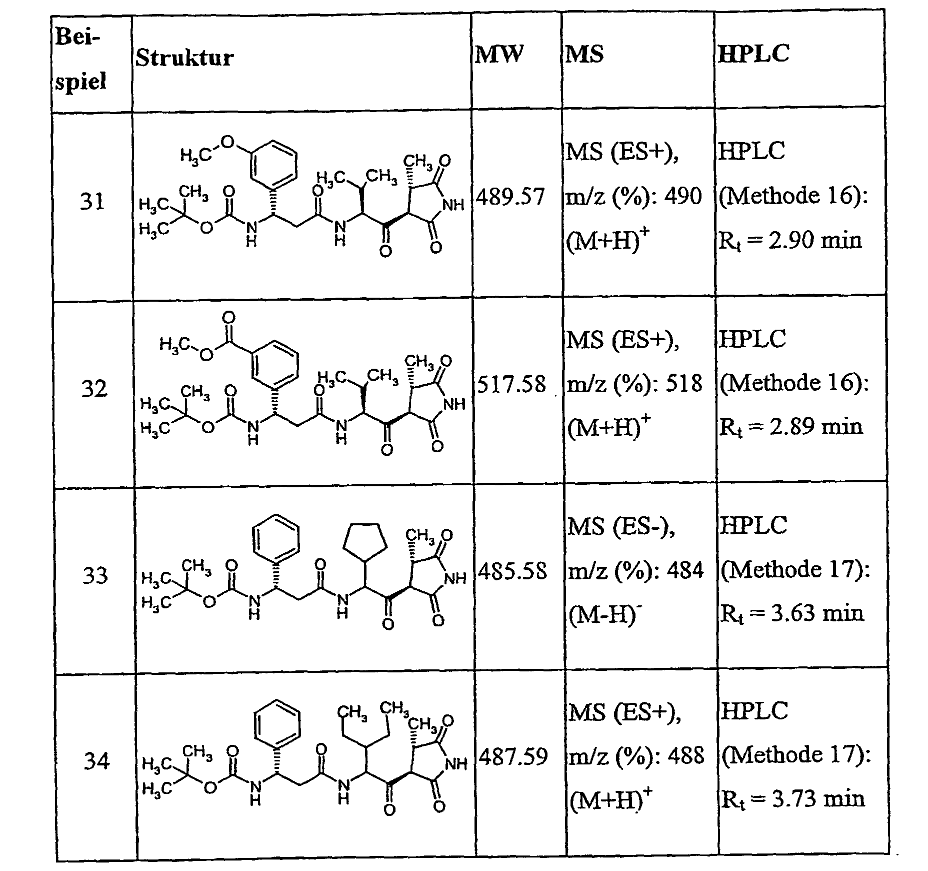

### Allgemeine Vorschrift J

### Umsetzung von acylalkylamino-substituierten 3-[2-Amino-alkanoyl]-2,5-pyrrolidindion Hydrochlorid Derivaten mit Chlorameisensäureestern

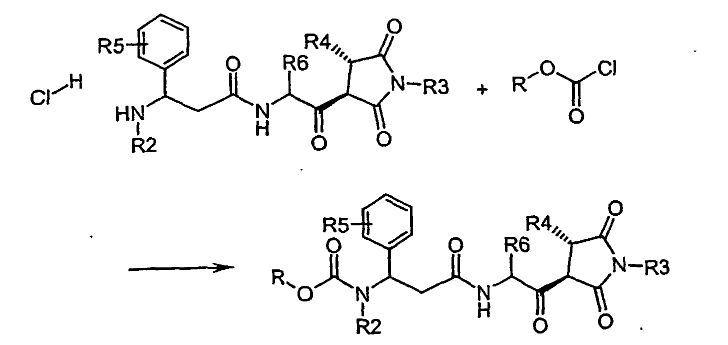

Das Amin-Hydochlorid wird in Tetrahydrofuran vorgelegt (ca. 0.1 mol/l) und mit Triethylamin (2 eq.) und dem Chlorameisensäureester (1.2 eq.) versetzt. Das Reaktionsgemisch wird über Nacht bei Raumtemperatur gerührt. Wenn noch keine vollständige Umsetzung stattgefunden hat (DC-Kontrolle), wird eine weitere Nacht bei 40°C gerührt. Anschließend wird das Reaktionsgemisch eingeengt und der Rückstand wird mit Dichlormethan und Wasser aufgenommen und über eine Extrelut-Kartusche (Merck, Deutschland) filtriert. Das Filtrat wird eingeengt und der Rückstand wird mittels RP-HPLC (Eluenten: variable Gradienten aus Acetonitril und Wasser + 0.3 ml 37%ige Salzsäure/l) gereinigt.

Nach der Allgemeinen Vorschrift J können folgende Verbindungen erhalten werden:

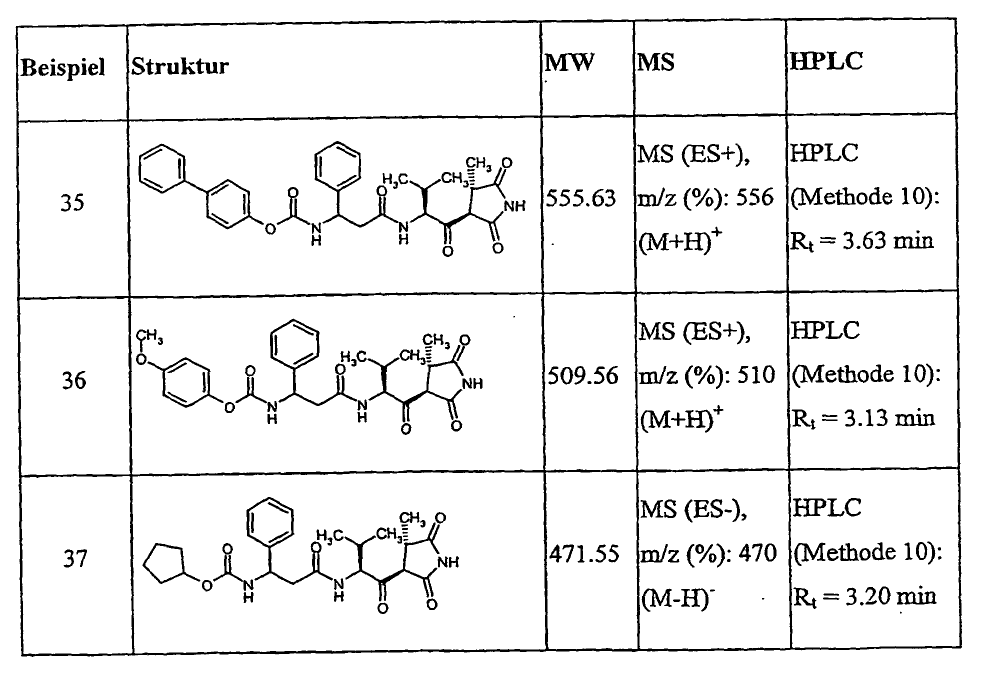

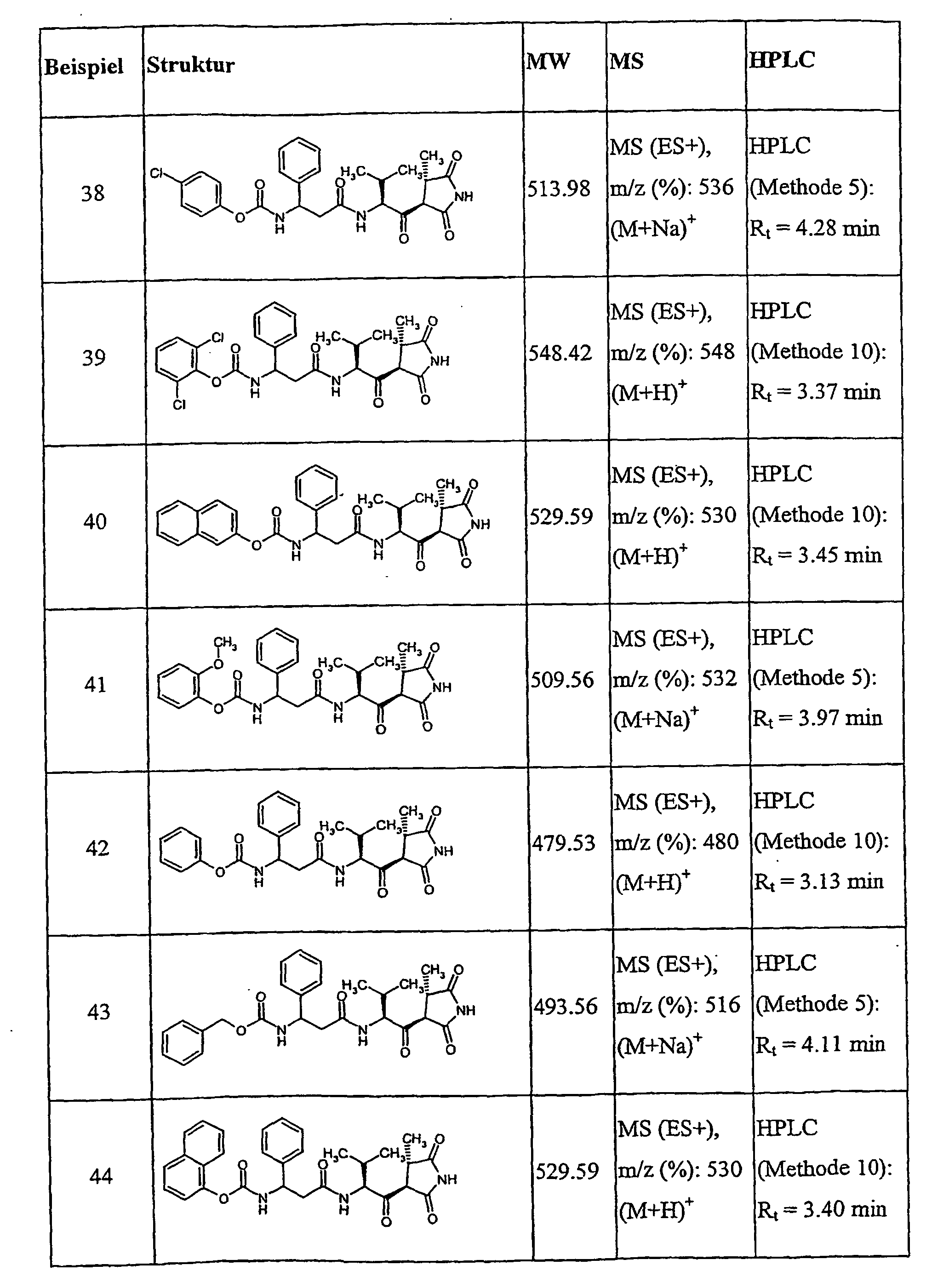

### Allgemeine Vorschrift K

### Darstellung von Sulfonamid-Derivaten ausgehend von substituierten (3R,4S)-3-[(2S)-2-Amino-3-methylbutanoyl]-4-methyl-2,5-pyrrolidindion Derivaten in Form ihrer Hydrochloride mit Sulfonsäurechloriden

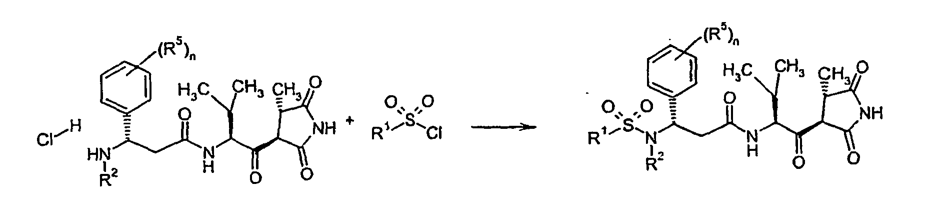

Zu dem Hydrochlorid (1.0 eq.) in absolutem Dichlorethan (ca. 0.1 mol/l) werden bei Raumtemperatur ca. 2.4 eq. polymergebundene Aminbase (PS-DIEA von Argonaut Technologies, Beladung ca. 3.8 mmol/g Harz) und Sulfonylchlorid (ca. 1.2 eq.) hinzugefügt. Die Mischung wird über Nacht bei Raumtemperatur geschüttelt und dann filtriert. Das Filtrat wird auf eine Silicagel-Kartusche gegeben. Das Produkt kann mit Gemischen aus Dichlormethan und Methanol (typischerweise 95:5) eluiert werden. Gegebenenfalls kann eine weitere Reinigung durch RP-HPLC erfolgen. Alternativ kann auch das Hydrochlorid in N,N-Dimethylformamid (ca. 0.1 mol/l) gelöst und mit 2 eq Triethylamin und 1 eq des Sulfonylchlorids versetzt werden. Das Reaktionsgemisch wird über Nacht bei Raumtemperatur gerührt, eingeengt und dann mittels RP-HPLC (Eluent: Wasser-Acetonitril, Gradient) gereinigt.

### Beispiel 45

### (3S)-N-((1S)-2-Methyl-1-{[(3R,4S)-4-methyl-2,5-dioxo-3-pyrrolidinyl]carbonyl}propyl)-3-[(pentylsulfonyl)amino]-3-phenylpropionamid

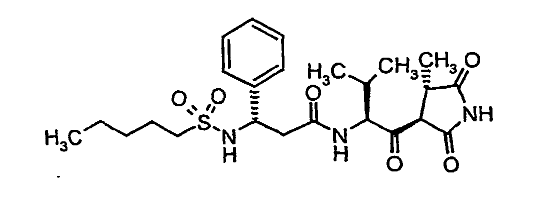

Die Synthese erfolgt nach der Allgemeinen Vorschrift K.
¹H-NMR (400 MHz, d₆-DMSO): δ = 11.31 (s, 1 H), 8.18 (d, 1 H), 7.74 (d, 1 H), 7.40-7.21 (m, 5 H), 4.78-4.69 (m, 1 H), 4.53 (dd, 1 H), 3.83 (d, 1 H), 2.90-2.62 (m, ca. 5 H), 2.32-2.26 (m, 1 H), 1.45-1.05 (m, ca. 6 H), 1.01 (d, 3 H), 0.85 (d, 3 H), 0.80-0.75 (m, 6 H).
MS (ESI+): m/z (%) = 494 (M+H⁺) (100).
HPLC (Methode 4): Rₜ = 2.47 min.

### Beispiel 46

### (3S)-N-((1S)-2-Methyl-1-{[(3R,4S)-4-methyl-2,5-dioxo-3-pyrrolidinyl]carbonyl}propyl)-3-[(octylsulfonyl)amino]-3-phenylpropionamid

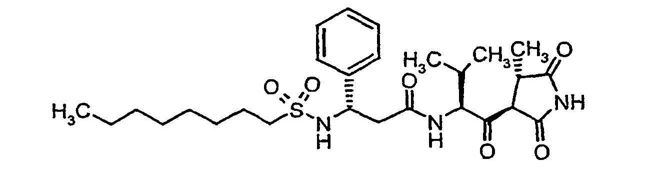

Die Synthese erfolgt nach der Allgemeinen Vorschrift K.
MS (ESI+): m/z (%) = 536 (M+H⁺) (100).
HPLC (Methode 4): Rₜ = 2.80 min.

### Beispiel 47

### (3S)-N-((1S)-2-Methyl-1-{[(3R,4S)-4-methyl-2,5-dioxo-3-pyrrolidinyl]carbonyl}propyl)-3-phenyl-3-({[(E)-2-phenylethenyl]sulfonyl}amino)propionamid

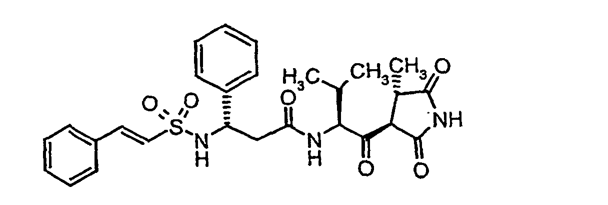

Die Synthese erfolgt nach der Allgemeinen Vorschrift K.
¹H-NMR (300 MHz, d₆-DMSO): δ = 11.30 (br. s, 1 H), 8.14 (d, 1 H), 7.97 (d, 1 H), 7.46-7.10 (m, 11 H), 6.70 (d, 1 H), 4.81-4.68 (m, 1 H), 4.51 (dd, 1 H), 3.80 (d, 1 H), 2.88-2.62 (m, 3 H), 2.30-2.19 (m, 1 H), 1.02 (d, 3 H), 0.78 (d, 3 H), 0.74 (d, 3 H).
MS (ESI+): m/z (%) = 526 (M+H⁺) (100).
HPLC (Methode 5): Rₜ = 3.91 min.

### Beispiel 48

### (3S)-3-[(1,1'-Biphenyl-4-ylsulfonyl)amino]-N-((1S)-2-methyl-1-{[(3R,4S)-4-methyl-2,5-dioxo-3-pyrrolidinyl]carbonyl}propyl)-3-phenylpropionamid

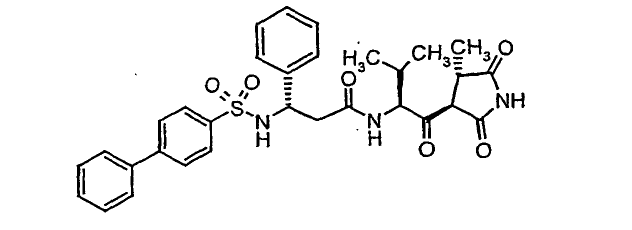

Die Synthese erfolgt nach der Allgemeinen Vorschrift K.
¹H-NMR (300 MHz, d₆-DMSO): δ = 11.30 (br. s, 1 H), 8.27 (d, 1 H), 8.11 (d, 1 H), 7.68-7.60 (m, 6 H), 7.52-7.39 (m, 3 H), 7.13-7.01 (m, 5 H), 4.80-4.68 (m, 1 H), 4.48 (dd, 1 H), 3.75 (d, 1 H), 2.86-2.78 (m, 1 H), 2.65 (d, 2 H), 2.28-2.18 (m, 1 H), 0.99 (d, 3 H), 0.79 (d, 3 H), 0.73 (d, 3 H).
MS (ESI+): m/z (%) = 576 (M+H⁺) (100).
HPLC (Methode 5): Rₜ = 4.23 min.

### Beispiel 49

### ((S)-2-{(S)-2-Methyl-1-[1-((3R,4S)-4-methyl-2,5-dioxo-pyrrolidin-3-yl)-methanoyl]-propylcarbamoyl}-1-phenyl-ethyl)-carbamidsäure-tert-butylester

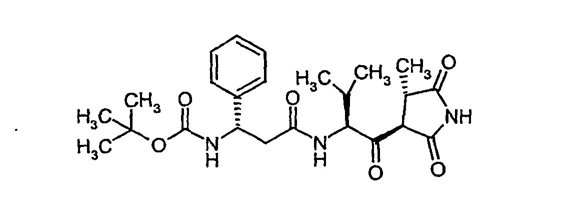

Die Synthese erfolgt nach der Allgemeinen Vorschrift A.
¹H-NMR (400 MHz, d₆-DMSO): δ = 11.45 (s, 1 H), 7.98 (d, 1 H), 7.31-7.24 (m, 5 H), 7.20 (br. s, 1 H), 4.88-4.82 (br. s, 1 H), 4.69 (br. s, 1 H), 3.98 (d, 1 H), 2.95-2.89 (m, 1 H), 2.77-2.69 (m, 1 H), 2.51-2.44 (m, 1 H), 2.35-2.29 (m, 1 H), 1.10 (d, 3 H), 0.85 (d, 3 H), 0.78 (d, 3 H).
MS (ESI+): m/z (%) = 460 (M+H⁺) (100).
HPLC (Methode 6): Rₜ = 3.90 min.

### C. Ausführungsbeispiele für pharmazeutische Zusammensetzungen

Die erfindungsgemäßen Verbindungen können folgendermaßen in pharmazeutische Zubereitungen überführt werden:

### Tablette:

### Zusammensetzung:

100 mg der Verbindung von Beispiel 1, 50 mg Lactose (Monohydrat), 50 mg Maisstärke (nativ), 10 mg Polyvinylpyrolidon (PVP 25) (Fa. BASF, Ludwigshafen, Deutschland) und 2 mg Magnesiumstearat.
Tablettengewicht 212 mg. Durchmesser 8 mm, Wölbungsradius 12 mm.

### Herstellung:

Die Mischung aus Wirkstoff, Lactose und Stärke wird mit einer 5%-igen Lösung (m/m) des PVPs in Wasser granuliert. Das Granulat wird nach dem Trocknen mit dem Magnesiumstearat für 5 min. gemischt. Diese Mischung wird mit einer üblichen Tablettenpresse verpresst (Format der Tablette siehe oben). Als Richtwert für die Verpressung wird eine Pesskraft von 15 kN verwendet.

### Oral applizierbare Suspension:

### Zusammensetzung:

1000 mg der Verbindung von Beispiel 1, 1000 mg Ethanol (96%), 400 mg Rhodigel (Xanthan gum der Fa. FMC, Pennsylvania, USA) und 99 g Wasser.
Einer Einzeldosis von 100 mg der erfindungsgemäßen Verbindung entsprechen 10 ml orale Suspension.

### Herstellung:

Das Rhodigel wird in Ethanol suspendiert, der Wirkstoff wird der Suspension zugefügt. Unter Rühren erfolgt die Zugabe des Wassers. Bis zum Abschluß der Quellung des Rhodigels wird ca. 6h gerührt.

## Patentansprüche

1. Verbindungen der allgemeinen Formel (I) 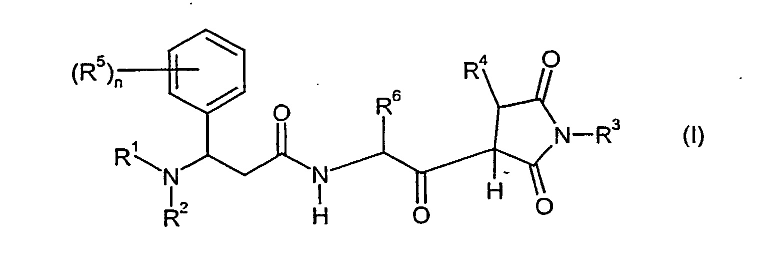 worin
R¹ eine Gruppe 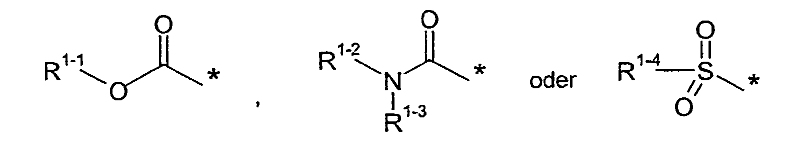
bedeutet, wobei
R¹⁻¹ gleich Alkyl, Cycloalkyl oder Aryl,
wobei
R¹⁻¹ gegebenenfalls substituiert sein kann mit 1 bis 3 Substituenten R¹⁻¹⁻¹, wobei R¹⁻¹⁻¹ unabhängig voneinander ausgewählt wird aus der Gruppe bestehend aus Halogen, Alkyl, Aryl, Alkoxy, Phenoxy, Amino, Monoalkylamino, Dialkylamino, Aminocarbonyl, Monoalkylaminocarbonyl, Dialkylaminocarbonyl, Carboxyl, Alkoxycarbonyl, Alkylcarbonyl, Heteroaryl und Heterocyclyl,
R¹⁻² und R¹⁻³ gleich oder verschieden sind und Wasserstoff, Alkyl, Alkenyl, Alkinyl, Cycloalkyl, Aryl, Heteroaryl oder Heterocyclyl bedeuten,
wobei
R¹⁻² gegebenenfalls substituiert sein kann mit 1 bis 3 Substituenten R¹⁻²⁻¹, wobei R¹⁻²⁻¹ unabhängig voneinander ausgewählt wird aus der Gruppe bestehend aus Halogen, Trifluormethyl, Trifluormethoxy, Nitro, Amino, Alkyl, Monoalkylamino, Dialkylamino, Cycloalkyl, Aryl, Heteroaryl, Heterocyclyl, Hydroxy, Alkoxy, Phenoxy, Carboxyl, Alkoxycarbonyl, Alkylcarbonyl, Aminocarbonyl, Monoalkylaminocarbonyl, Dialkylaminocarbonyl, Aminosulfonyl, Alkylaminosulfonyl, Dialkylaminosulfonyl, Arylaminosulfonyl, Heterocyclylaminosulfonyl, Heteroarylaminosulfonyl, Aminocarbonylamino, Hydroxycarbonylamino und Alkoxycarbonylamino, wobei Aryl und Heteroaryl substituiert sein können mit 1 bis 2 Substituenten, die unabhängig voneinander ausgewählt werden aus der Gruppe bestehend aus Halogen, Hydroxy, Alkyl, Alkoxy, Trifluormethyl, Trifluormethoxy, Nitro, Amino, Cyano, Monoalkylamino und Dialkylamino,
oder
R¹⁻² und R¹⁻³ zusammen mit dem Stickstoffatom, an das sie gebunden sind, einen Heterocyclus bilden, der gegebenenfalls benzanneliert sein kann,
R¹⁻⁴ gleich Alkyl, Alkenyl, Alkinyl, Cycloalkyl oder Aryl,
wobei
R¹⁻⁴ gegebenenfalls substituiert sein kann mit 1 bis 3 Substituenten R¹⁻⁴⁻¹, wobei R¹⁻⁴⁻¹ unabhängig voneinander ausgewählt wird aus der Gruppe bestehend aus Halogen, Alkyl, Aryl, Amino, Monoalkylamino, Dialkylamino, Aminocarbonyl, Monoalkylaminocarbonyl, Dialkylaminocarbonyl, Carboxyl, Alkoxycarbonyl, Alkylcarbonyl, Heteroaryl und Heterocyclyl,
R² gleich Wasserstoff oder Methyl,
R³ gleich Wasserstoff oder C₁-C₃-Alkyl,
R⁴ gleich Wasserstoff oder C₁-C₃-Alkyl,
R⁵ ausgewählt wird aus der Gruppe bestehend aus Halogen, Trifluormethyl, Trifluormethoxy, Nitro, Amino, Monoalkylamino, Dialkylamino, Hydroxy, Alkyl, Alkoxy, Carboxyl, Alkoxycarbonyl, Aminocarbonyl, Monoalkylaminocarbonyl, Dialkylaminocarbonyl, Aryl und Heteroaryl,
oder
zwei Substituenten R⁵ zusammen mit den Kohlenstoffatomen, an die sie gebunden sind, ein Cycloalkyl oder Heterocyclyl bilden,
wobei dieses Cycloalkyl oder Heterocyclyl substituiert sein kann mit 0, 1 oder 2 Substituenten R⁵⁻¹, wobei die Substituenten R⁵⁻¹ unabhängig voneinander ausgewählt werden aus der Gruppe bestehend aus Halogen, Alkyl, Nitro, Amino, Trifluormethyl, Hydroxy und Alkoxy,
n gleich einer Zahl 0, 1, 2 oder 3,
wobei bei n gleich 2 oder 3 die Reste R⁵ gleich oder verschieden sein können,
R⁶ gleich Alkyl, Cycloalkyl oder Heterocyclyl,
wobei R⁶ substituiert sein kann mit 0, 1 oder 2 Substituenten R⁶⁻¹, wobei die Substituenten R⁶⁻¹ unabhängig voneinander ausgewählt werden aus der Gruppe bestehend aus Halogen, Nitro, Amino, Trifluormethyl, Hydroxy, Alkyl und Alkoxy,
sowie deren pharmazeutisch verträgliche Salze, Solvate und Hydrate.

2. Verbindungen der allgemeinen Formel (I) nach Anspruch 1,
worin
R¹⁻¹ gleich Alkyl, Cycloalkyl oder Aryl,
wobei
R¹⁻¹ gegebenenfalls substituiert sein kann mit 1 bis 3 Substituenten R¹⁻¹⁻¹, wobei R¹⁻¹⁻¹ unabhängig voneinander ausgewählt wird aus der Gruppe bestehend aus Halogen, Alkyl, Aryl und Alkoxy,
R¹⁻² und R¹⁻³ gleich oder verschieden sind und Wasserstoff, Alkyl, Alkenyl, Cycloalkyl, Aryl oder Heteroaryl bedeuten,
wobei
R¹⁻² gegebenenfalls substituiert sein kann mit 1 bis 2 Substituenten R¹⁻²⁻¹, wobei R¹⁻²⁻¹ unabhängig voneinander ausgewählt wird aus der Gruppe bestehend aus Halogen, Trifluormethyl, Trifluormethoxy, Nitro, Amino, Alkyl, Monoalkylamino, Dialkylamino, Cycloalkyl, Aryl, Heteroaryl, Heterocyclyl, Hydroxy, Alkoxy, Phenoxy, Alkylcarbonyl, Aminocarbonyl, Monoalkylaminocarbonyl, Dialkylaminocarbonyl, Aminosulfonyl, wobei Aryl und Heteroaryl substituiert sein können mit 1 bis 2 Substituenten, die unabhängig voneinander ausgewählt werden aus der Gruppe bestehend aus Halogen, Hydroxy, Alkoxy, Trifluormethyl, Trifluormethoxy, Nitro und Amino,
oder R¹⁻² und R¹⁻³ zusammen mit dem Stickstoffatom, an das sie gebunden sind, einen Heterocyclus bilden, der gegebenenfalls benzanneliert sein kann,
R¹⁻⁴ gleich Alkyl, Alkenyl oder Aryl,
wobei
R¹⁻⁴ gegebenenfalls substituiert sein kann mit 1 bis 3 Substituenten R¹⁻⁴⁻¹, wobei R¹⁻⁴⁻¹ unabhängig voneinander ausgewählt wird aus der Gruppe bestehend aus Halogen, Alkyl und Aryl,
R² gleich Wasserstoff,
R³ gleich Wasserstoff oder Methyl,
R⁴ gleich Methyl,
R⁵ ausgewählt wird aus der Gruppe bestehend aus Fluor, Chlor, Trifluormethyl, Trifluormethoxy, Nitro, Amino, Monoalkylamino, Dialkylamino, Hydroxy, Alkyl, Alkoxy, Alkoxycarbonyl, Aminocarbonyl, Phenyl und 5- bis 6-gliedriges Heteroaryl,
oder
zwei Substituenten R⁵ zusammen mit den Kohlenstoffatomen, an die sie gebunden sind, ein Cycloalkyl oder Heterocyclyl bilden,
n gleich einer Zahl 0, 1 oder 2,
wobei bei n gleich 2 die Reste R⁵ gleich oder verschieden sein können,
R⁶ gleich Alkyl oder Cycloalkyl,
wobei R⁶ substituiert sein kann mit 0, 1 oder 2 Substituenten R⁶⁻¹, wobei die Substituenten R⁶⁻¹ unabhängig voneinander ausgewählt werden aus der Gruppe bestehend aus Halogen, Trifluormethyl und Alkoxy,
sowie deren pharmazeutisch verträgliche Salze, Solvate und Hydrate.

3. Verbindungen der allgemeinen Formel (I) nach Anspruch 1 oder 2,
worin
R¹⁻¹ gleich C₁-C₄-Alkyl, Cyclopentyl, Cyclohexyl, Phenyl oder Naphtyl,
wobei
R¹⁻¹ gegebenenfalls substituiert sein kann mit 1 bis 2 Substituenten R¹⁻¹⁻¹, wobei R¹⁻¹⁻¹ unabhängig voneinander ausgewählt wird aus der Gruppe bestehend aus Fluor, Chlor, Methyl, Ethyl, Phenyl, Methoxy und Ethoxy,
R¹⁻² Alkyl, Alkenyl, Cycloalkyl, Aryl oder Heteroaryl bedeutet,
wobei
R¹⁻² gegebenenfalls substituiert sein kann mit 1 Substituenten R¹⁻²⁻¹, wobei R¹⁻²⁻¹ ausgewählt wird aus der Gruppe bestehend aus Fluor, Chlor, Trifluormethyl, Amino, Alkyl, Monoalkylamino, Dialkylamino, Alkylcarbonyl, Aryl, Heteroaryl, Hydroxy, Methoxy und Phenoxy, wobei Aryl und Heteroaryl substituiert sein können mit 1 Substituenten ausgewählt aus der Gruppe bestehend aus Halogen, Methoxy, Trifluormethyl und Amino,
R¹⁻³ gleich Wasserstoff oder Methyl bedeutet,
oder
R¹⁻² und R¹⁻³ zusammen mit dem Stickstoffatom, an das sie gebunden sind, einen Heterocyclus bilden, der gegebenenfalls benzanneliert sein kann,
R¹⁻⁴ gleich Alkyl, Alkenyl oder Phenyl,
wobei
R¹⁻⁴ gegebenenfalls substituiert sein kann mit 1 Substituenten R¹⁻⁴⁻¹, wobei R¹⁻⁴⁻¹ ausgewählt wird aus der Gruppe bestehend aus Fluor, Chlor und Phenyl,
R² gleich Wasserstoff,
R³ gleich Wasserstoff,
R⁴ gleich Methyl,
R⁵ ausgewählt wird aus der Gruppe bestehend aus Fluor, Chlor, Trifluormethyl, Alkoxy, Methoxycarbonyl, C₁-C₄-Alkyl, Phenyl und Pyridyl,
n gleich einer Zahl 0, 1 oder 2,
wobei bei n gleich 2 die Reste R⁵ gleich oder verschieden sein können,
R⁶ gleich Isopropyl, Isobutyl, Isopentyl, Cyclopentyl,
sowie deren pharmazeutisch verträgliche Salze, Solvate und Hydrate.

4. Verbindungen der allgemeinen Formel (I) nach Anspruch 1, 2 oder 3, welche die allgemeine Formel (Ic) aufweisen: 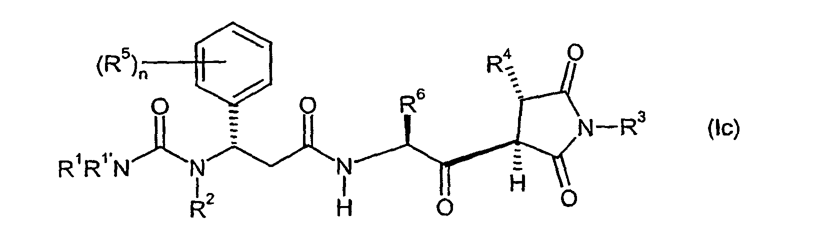 worin
R¹ bis R⁶ und n wie in Anspruch 1 definiert sind.

5. Verbindungen der allgemeinen Formel (I) nach Anspruch 1,2 oder 3, worin
R¹ eine Gruppe 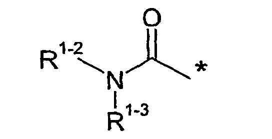
bedeutet, wobei
R¹⁻² gleich Alkyl, Alkenyl, Cycloalkyl oder Aryl bedeutet,
wobei
R¹⁻² gegebenenfalls substituiert sein kann mit 1 bis 2, insbesondere einem Substituenten R¹⁻²⁻¹, wobei R¹⁻²⁻¹ ausgewählt wird aus der Gruppe bestehend aus Halogen, Nitro, Amino, Alkyl, Aryl, Heteroaryl, Hydroxy, Alkoxy, Carboxyl, Alkylcarbonyl, Alkoxycarbonyl und Aminocarbonyl, insbesondere ausgewählt aus der Gruppe bestehend aus Alkyl, Aryl, Heteroaryl und Alkoxy.

6. Verbindungen der allgemeinen Formel (I) nach Anspruch 1, worin
R¹ eine Gruppe 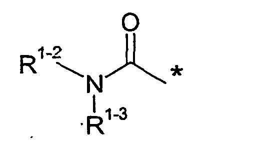
bedeutet,
worin R¹⁻³ gleich Wasserstoff oder Methyl ist.

7. Verbindungen der allgemeinen Formel (I) nach Anspruch 1, 2 oder 3, worin R² gleich Wasserstoff ist.

8. Verbindungen der allgemeinen Formel (I) nach Anspruch 1, 2 oder 3, worin R³ gleich Wasserstoff ist.

9. Verbindungen der allgemeinen Formel (I) nach Anspruch 1, 2 oder 3, worin R⁴ gleich Methyl ist.

10. Verbindungen der allgemeinen Formel (I) nach Anspruch 1, 2 oder 3, worin R⁵ gleich Wasserstoff ist.

11. Verbindungen der allgemeinen Formel (I) nach Anspruch 1, 2 oder 3, worin R⁶ gleich Isopropyl ist.

12. Verfahren zur Herstellung der Verbindungen der allgemeinen Formel (I) nach Anspruch 1, worin Verbindungen der allgemeinen Formel (II) 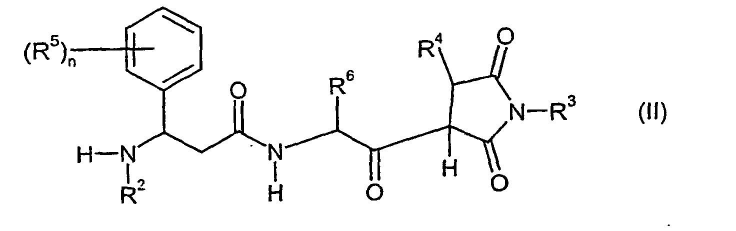 in welcher
R² bis R⁶ und n die in Anspruch 1 angegebene Bedeutung aufweisen,
wobei diese gegebenenfalls in Form ihrer Salze vorliegen können,
für den Fall, dass
R¹ eine Gruppe 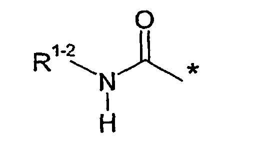
in welcher
R¹⁻² die in Anspruch 1 angegebene Bedeutung aufweist,
bedeutet,
[A] mit Verbindungen der allgemeinen Formel (IIIa) 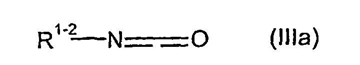 in welcher
R¹⁻² die in Anspruch 1 angegebene Bedeutung aufweist,
umgesetzt werden, oder
für den Fall, dass
R¹ eine Gruppe 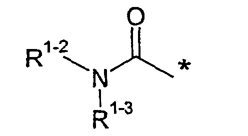
bedeutet, wobei
R¹⁻² die in Anspruch 1 angegebene Bedeutung hat,
R¹⁻³ ungleich Wasserstoff ist, oder R¹⁻² und R¹⁻³ einen Heterocyclus bilden,
[B] mit Verbindungen der allgemeinen Formel (IIIb) 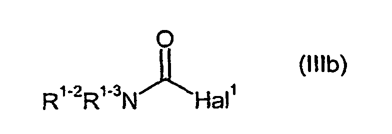 in welcher
R¹⁻² und R¹⁻³ die in Anspruch 1 angegebene Bedeutung aufweisen und Hal¹ für ein Halogenid oder eine andere Fluchtgruppe steht,
umgesetzt werden, oder
für den Fall, dass
R¹ eine Gruppe 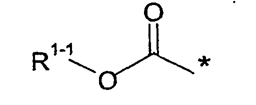
in welcher
R¹⁻¹ die in Anspruch 1 angegebene Bedeutung aufweist,
bedeutet,
[C] mit Verbindungen der allgemeinen Formel (IIIc) 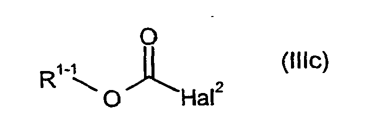 in welcher
R¹⁻¹ die in Anspruch 1 angegebene Bedeutung aufweist und Hal² für ein Halogenid oder eine andere Fluchtgruppe steht,
umgesetzt werden, oder
für den Fall, dass
R¹ eine Gruppe 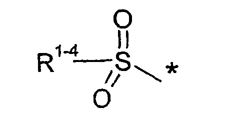
bedeutet, in welcher
R¹⁻⁴ die in Anspruch 1 angegebene Bedeutung aufweist,
[D] mit Verbindungen der allgemeinen Formel (IIId) 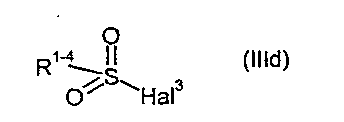
in welcher
R¹⁻⁴ die in Anspruch 1 angegebene Bedeutung aufweist und Hal³ für ein Halogenid oder eine andere Fluchtgruppe steht,
umgesetzt werden.

13. Verbindungen der allgemeinen Formel (I) nach Anspruch 1, 2 oder 3, zur Bekämpfung von Erkrankungen.

14. Verbindungen der allgemeinen Formel (I) nach Anspruch 1, 2 oder 3, zur Bekämpfung bakterieller Erkrankungen.

15. Arzneimittel, enthaltend Verbindungen der allgemeinen Formel (I) nach Anspruch 1, 2 oder 3 und Hilfsstoffe.

16. Verwendung von Verbindungen der allgemeinen Formel (I) nach Anspruch 1, 2 oder 3 zur Herstellung eines Arzneimittels zur Behandlung von Erkrankungen.

17. Verwendung von Verbindungen der allgemeinen Formel (I) nach Anspruch 1, 2 oder 3 zur Herstellung eines Arzneimittels zur Behandlung bakterieller Erkrankungen.

## Claims

1. Compounds of the general formula (I) 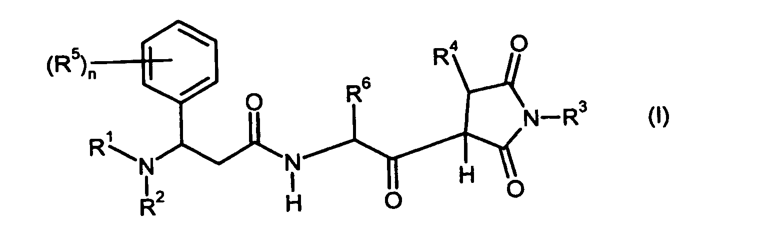 in which
R¹ is a group 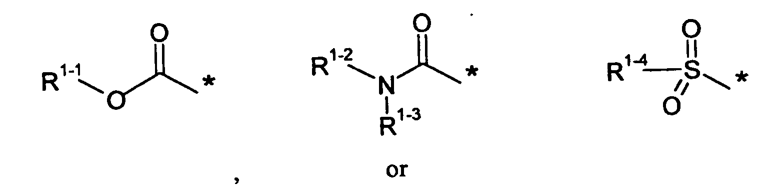
where
R¹⁻¹ is alkyl, cycloalkyl or aryl,
where
R¹⁻¹ can optionally be substituted by from 1 to 3 substituents R¹⁻¹⁻¹, R¹⁻¹⁻¹ being selected independently at each occurrence from the group consisting of halogen, alkyl, aryl, alkoxy, phenoxy, amino, monoalkylamino, dialkylamino, aminocarbonyl, monoalkylaminocarbonyl, dialkylaminocarbonyl, carboxyl, alkoxycarbonyl, alkylcarbonyl, heteroaryl and heterocyclyl,
R¹⁻² and R¹⁻³ are identical or different and are hydrogen, alkyl, alkenyl, alkynyl, cycloalkyl, aryl, heteroaryl or heterocyclyl,
where
R¹⁻² can optionally be substituted by from 1 to 3 substituents R¹⁻²⁻¹, R¹⁻²⁻¹ being selected independently at each occurrence from the group consisting of halogen, trifluoromethyl, trifluoromethoxy, nitro, amino, alkyl, monoalkylamino, dialkylamino, cycloalkyl, aryl, heteroaryl, heterocyclyl, hydroxyl, alkoxy, phenoxy, carboxyl, alkoxycarbonyl, alkyl-carbonyl, aminocarbonyl, monoalkylaminocarbonyl, dialkylaminocarbonyl, amino-sulphonyl, alkylaminosulphonyl, dialkylamino-sulphonyl, arylaminosulphonyl, heterocyclyl-aminosulphonyl, heteroarylaminosulphonyl, aminocarbonylamino, hydroxycarbonylamino and alkoxycarbonylamino, it being possible for aryl and heteroaryl to be substituted by from 1 to 2 substituents selected independently of one another from the group consisting of halogen, hydroxyl, alkyl, alkoxy, trifluoromethyl, trifluoromethoxy, nitro, amino, cyano, monoalkylamino and dialkylamino,
or
R¹⁻² and R¹⁻³ together with the nitrogen atom to which they are attached form a heterocycle which can optionally be benzo-fused,
R¹⁻⁴ is alkyl, alkenyl, alkynyl, cycloalkyl or aryl,
where
R¹⁻⁴ can optionally be substituted by from 1 to 3 substituents R¹⁻⁴⁻¹, R¹⁻⁴⁻¹ being selected independently at each occurrence from the group consisting of halogen, alkyl, aryl, amino, monoalkylamino, dialkylamino, aminocarbonyl, monoalkylaminocarbonyl, dialkylaminocarbonyl, carboxyl, alkoxycarbonyl, alkylcarbonyl, heteroaryl and heterocyclyl,
R² is hydrogen or methyl,
R³ is hydrogen or C₁-C₃-alkyl,
R⁴ is hydrogen or C₁-C₃-alkyl,
R⁵ is selected from the group consisting of halogen, trifluoromethyl, trifluoromethoxy, nitro, amino, monoalkylamino, dialkylamino, hydroxyl, alkyl, alkoxy, carboxyl, alkoxycarbonyl, aminocarbonyl, monoalkylaminocarbonyl, dialkylaminocarbonyl, aryl and heteroaryl,
or
two substituents R⁵ together with the carbon atoms to which they are attached form a cycloalkyl or heterocyclyl,
it being possible for this cycloalkyl or heterocyclyl to be substituted by 0, 1 or 2 substituents R⁵⁻¹, the substituents R⁵⁻¹ being selected independently of one another from the group consisting of halogen, alkyl, nitro, amino, trifluoromethyl, hydroxyl and alkoxy,
n is 0, 1, 2 or 3,
where if n is 2 or 3 the radicals R⁵ can be identical or different,
R⁶ is alkyl, cycloalkyl or heterocyclyl,
it being possible for R⁶ to be substituted by 0, 1 or 2 substituents R⁶⁻¹, the substituents R⁶⁻¹ being selected independently of one another from the group consisting of halogen, nitro, amino, trifluoromethyl, hydroxyl, alkyl and alkoxy,
and also their pharmaceutically compatible salts, solvates and hydrates.

2. Compounds of the general formula (I) according to Claim 1,
in which
R¹⁻¹ is alkyl, cycloalkyl or aryl,
where
R¹⁻¹ can be optionally substituted by from 1 to 3 substituents R¹⁻¹⁻¹, R¹⁻¹⁻¹ being selected independently at each occurrence from the group consisting of halogen, alkyl, aryl and alkoxy,
R¹⁻² and R¹⁻³ are identical or different and are hydrogen, alkyl, alkenyl, cycloalkyl, aryl or heteroaryl,
where
R¹⁻² can be substituted by from 1 to 2 substituents R¹⁻²⁻¹, R¹⁻²⁻¹ being selected independently at each occurrence from the group consisting of halogen, trifluoromethyl, trifluoromethoxy, nitro, amino, alkyl, monoalkylamino, dialkylamino, cycloalkyl, aryl, heteroaryl, heterocyclyl, hydroxyl, alkoxy, phenoxy, alkylcarbonyl, aminocarbonyl, monoalkylaminocarbonyl, dialkylaminocarbonyl and aminosulphonyl, it being possible for aryl and heteroaryl to be substituted by from 1 to 2 substituents selected independently of one another from the group consisting of halogen, hydroxyl, alkoxy, trifluoromethyl, trifluoromethoxy, nitro and amino,
or R¹⁻² and R¹⁻³ together with the nitrogen atom to which they are attached form a heterocycle which can optionally be benzo-fused,
R¹⁻⁴ is alkyl, alkenyl or aryl,
where
R¹⁻⁴ can optionally be substituted by from 1 to 3 substituents R¹⁻⁴⁻¹, R¹⁻⁴⁻¹ being selected independently at each occurrence from the group consisting of halogen, alkyl and aryl,
R² is hydrogen,
R³ is hydrogen or methyl,
R⁴ is methyl,
R⁵ is selected from the group consisting of fluorine, chlorine, trifluoromethyl, trifluoromethoxy, nitro, amino, monoalkylamino, dialkylamino, hydroxyl, alkyl, alkoxy, alkoxycarbonyl, aminocarbonyl, phenyl, and 5- to 6-membered heteroaryl,
or
two substituents R⁵ together with the carbon atoms to which they are attached form a cycloalkyl or heterocyclyl,
n is 0, 1 or 2,
where if n is 2 the radicals R⁵ can be identical or different,
R⁶ is alkyl or cycloalkyl,
where R⁶ can be substituted by 0, 1 or 2 substituents R⁶⁻¹, the substituents R⁶⁻¹ being selected independently of one another from the group consisting of halogen, trifluoromethyl and alkoxy,
and also their pharmaceutically compatible salts, solvates and hydrates.

3. Compounds of the general formula (I) according to Claim 1 or 2,
in which
R¹⁻¹ is C₁-C₄-alkyl, cyclopentyl, cyclohexyl, phenyl or naphthyl,
where
R¹⁻¹ can optionally be substituted by from 1 to 2 substituents R¹⁻¹⁻¹, R¹⁻¹⁻¹ being selected independently at each occurrence from the group consisting of fluorine, chlorine, methyl, ethyl, phenyl, methoxy and ethoxy,
R¹⁻² is alkyl, alkenyl, cycloalkyl, aryl or heteroaryl,
it being possible for R¹⁻² to be optionally substituted by 1 substituent R¹⁻²⁻¹, R¹⁻²⁻¹ being selected from the group consisting of fluorine, chlorine, trifluoromethyl, amino, alkyl, monoalkylamino, dialkylamino, alkylcarbonyl, aryl, heteroaryl, hydroxyl, methoxy and phenoxy, it being possible for aryl and heteroaryl to be substituted by 1 substituent selected from the group consisting of halogen, methoxy, trifluoromethyl and amino,
R¹⁻³ is hydrogen or methyl,
or
R¹⁻² and R¹⁻³ together with the nitrogen atom to which they are attached form a heterocycle which can optionally be benzo-fused,
R¹⁻⁴ is alkyl, alkenyl or phenyl,
where
R¹⁻⁴ can optionally be substituted by 1 substituent R¹⁻⁴⁻¹, R¹⁻⁴⁻¹ being selected from the group consisting of fluorine, chlorine and phenyl,
R² is hydrogen,
R³ is hydrogen,
R⁴ is methyl,
R⁵ is selected from the group consisting of fluorine, chlorine, trifluoromethyl, alkoxy, methoxycarbonyl, C₁-C₄-alkyl, phenyl and pyridyl,
n is 0, 1 or 2,
where if n is 2 the radicals R⁵ can be identical or different,
R⁶ is isopropyl, isobutyl, isopentyl or cyclopentyl,
and also their pharmaceutically acceptable salts, solvates and hydrates.

4. Compounds of the general formula (I) according to Claim 1, 2 or 3 which have the general formula (Ic): 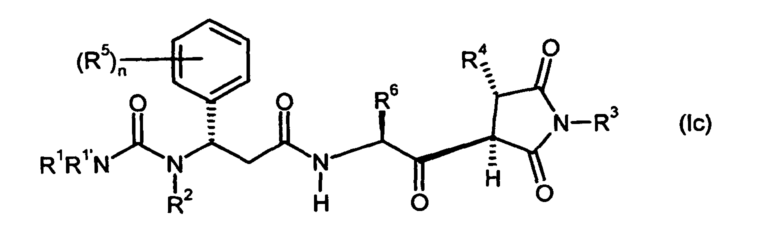 in which
R¹ to R⁶ and n are as defined in claim 1.

5. Compounds of the general formula (I) according to Claim 1, 2 or 3, in which
R¹ is a group 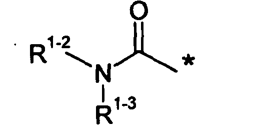 where
R¹⁻² is alkyl, alkenyl, cycloalkyl or aryl,
it being possible for R¹⁻² to be optionally substituted by from 1 to 2, in particular one, substituent(s) R¹⁻²⁻¹, R¹⁻²⁻¹ being selected from the group consisting of halogen, nitro, amino, alkyl, aryl, heteroaryl, hydroxyl, alkoxy, carboxyl, alkylcarbonyl, alkoxycarbonyl and aminocarbonyl, in particular selected from the group consisting of alkyl, aryl, heteroaryl and alkoxy.

6. Compounds of the general formula (I) according to Claim 1, in which
R¹ is a group 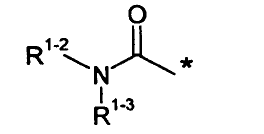
in which R¹⁻³ is hydrogen or methyl.

7. Compounds of the general formula (I) according to Claim 1, 2 or 3, in which R² is hydrogen.

8. Compounds of the general formula (I) according to Claim 1, 2 or 3, in which R³ is hydrogen.

9. Compounds of the general formula (I) according to Claim 1, 2 or 3, in which R⁴ is methyl.

10. Compounds of the general formula (I) according to Claim 1, 2 or 3, in which R⁵ is hydrogen.

11. Compounds of the general formula (I) according to Claim 1, 2 or 3, in which R⁶ is isopropyl.

12. Process for preparing the compounds of the general formula (I), according to Claim 1, in which compounds of the general formula (II) 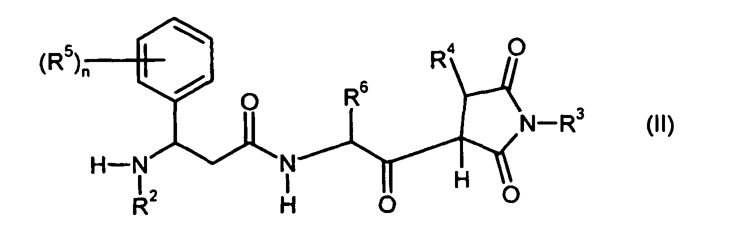 in which
R² to R⁶ and n are as defined in Claim 1,
which may optionally be present in the form of their salts,
for the case where
R¹ is a group 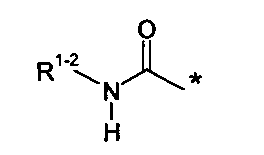
in which R¹⁻² is as defined in Claim 1,
[A] are reacted with compounds of the general formula (IIIa) 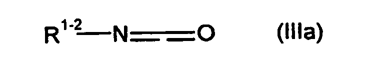 in which
R¹⁻² is as defined in Claim 1,
or,
for the case where
R¹ is a group 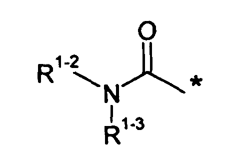
where
R¹⁻² is as defined in Claim 1,
R¹⁻³ is not hydrogen, or R¹⁻² and R¹⁻³ form a heterocycle,
[B] are reacted with compounds of the general formula (IIIb) 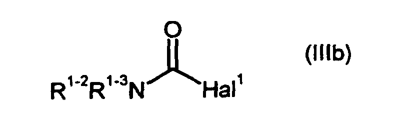 in which
R¹⁻² and R¹⁻³ are as defined in Claim 1 and Hal¹ stands for a halide or another leaving group,
or,
for the case where
R¹ is a group 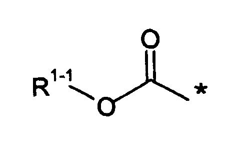
in which
R¹⁻¹ is as defined in Claim 1,
[C] are reacted with compounds of the general formula (IIIc) 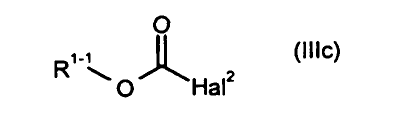 in which
R¹⁻¹ is as defined in Claim 1 and Hal² stands for a halide or another leaving group,
or,
for the case where
R¹ is a group 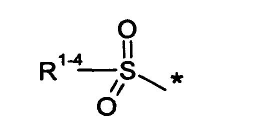
in which
R¹⁻⁴ is as defined in Claim 1,
[D] are reacted with compounds of the general formula (IIId) 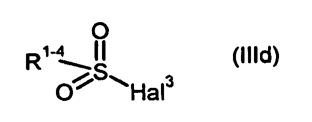
in which
R¹⁻⁴ is as defined in Claim 1 and Hal³ stands for a halide or another leaving group.

13. Compounds of the general formula (I) according to Claim 1, 2 or 3 for fighting diseases.

14. Compounds of the general formula (I) according to Claim 1, 2 or 3 for fighting bacterial diseases.

15. Medicinal products comprising compounds of the general formula (I) of claim 1, 2 or 3 and excipients.

16. Use of compounds of the general formula (I) according to Claim 1, 2 or 3 for producing a medicinal product for treating diseases.

17. Use of compounds of the general formula (I) according to Claim 1, 2 or 3 for producing a medicinal product for treating bacterial diseases.

## Revendications

1. Composés de formule générale (I) 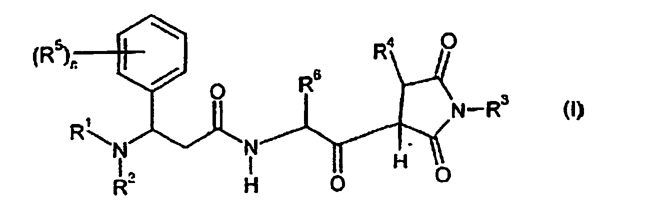 dans laquelle
R¹ est un groupe 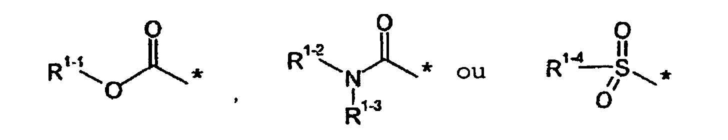
dans lequel
R¹⁻¹ est un reste alkyle, cycloalkyle ou aryle,
R¹⁻¹ pouvant éventuellement être substitué avec 1 à 3 substituants R¹⁻¹⁻¹, les substituants R¹⁻¹⁻¹ étant choisis indépendamment les uns des autres dans le groupe constitué d'halogène, alkyle, aryle, alkoxy, phénoxy, amino, monoalkylamino, dialkylamino, aminocarbonyle, monoalkylaminocarbonyle, dialkylaminocarbonyle, carboxyle, alkoxycarbonyle, alkylcarbonyle, hétéroaryle et hétérocyclyle,
R¹⁻² et R¹⁻³ sont identiques ou différents et représentent l'hydrogène, un reste alkyle, alcényle, alcynyle, cycloalkyle, aryle, hétéroaryle ou hétérocyclyle,
R¹⁻² pouvant éventuellement être substitué avec 1 à 3 substituants R¹⁻²⁻¹, les substituants R¹⁻²⁻¹ étant choisis indépendamment les uns des autres dans le groupe constitué d'halogène, trifluorométhyle, trifluorométhoxy, nitro, amino, alkyle, monoalkylamino, dialkylamino, cycloalkyle, aryle, hétéroaryle, hétérocyclyle, hydroxy, alkoxy, phénoxy, carboxyle, alkoxycarbonyle, alkylcarbonyle, aminocarbonyle, monoalkylaminocarbonyle, dialkylaminocarbonyle, aminosulfonyle, alkylaminosulfonyle, dialkylaminosulfonyle, arylaminosulfonyle, hétérocyclylaminosulfonyle, hétéroarylaminosulfonyle, aminocarbonylamino, hydroxycarbonylamino et alkoxycarbonylamino, les restes aryle et hétéroaryle pouvant porter 1 ou 2 substituants qui sont choisis indépendamment l'un de l'autre dans le groupe constitué d'halogène, hydroxy, alkyle, alkoxy, trifluorométhyle, trifluorométhoxy, nitro, amino, cyano, monoalkylamino et dialkylamino,
ou bien
R¹⁻² et R¹⁻³ forment, conjointement avec l'atome d'azote auquel ils sont liés, un hétérocycle qui peut éventuellement être condensé au benzène,
R¹⁻⁴ représente un reste alkyle, alcényle, alcynyle, cycloalkyle ou aryle,
R¹⁻⁴ pouvant éventuellement être substitué avec 1 à 3 substituants R¹⁻⁴⁻¹, les substituants R¹⁻⁴⁻¹ étant choisis indépendamment les uns des autres dans le groupe constitué d'halogène, alkyle, aryle, amino, monoalkylamino, dialkylamino, aminocarbonyle, monoalkylaminocarbonyle, dialkylaminocarbonyle, carboxyle, alkoxycarbonyle, alkylcarbonyle, hétéroaryle et hétérocyclyle,
R² représente l'hydrogène ou le reste méthyle,
R³ représente l'hydrogène ou un reste alkyle en C₁ à C₃,
R⁴ représente l'hydrogène ou un reste alkyle en C₁ à C₃,
R⁵ est choisi dans le groupe constitué d'halogène, trifluorométhyle, trifluorométhoxy, nitro, amino, monoalkylamino, dialkylamino, hydroxy, alkyle, alkoxy, carboxyle, alkoxycarbonyle, aminocarbonyle, monoalkylaminocarbonyle, dialkylaminocarbonyle, aryle et hétéroaryle,
ou bien
deux substituants R⁵ forment, conjointement avec les atomes de carbone auxquels ils sont liés, un reste cycloalkyle ou hétérocyclyle,
ce reste cycloalkyle ou hétérocyclyle pouvant être substitué avec 0, 1 ou 2 substituants R⁵⁻¹, les substituants R⁵⁻¹ étant choisis indépendamment l'un de l'autre dans le groupe constitué d'halogène, alkyle, nitro, amino, trifluorométhyle, hydroxy et alkoxy,
n représente le nombre 0, 1, 2 ou 3,
les restes R⁵ pouvant être identiques ou différents lorsque n a la valeur 2 ou 3,
R⁶ est un reste alkyle, cycloalkyle ou hétérocyclyle,
R⁶ pouvant être substitué avec 0, 1 ou 2 substituants R⁶⁻¹, les substituants R⁶⁻¹ étant choisis indépendamment l'un de l'autre dans le groupe constitué d'halogène, nitro, amino, trifluorométhyle, hydroxy, alkyle et alkoxy,
ou leurs sels, leurs produits de solvatation et leurs hydrates acceptables du point de vue pharmaceutique.

2. Composés de formule générale (I) suivant la revendication 1,
formule dans laquelle
R¹⁻¹ est un reste alkyle, cycloalkyle ou aryle,
R¹⁻¹ pouvant éventuellement être substitué avec 1 à 3 substituants R¹⁻¹⁻¹, les substituants R¹⁻¹⁻¹ étant choisis indépendamment les uns des autres dans le groupe constitué d'halogène, alkyle, aryle et alkoxy,
R¹⁻² et R¹⁻³ sont identiques ou différents et représentent l'hydrogène, un reste alkyle, alcényle, cycloalkyle, aryle ou hétéroaryle,
R¹⁻² pouvant éventuellement être substitué avec 1 ou 2 substituants R¹⁻²⁻¹, les substituants R¹⁻²⁻¹ étant choisis indépendamment l'un de l'autre dans le groupe constitué d'halogène, trifluorométhyle, trifluorométhoxy, nitro, amino, alkyle, monoalkylamino, dialkylamino, cycloalkyle, aryle, hétéroaryle, hétérocyclyle, hydroxy, alkoxy, phénoxy, alkylcarbonyle, aminocarbonyle, monoalkylaminocarbonyle, dialkylaminocarbonyle, aminosulfonyle, les restes aryle et hétéroaryle pouvant porter 1 ou 2 substituants qui sont choisis indépendamment l'un de l'autre dans le groupe constitué d'halogène, hydroxy, alkoxy, trifluorométhyle, trifluorométhoxy, nitro et amino,
ou bien R¹⁻² et R¹⁻³ forment, conjointement avec l'atome d'azote auquel ils sont liés, un hétérocycle qui peut éventuellement être condensé au benzène.
R¹⁻⁴ est un reste alkyle, alcényle ou aryle,
R¹⁻⁴ pouvant éventuellement être substitué avec 1 à 3 substituants R¹⁻⁴⁻¹, les substituants R¹⁻⁴⁻¹ étant choisis indépendamment les uns des autres dans le groupe constitué d'halogène, alkyle et aryle,
R² représente l'hydrogène
R³ représente l'hydrogène ou le reste méthyle
R⁴ représente le reste méthyle
R⁵ est choisi dans le groupe constitué de fluor, chlore, trifluorométhyle, trifluorométhoxy, nitro, amino, monoalkylamino, dialkylamino, hydroxy, alkyle, alkoxy, alkoxycarbonyle, aminocarbonyle, phényle et hétéroaryle pentagonal ou hexagonal,
ou bien
deux substituants R⁵ forment conjointement avec les atomes de carbone auxquels ils sont liés un reste cycloalkyle ou hétérocyclyle,
n a la valeur 0, 1 ou 2,
les restes R⁵ pouvant être identiques ou différents lorsque n est égal à 2,
R⁶ est un reste alkyle ou cycloalkyle,
R⁶ pouvant être substitué avec 0, 1 ou 2 substituants R⁶⁻¹, les substituants R⁶⁻¹ étant choisis indépendamment l'un de l'autre dans le groupe constitué d'halogène, trifluorométhyle et alkoxy,
ainsi que leurs sels, leurs produits de solvatation et leurs hydrates acceptables du point de vue pharmaceutique.

3. Composés de formule générale (I) suivant la revendication 1 ou 2,
formule dans laquelle
R¹⁻¹ est un reste alkyle en C₁ à C₄, cyclopentyle, cyclohexyle, phényle ou naphtyle,
R¹⁻¹ pouvant éventuellement être substitué avec 1 ou 2 substituants R¹⁻¹⁻¹, les substituants R¹⁻¹⁻¹ étant choisis indépendamment l'un de l'autre dans le groupe constitué de fluor, chlore, méthyle, éthyle, phényle, méthoxy et éthoxy,
R¹⁻² est un reste alkyle, alcényle, cycloalkyle, aryle ou hétéroaryle,
R¹⁻² pouvant éventuellement être substitué avec un substituant R¹⁻²⁻¹, le substituant R¹⁻²⁻¹ étant choisi dans le groupe constitué de fluor, chlore, trifluorométhyle, amino, alkyle, monoalkylamino, dialkylamino, alkylcarbonyle, aryle, hétéroaryle, hydroxy, méthoxy et phénoxy, les restes aryle et hétéroaryle pouvant être substitués avec un substituant choisi dans le groupe constitué d'halogène, méthoxy, trifluorométhyle et amino,
R¹⁻³ représente l'hydrogène ou le reste méthyle,
ou bien
R¹⁻² et R¹⁻³ forment, conjointement avec l'atome d'azote auquel ils sont liés, un hétérocycle, qui peut éventuellement être condensé au benzène,
R¹⁻⁴ est un reste alkyle, alcényle ou phényle,
R¹⁻⁴ pouvant éventuellement être substitué avec un substituant R¹⁻⁴⁻¹, R¹⁻⁴⁻¹ étant choisi dans le groupe constitué de fluor, chlore et phényle,
R² représente l'hydrogène
R³ représente l'hydrogène
R⁴ est un reste méthyle
R⁵ est choisi dans le groupe constitué de fluor, chlore, trifluorométhyle, alkoxy, méthoxycarbonyle, alkyle en C₁ à C₄, phényle et pyridyle,
n a la valeur 0, 1 ou 2,
les restes R⁵ pouvant être identiques ou différents lorsque n est égal à 2,
R⁶ est un reste isopropyle, isobutyle, isopentyle, cyclopentyle,
ainsi que leurs sels, leurs produits de solvatation et leurs hydrates acceptables du point de vue pharmaceutique.

4. Composés de formule générale (I) suivant la revendication 1, 2 ou 3, qui répondent à la formule générale (Ic) : 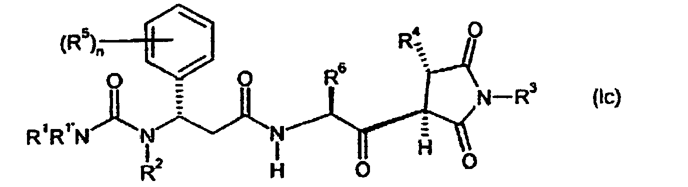 dans laquelle
R¹ à R⁶ et n sont tels que définis dans la revendication 1.

5. Composés de formule générale (I) suivant la revendication 1, 2 ou 3, formule dans laquelle
R¹ est un groupe 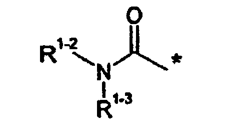
dans lequel
R¹⁻² est un reste alkyle, alcényle, cycloalkyle ou aryle,
R¹⁻² pouvant éventuellement être substitué avec 1 ou 2 substituants, en particulier un substituant R¹⁻²⁻¹, le substituant R¹⁻²⁻¹ étant choisi dans le groupe constitué d'halogène, nitro, amino, alkyle, aryle, hétéroaryle, hydroxy, alkoxy, carboxyle, alkylcarbonyle, alkoxycarbonyle et aminocarbonyle, en particulier choisi dans le groupe constitué d'alkyle, aryle, hétéroaryle et alkoxy.

6. Composés de formule générale (I) suivant la revendication 1, formule dans laquelle
R¹ est un groupe 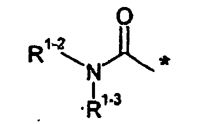
dans lequel R¹⁻³ représente l'hydrogène ou le reste méthyle.

7. Composés de formule générale (I) suivant la revendication 1, 2 ou 3, dans lesquels R² représente l'hydrogène.

8. Composés de formule générale (I) suivant la revendication 1, 2 ou 3, dans lesquels R³ représente l'hydrogène.

9. Composés de formule générale (I) suivant la revendication 1, 2 ou 3, dans lesquels R⁴ représente le reste méthyle.

10. Composés de formule générale (I) suivant la revendication 1, 2 ou 3, dans lesquels R⁵ représente l'hydrogène.

11. Composés de formule générale (I) suivant la revendication 1, 2 ou 3, dans lesquels R⁶ représente le reste isopropyle.

12. Procédé de production de composés de formule générale (I) suivant la revendication 1, dans lequel on fait réagir des composés de formule générale (II) 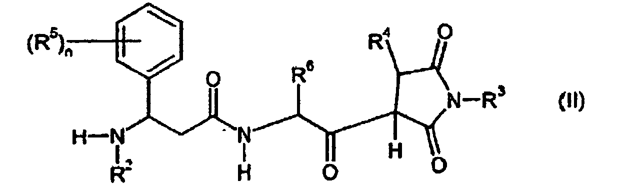 dans laquelle
R² à R⁶ et n ont la définition indiquée dans la revendication 1,
ces composés pouvant éventuellement être sous forme de leurs sels,
au cas où
R¹ représente un groupe 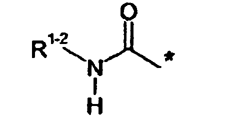
dans lequel
R¹⁻² a la définition indiquée dans la revendication 1,
[A] avec des composés de formule générale (IIIa) 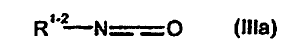 dans laquelle
R¹⁻² a la définition indiquée dans la revendication 1,
ou bien
au cas où
R¹ représente un groupe 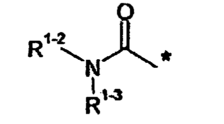
dans lequel
R¹⁻² a la définition indiquée dans la revendication 1,
R¹⁻³ ne représente pas l'hydrogène, ou bien R¹⁻² et R¹⁻³ forment un hétérocycle,
[B] avec des composés de formule générale (IIIb) 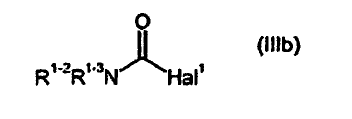 dans laquelle
R¹⁻² et R¹⁻³ ont la définition indiquée dans la revendication 1, et Hal¹ représente un halogénure ou un autre groupe partant,
ou bien
au cas où
R¹ est un groupe 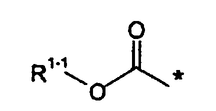
dans lequel
R¹⁻¹ a la définition indiquée dans la revendication 1,
[C] avec des composés de formule générale (IIIc) 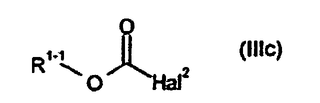 dans laquelle
R¹⁻¹ a la définition indiquée dans la revendication 1 et Hal² représente un halogénure ou un autre groupe partant,
ou bien
au cas où
R¹ est un groupe 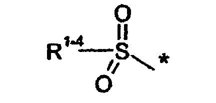
dans lequel
R¹⁻⁴ a la définition indiquée dans la revendication 1,
[D] avec des composés de formule générale (IIId) 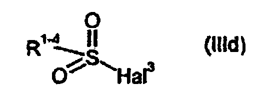
dans laquelle
R¹⁻⁴ a la définition indiquée dans la revendication 1 et Hal³ représente un halogénure ou un autre groupe partant.

13. Composés de formule générale (I) suivant la revendication 1, 2 ou 3 destinés à combattre des maladies.

14. Composés de formule générale (I) suivant la revendication 1, 2 ou 3 destinés à combattre des maladies bactériennes.

15. Médicaments, contenant des composés de formule générale (I) suivant la revendication 1, 2 ou 3 et des substances auxiliaires.

16. Utilisation de composés de formule générale (I) suivant la revendication 1, 2 ou 3 pour la préparation d'un médicament destiné au traitement de maladies.

17. Utilisation de composés de formule générale (I) suivant la revendication 1, 2 ou 3 pour la préparation d'un médicament destiné à combattre des maladies bactériennes.
